# EUROPEAN PATENT SPECIFICATION

(11) **EP 1 124 973 B1**
(45) Date of publication and mention of the grant of the patent: **13.02.2008**
(21) Application number: 99937341.8
(22) Date of filing: 21.07.1999
(51) Int. Cl.: C12N 15/82, C12N 9/02, C12N 9/16, C07K 16/16, A01H 5/00, C12N 5/12

(54) **ANTIBODY-MEDIATED DOWN-REGULATION OF PLANT PROTEINS**
ANTIKÖRPER-VERMITTELTE VERMINDERUNGSREGULATION VON PFLANZENPROTEINEN
REGULATION NEGATIVE DE PROTEINES VEGETALES A MEDIATION PAR ANTICORPS

(30) Priority: 21.07.1998 US 93587 P
(43) Date of publication of application: 22.08.2001
(73) Proprietor: Dow AgroSciences LLC, Indianapolis, Indiana 46268 (US)
(72) Inventor: SUKHAPINDA, Kitisri, Zionsville, IN 46077 (US); HASLER, James, M., Danville, IN 46122 (US); PETELL, James, K., Zionsville, IN 46077 (US); STRICKLAND, James, A., Goodlettsville, TN 37072 (US); FOLKERTS, Otto, Guilford, CT 06437 (US)
(74) Representative: Smart, Peter John
(86) International application number: PCT/US1999/016405
(87) International publication number: WO 2000/005391

(56) References cited:
- WO-A-96/01320
- WO-A-97/10328
- WO-A-97/12047
- WO-A-98/50569
- JONES A ET AL: "PALMITOYL-ACYL CARRIER PROTEIN (ACP) THIOESTERASE AND THE EVOLUTIONARY ORIGIN OF PLANT ACYL-ACP THIOESTERASES" PLANT CELL,US,AMERICAN SOCIETY OF PLANT PHYSIOLOGISTS, ROCKVILLE, MD, vol. 7, no. 3, page 359-371 XP002014222 ISSN: 1040-4651
- FIREK S ET AL: "SECRETION OF A FUNCTIONAL SINGLE-CHAIN FV PROTEIN IN TRANSGENIC TOBACCO PLANTS AND CELL SUSPENSION CULTURES" PLANT MOLECULAR BIOLOGY,NL,NIJHOFF PUBLISHERS, DORDRECHT, vol. 23, page 861-870 XP002033959 ISSN: 0167-4412
- ARTSAENKO O ET AL: "EXPRESSION OF A SINGLE-CHAIN FV ANTIBODY AGAINST ABSCISIC ACID CREATES A WILTY PHENOTYPE IN TRANSGENIC TOBACCO" PLANT JOURNAL,GB,BLACKWELL SCIENTIFIC PUBLICATIONS, OXFORD, vol. 8, no. 5, page 745-750 XP002074417 ISSN: 0960-7412
- CONRAD U ET AL: "EXPRESSION OF ENGINEERED ANTIBODIES IN PLANT CELLS" PLANT MOLECULAR BIOLOGY,NL,NIJHOFF PUBLISHERS, DORDRECHT, vol. 26, no. 4, page 1023-1030 XP002033960 ISSN: 0167-4412
- VAN ENGELEN F A ET AL.: "Coordinate expression of antibody subunit genes yields high levels of functional antibodies in roots of transgenic tobacco" PLANT MOLECULAR BIOLOGY, vol. 26, 1994, pages 1701-1710, XP002122098
- KEEGSTRA K: "Transport and routing of proteins into chloroplasts" CELL, vol. 56, 27 January 1989 (1989-01-27), pages 247-253, XP002122099 cited in the application
- KNUTZON D S ET AL: "MODIFICATION OF BRASSICA SEED OIL BY ANTISENSE EXPRESSION OF A STERAROYL-ACYL CARRIER PROTEIN DESATURASE GENE" PROCEEDINGS OF THE NATIONAL ACADEMY OF SCIENCES OF USA,US,NATIONAL ACADEMY OF SCIENCE. WASHINGTON, vol. 89, page 2624-2628 XP002018311 ISSN: 0027-8424
- THOMPSON G ET AL.: "Primary structures of the precursor and mature forms of stearoyl-acyl carrier protein desaturase from safflower embryos and requirement of ferredoxin for enzyme activity" PROCEEDINGS OF THE NATIONAL ACADEMY OF SCIENCES OF USA., vol. 88, March 1991 (1991-03), pages 2578-2582, XP002016567 USNATIONAL ACADEMY OF SCIENCE. WASHINGTON.
- HARALAMPIDIS K ET AL.: "Temporal and transient expression of stearoyl-ACP carrier protein desaturase gene during olive fruit development" JOURNAL OF EXPERIMENTAL BOTANY., vol. 49, no. 327, October 1998 (1998-10), pages 1661-1669, GBOXFORD UNIVERSITY PRESS.

### SEQUENCE LISTING

<110> Sukhapinda, Kitisri
   Hasler, James M
   Petell, James K
   Strickland, James A
   Folkerts, Otto
<120> ANTIBODY-MEDIATED DOWN-REGULATION OF PLANT PROTEINS
<130> 50447
<140>
   <141>
<150> US 60/093,587
   <151> 1998-07-21
<160> 56
<170> PatentIn Ver. 2.0
<210> 1
   <211> 17
   <212> DNA
   <213> Artificial Sequence
<220>
   <223> Description of Artificial Sequence:5' primer
<400> 1
   gargaraaym gncaygg 17
<210> 2
   <211> 18
   <212> DNA
   <213> Artificial Sequence
<220>
   <223> Description of Artificial Sequence:3' primer
<400> 2
   ytcrtgncky ttytcrtc 18
<210> 3
   <211> 276
   <212> DNA
   <213> Zea mays
<220>
   <221> CDS
   <222> (1)..(276)
<400> 3
   gag gag aac agg cat ggt gat ctg ctc aac aag tat atg tac ctc act    48
   Glu Glu Asn Arg His Gly Asp Leu Leu Asn Lys Tyr Met Tyr Leu Thr
    1               5                  10                  15
   ggg agg gtg gat atg agg cag att gag aag aca att cag tat ctt att    96
   Gly Arg Val Asp Met Arg Gln Ile Glu Lys Thr Ile Gln Tyr Leu Ile
               20                  25                  30
   ggc tct gga atg gat cct agg act gag aat aat cct tat ctt ggt ttc    144
   Gly Ser Gly Met Asp Pro Arg Thr Glu Asn Asn Pro Tyr Leu Gly Phe
           35                  40                  45
   atc tac acc tcc ttc caa gag cgg gcg acc ttc atc tca cac ggg aac    192
   Ile Tyr Thr Ser Phe Gln Glu Arg Ala Thr Phe Ile Ser His Gly Asn
       50                  55                  60
   act gct cgt cac gcc aag gac ttt ggc gac tta aag ctt gca caa atc    240
   Thr Ala Arg His Ala Lys Asp Phe Gly Asp Leu Lys Leu Ala Gln Ile
   65                  70                  75                  80
   tgc ggc atc atc gcc tca gat gag aag cga cat gaa                    276
   Cys Gly Ile Ile Ala Ser Asp Glu Lys Arg His Glu
                   85                  90
<210> 4
   <211> 92
   <212> PRT
   <213> Zea mays
<400> 4
   Glu Glu Asn Arg His Gly Asp Leu Leu Asn Lys Tyr Met Tyr Leu Thr
     1               5                  10                  15
   Gly Arg Val Asp Met Arg Gln Ile Glu Lys Thr Ile Gln Tyr Leu Ile
               20                  25                  30
   Gly Ser Gly Met Asp Pro Arg Thr Glu Asn Asn Pro Tyr Leu Gly Phe
           35                  40                  45
   Ile Tyr Thr Ser Phe Gln Glu Arg Ala Thr Phe Ile Ser His Gly Asn
       50                  55                  60
   Thr Ala Arg His Ala Lys Asp Phe Gly Asp Leu Lys Leu Ala Gln Ile
   65                  70                  75                  80
   Cys Gly Ile Ile Ala Ser Asp Glu Lys Arg His Glu
                   85                  90
<210> 5
   <211> 1621
   <212> DNA
   <213> Zea mays
<220>
   <221> CDS
   <222> (146)..(1324)
<220>
   <221> mat_peptide
   <222> (239)..(1324)
<400> 5
   cgcacgcgcc ctctgccgct tgttcgttcc tcgcgctcgc caccaggcac caccacacac 60
   atcccaatct cgcgagggca agcagcaggg tctgcggcgg cggcggcggc cgcgcttccg 120
   gctccccttc ccattggcct ccacg atg gcg ctc cgc ctc aac gac gtc gcg   172
                               Met Ala Leu Arg Leu Asn Asp Val Ala
                                   -30                 -25
   ctc tgc ctc tcc ccg ccg ctc gcc gcc cgc cgc cgc cgc cgc agc agc   220
   Leu Cys Leu Ser Pro Pro Leu Ala Ala Arg Arg Arg Arg Arg Ser Ser
           -20                 -15                 -10
   ggc agg ttc gtc gcc gtc gcc tcc atg acg tcc gcc gtc tcc acc aag   268
   Gly Arg Phe Val Ala Val Ala Ser Met Thr Ser Ala Val Ser Thr Lys
       -5                  -1   1               5                  10
   gtc gag aat aag aag cca ttt gct cct cca agg gag gta cat gtc cag   316
   Val Glu Asn Lys Lys Pro Phe Ala Pro Pro Arg Glu Val His Val Gln
                   15                  20                  25
   gtt aca cat tca atg cca cct cac aag att gaa att ttc aag tcg ctt   364
   Val Thr His Ser Met Pro Pro His Lys Ile Glu Ile Phe Lys Ser Leu
               30                  35                  40
   gat gat tgg gct aga gat aat atc ttg acg cat ctc aag cca gtc gag   412
   Asp Asp Trp Ala Arg Asp Asn Ile Leu Thr His Leu Lys Pro Val Glu
           45                  50                  55
   aag tgt tgg cag cca cag gat ttc ctc ccg gac cca gca tct gaa gga   460
   Lys Cys Trp Gln Pro Gln Asp Phe Leu Pro Asp Pro Ala Ser Glu Gly
       60                  65                  70
   ttt cat gat gaa gtt aag gag ctc aga gaa cgt gcc aag gaa atc cct   508
   Phe His Asp Glu Val Lys Glu Leu Arg Glu Arg Ala Lys Glu Ile Pro
   75                  80                  85                  90
   gat gat tat ttt gtt tgt ttg gtg gga gac atg att acc gag gaa gct   556
   Asp Asp Tyr Phe Val Cys Leu Val Gly Asp Met Ile Thr Glu Glu Ala
                   95                  100                 105
   cta cca aca tac cag act atg ctt aac acc ctc gac ggt gtc aga gat   604
   Leu Pro Thr Tyr Gln Thr Met Leu Asn Thr Leu Asp Gly Val Arg Asp
               110                 115                 120
   gag aca ggt gca agc ccc act gcc tgg gct gtt tgg acg agg gca tgg   652
   Glu Thr Gly Ala Ser Pro Thr Ala Trp Ala Val Trp Thr Arg Ala Trp
           125                 130                 135
   act gct gag gag aac agg cat ggt gat ctg ctc aac aag tat atg tac   700
   Thr Ala Glu Glu Asn Arg His Gly Asp Leu Leu Asn Lys Tyr Met Tyr
       140                 145                 150
   ctc act ggg agg gtg gat atg agg cag att gag aag aca att cag tat   748
   Leu Thr Gly Arg Val Asp Met Arg Gln Ile Glu Lys Thr Ile Gln Tyr
   155                 160                 165                 170
   ctt att ggc tct gga atg gat cct agg act gag aat aat cct tat ctt   796
   Leu Ile Gly Ser Gly Met Asp Pro Arg Thr Glu Asn Asn Pro Tyr Leu
                   175                 180                 185
   ggt ttc atc tac acc tcc ttc caa gag cgg gcg acc ttc atc tca cac   844
   Gly Phe Ile Tyr Thr Ser Phe Gln Glu Arg Ala Thr Phe Ile Ser His
               190                 195                 200
   ggg aac act gct cgt cac gcc aag gac ttt ggc gac tta aag ctt gca     892
   Gly Asn Thr Ala Arg His Ala Lys Asp Phe Gly Asp Leu Lys Leu Ala
           205                 210                 215
   caa atc tgc ggc atc atc gcc tca gat gag aag cga cat gaa act gcg     940
   Gln Ile Cys Gly Ile Ile Ala Ser Asp Glu Lys Arg His Glu Thr Ala
       220                 225                 230
   tac acc aag atc gtg gag aag ctg ttt gag atc gac cct gat ggt acc     988
   Tyr Thr Lys Ile Val Glu Lys Leu Phe Glu Ile Asp Pro Asp Gly Thr
   235                 240                 245                 250
   gtg gtc gct ctg gct gac atg atg agg aag aag atc tca atg cct gcc     1036
   Val Val Ala Leu Ala Asp Met Met Arg Lys Lys Ile Ser Met Pro Ala
                   255                 260                 265
   cac ctg atg ttt gac ggg cag gac gac aag ctg ttc gag cac ttc tcc     1084
   His Leu Met Phe Asp Gly Gln Asp Asp Lys Leu Phe Glu His Phe Ser
               270                 275                 280
   atg gtc gcg cag agg ctt ggc gtt tac acc gcc agg gac tac gcc gac     1132
   Met Val Ala Gln Arg Leu Gly Val Tyr Thr Ala Arg Asp Tyr Ala Asp
           285                 290                 295
   atc ctc gag ttc ctc gtc gac agg tgg aag gtg gcg agc ctg act ggt     1180
   Ile Leu Glu Phe Leu Val Asp Arg Trp Lys Val Ala Ser Leu Thr Gly
       300                 305                 310
   ctg tcg ggt gaa ggg aac aag gcg cag gac tac ctt tgc acc ctt gct     1228
   Leu Ser Gly Glu Gly Asn Lys Ala Gln Asp Tyr Leu Cys Thr Leu Ala
   315                 320                 325                 330
   tca aga atc agg agg ctg gag gag agg gcc cag agc aga gcc aag aaa     1276
   Ser Arg Ile Arg Arg Leu Glu Glu Arg Ala Gln Ser Arg Ala Lys Lys
                   335                 340                 345
   gcc ggc acg ctg cct ttc agc tgg gta tac ggt agg gac gtc caa ctg     1324
   Ala Gly Thr Leu Pro Phe Ser Trp Val Tyr Gly Arg Asp Val Gln Leu
               350                 355                 360
   tgagatcgga aacctgctgc ggactgctta gacaagacct gctgtgtctg cgttacatag   1384
   gtctccaggt tttgatcaaa tggtcccgtg tcgtcttata gagcgatagg agaacgtgtt   1444
   ggtctgtggt gtagctttgt ttttattttg tatttttctg ctttgatgta caacctgtgg   1504
   ccgcatgaac tggggcgtgg agatgggagc gaccatgccg tactttgtct gtcgctggcg   1564
   gtgtgtttcg gtatgttatt tgagttgctc agatctgtta aaaaaaaaaa aaaaaaa      1621
<210> 6
   <211> 393
   <212> PRT
   <213> Zea mays
<400> 6
   Met Ala Leu Arg Leu Asn Asp Val Ala Leu Cys Leu Ser Pro Pro Leu
       -30                 -25                 -20
   Ala Ala Arg Arg Arg Arg Arg Ser Ser Gly Arg Phe Val Ala Val Ala
   -15                 -10                 -5                  -1   1
   Ser Met Thr Ser Ala Val Ser Thr Lys Val Glu Asn Lys Lys Pro Phe
               5                   10                  15
   Ala Pro Pro Arg Glu Val His Val Gln Val Thr His Ser Met Pro Pro
           20                  25                  30
   His Lys Ile Glu Ile Phe Lys Ser Leu Asp Asp Trp Ala Arg Asp Asn
       35                  40                  45
   Ile Leu Thr His Leu Lys Pro Val Glu Lys Cys Trp Gln Pro Gln Asp
   50                  55                  60                  65
   Phe Leu Pro Asp Pro Ala Ser Glu Gly Phe His Asp Glu Val Lys Glu
                   70                  75                  80
   Leu Arg Glu Arg Ala Lys Glu Ile Pro Asp Asp Tyr Phe Val Cys Leu
               85                  90                  95
   Val Gly Asp Met Ile Thr Glu Glu Ala Leu Pro Thr Tyr Gln Thr Met
           100                 105                 110
   Leu Asn Thr Leu Asp Gly Val Arg Asp Glu Thr Gly Ala Ser Pro Thr
       115                 120                 125
   Ala Trp Ala Val Trp Thr Arg Ala Trp Thr Ala Glu Glu Asn Arg His
   130                 135                 140                 145
   Gly Asp Leu Leu Asn Lys Tyr Met Tyr Leu Thr Gly Arg Val Asp Met
                   150                 155                 160
   Arg Gln Ile Glu Lys Thr Ile Gln Tyr Leu Ile Gly Ser Gly Met Asp
               165                 170                 175
   Pro Arg Thr Glu Asn Asn Pro Tyr Leu Gly Phe Ile Tyr Thr Ser Phe
           180                 185                 190
   Gln Glu Arg Ala Thr Phe Ile Ser His Gly Asn Thr Ala Arg His Ala
       195                 200                 205
   Lys Asp Phe Gly Asp Leu Lys Leu Ala Gln Ile Cys Gly Ile Ile Ala
   210                 215                 220                 225
   Ser Asp Glu Lys Arg His Glu Thr Ala Tyr Thr Lys Ile Val Glu Lys
                   230                 235                 240
   Leu Phe Glu Ile Asp Pro Asp Gly Thr Val Val Ala Leu Ala Asp Met
               245                 250                 255
   Met Arg Lys Lys Ile Ser Met Pro Ala His Leu Met Phe Asp Gly Gln
           260                 265                 270
   Asp Asp Lys Leu Phe Glu His Phe Ser Met Val Ala Gln Arg Leu Gly
       275                 280                 285
   Val Tyr Thr Ala Arg Asp Tyr Ala Asp Ile Leu Glu Phe Leu Val Asp
   290                 295                 300                 305
   Arg Trp Lys Val Ala Ser Leu Thr Gly Leu Ser Gly Glu Gly Asn Lys
                   310                 315                 320
   Ala Gln Asp Tyr Leu Cys Thr Leu Ala Ser Arg Ile Arg Arg Leu Glu
               325                 330                 335
   Glu Arg Ala Gln Ser Arg Ala Lys Lys Ala Gly Thr Leu Pro Phe Ser
           340                 345                 350
   Trp Val Tyr Gly Arg Asp Val Gln Leu
       355                 360
<210> 7
   <211> 30
   <212> DNA
   <213> Artificial Sequence
<220>
   <223> Description of Artificial Sequence:primer
<400> 7
   atggctagcc tccgcctcaa cgacgtcgcg 30
<210> 8
   <211> 36
   <212> DNA
   <213> Artificial Sequence
<220>
   <223> Description of Artificial Sequence:primer
<400> 8
   aaagctagct catcacagtt ggacgtccct accgta 36
<210> 9
   <211> 30
   <212> DNA
   <213> Artificial Sequence
<220>
   <223> Description of Artificial Sequence:primer
<400> 9
   accatggcta gcatgacgtc cgccgtctcc 30
<210> 10
   <211> 30
   <212> DNA
   <213> Artificial Sequence
<220>
   <223> Description of Artificial Sequence:primer
<400> 10
   gatgatgcta gctcacagtt ggacgtccct 30
<210> 11
   <211> 1107
   <212> DNA
   <213> Zea mays
<220>
   <221> CDS
   <222> (4)..(1092)
<400> 11
   acc atg gct acg atg acg tcc gcc gtc tcc acc aag gtc gag aat aag      48
       Met Ala Thr Met Thr Ser Ala Val Ser Thr Lys Val Glu Asn Lys
        1               5                  10                  15
   aag cca ttt gct cct cca agg gag gta cat gtc cag gtt aca cat tca      96
   Lys Pro Phe Ala Pro Pro Arg Glu Val His Val Gln Val Thr His Ser
                   20                  25                  30
   atg cca cct cac aag att gaa att ttc aag tcg ctt gat gat tgg gct     144
   Met Pro Pro His Lys Ile Glu Ile Phe Lys Ser Leu Asp Asp Trp Ala
               35                  40                  45
   aga gat aat atc ttg acg cat ctc aag cca gtc gag aag tgt tgg cag     192
   Arg Asp Asn Ile Leu Thr His Leu Lys Pro Val Glu Lys Cys Trp Gln
           50                  55                  60
   cca cag gat ttc ctc ccg gac cca gca tct gaa gga ttt cat gat gaa     240
   Pro Gln Asp Phe Leu Pro Asp Pro Ala Ser Glu Gly Phe His Asp Glu
       65                  70                  75
   gtt aag gag ctc aga gaa cgt gcc aag gaa atc cct gat gat tat ttt     288
   Val Lys Glu Leu Arg Glu Arg Ala Lys Glu Ile Pro Asp Asp Tyr Phe
   80                  85                  90                  95
   gtt tgt ttg gtg gga gac atg att acc gag gaa gct cta cca aca tac     336
   Val Cys Leu Val Gly Asp Met Ile Thr Glu Glu Ala Leu Pro Thr Tyr
                   100                 105                 110
   cag act atg ctt aac acc ctc gac ggt gtc aga gat gag aca ggt gca     384
   Gln Thr Met Leu Asn Thr Leu Asp Gly Val Arg Asp Glu Thr Gly Ala
               115                 120                 125
   agc ccc act gcc tgg gct gtt tgg acg agg gca tgg act gct gag gag     432
   Ser Pro Thr Ala Trp Ala Val Trp Thr Arg Ala Trp Thr Ala Glu Glu
           130                 135                 140
   aac agg cat ggt gat ctg ctc aac aag tat atg tac ctc act ggg agg     480
   Asn Arg His Gly Asp Leu Leu Asn Lys Tyr Met Tyr Leu Thr Gly Arg
       145                 150                 155
   gtg gat atg agg cag att gag aag aca att cag tat ctt att ggc tct     528
   Val Asp Met Arg Gln Ile Glu Lys Thr Ile Gln Tyr Leu Ile Gly Ser
   160                 165                 170                 175
   gga atg gat cct agg act gag aat aat cct tat ctt ggt ttc atc tac     576
   Gly Met Asp Pro Arg Thr Glu Asn Asn Pro Tyr Leu Gly Phe Ile Tyr
                   180                 185                 190
   acc tcc ttc caa gag cgg gcg acc ttc atc tca cac ggg aac act gct     624
   Thr Ser Phe Gln Glu Arg Ala Thr Phe Ile Ser His Gly Asn Thr Ala
               195                 200                 205
   cgt cac gcc aag gac ttt ggc gac tta aag ctt gca caa atc tgc ggc     672
   Arg His Ala Lys Asp Phe Gly Asp Leu Lys Leu Ala Gln Ile Cys Gly
           210                 215                 220
   atc atc gcc tca gat gag aag cga cat gaa act gcg tac acc aag atc     720
   Ile Ile Ala Ser Asp Glu Lys Arg His Glu Thr Ala Tyr Thr Lys Ile
       225                 230                 235
   gtg gag aag ctg ttt gag atc gac cct gat ggt acc gtg gtc gct ctg     768
   Val Glu Lys Leu Phe Glu Ile Asp Pro Asp Gly Thr Val Val Ala Leu
   240                 245                 250                 255
   gct gac atg atg agg aag aag atc tca atg cct gcc cac ctg atg ttt     816
   Ala Asp Met Met Arg Lys Lys Ile Ser Met Pro Ala His Leu Met Phe
                   260                 265                 270
   gac ggg cag gac gac aag ctg ttc gag cac ttc tcc atg gtc gcg cag     864
   Asp Gly Gln Asp Asp Lys Leu Phe Glu His Phe Ser Met Val Ala Gln
               275                 280                 285
   agg ctt ggc gtt tac acc gcc agg gac tac gcc gac atc ctc gag ttc     912
   Arg Leu Gly Val Tyr Thr Ala Arg Asp Tyr Ala Asp Ile Leu Glu Phe
           290                 295                 300
   ctc gtc gac agg tgg aag gtg gcg agc ctg act ggt ctg tcg ggt gaa     960
   Leu Val Asp Arg Trp Lys Val Ala Ser Leu Thr Gly Leu Ser Gly Glu
       305                 310                 315
   ggg aac aag gcg cag gac tac ctt tgc acc ctt gct tca aga atc agg     1008
   Gly Asn Lys Ala Gln Asp Tyr Leu Cys Thr Leu Ala Ser Arg Ile Arg
   320                 325                 330                 335
   agg ctg gag gag agg gcc cag agc aga gcc aag aaa gcc ggc acg ctg     1056
   Arg Leu Glu Glu Arg Ala Gln Ser Arg Ala Lys Lys Ala Gly Thr Leu
                   340                 345                 350
   cct ttc agc tgg gta tac ggt agg gac gtc caa ctg tgagctagca tcatc    1107
   Pro Phe Ser Trp Val Tyr Gly Arg Asp Val Gln Leu
               355                 360
<210> 12
   <211> 363
   <212> PRT
   <213> Zea mays
<400> 12
   Met Ala Thr Met Thr Ser Ala Val Ser Thr Lys Val Glu Asn Lys Lys
    1               5                  10                  15
   Pro Phe Ala Pro Pro Arg Glu Val His Val Gln Val Thr His Ser Met
               20                  25                  30
   Pro Pro His Lys Ile Glu Ile Phe Lys Ser Leu Asp Asp Trp Ala Arg
           35                  40                  45
   Asp Asn Ile Leu Thr His Leu Lys Pro Val Glu Lys Cys Trp Gln Pro
       50                  55                  60
   Gln Asp Phe Leu Pro Asp Pro Ala Ser Glu Gly Phe His Asp Glu Val
   65                  70                  75                  80
   Lys Glu Leu Arg Glu Arg Ala Lys Glu Ile Pro Asp Asp Tyr Phe Val
                   85                  90                  95
   Cys Leu Val Gly Asp Met Ile Thr Glu Glu Ala Leu Pro Thr Tyr Gln
               100                 105                 110
   Thr Met Leu Asn Thr Leu Asp Gly Val Arg Asp Glu Thr Gly Ala Ser
           115                 120                 125
   Pro Thr Ala Trp Ala Val Trp Thr Arg Ala Trp Thr Ala Glu Glu Asn
       130                 135                 140
   Arg His Gly Asp Leu Leu Asn Lys Tyr Met Tyr Leu Thr Gly Arg Val
   145                 150                 155                 160
   Asp Met Arg Gln Ile Glu Lys Thr Ile Gln Tyr Leu Ile Gly Ser Gly
                   165                 170                 175
   Met Asp Pro Arg Thr Glu Asn Asn Pro Tyr Leu Gly Phe Ile Tyr Thr
               180                 185                 190
   Ser Phe Gln Glu Arg Ala Thr Phe Ile Ser His Gly Asn Thr Ala Arg
           195                 200                 205
   His Ala Lys Asp Phe Gly Asp Leu Lys Leu Ala Gln Ile Cys Gly Ile
       210                 215                 220
   Ile Ala Ser Asp Glu Lys Arg His Glu Thr Ala Tyr Thr Lys Ile Val
   225                 230                 235                 240
   Glu Lys Leu Phe Glu Ile Asp Pro Asp Gly Thr Val Val Ala Leu Ala
                   245                 250                 255
   Asp Met Met Arg Lys Lys Ile Ser Met Pro Ala His Leu Met Phe Asp
               260                 265                 270
   Gly Gln Asp Asp Lys Leu Phe Glu His Phe Ser Met Val Ala Gln Arg
           275                 280                 285
   Leu Gly Val Tyr Thr Ala Arg Asp Tyr Ala Asp Ile Leu Glu Phe Leu
       290                 295                 300
   Val Asp Arg Trp Lys Val Ala Ser Leu Thr Gly Leu Ser Gly Glu Gly
   305                 310                 315                 320
   Asn Lys Ala Gln Asp Tyr Leu Cys Thr Leu Ala Ser Arg Ile Arg Arg
                   325                 330                 335
   Leu Glu Glu Arg Ala Gln Ser Arg Ala Lys Lys Ala Gly Thr Leu Pro
               340                 345                 350
   Phe Ser Trp Val Tyr Gly Arg Asp Val Gln Leu
           355                 360
<210> 13
   <211> 9
   <212> PRT
   <213> Zea mays
<400> 13
   Met Thr Ser Ala Val Ser Thr Lys Val
     1               5
<210> 14
   <211> 10
   <212> PRT
   <213> Zea mays
<400> 14
   Glu Val His Val Gln Val Thr Xaa Ser Met
     1               5                  10
<210> 15
   <211> 31
   <212> PRT
   <213> Zea mays
<400> 15
   Met Ala Leu Arg Leu Asn Asp Val Ala Leu Cys Leu Ser Pro Pro Leu
     1               5                  10                  15
   Ala Ala Arg Arg Arg Arg Arg Ser Ser Gly Arg Phe Val Ala Val
               20                  25                  30
<210> 16
   <211> 42
   <212> DNA
   <213> Artificial Sequence
<220>
   <223> Description of Artificial Sequence:primer
<400> 16
   gcactgcaag tatctagact sstrwcasty gywgmwggkr yc 42
<210> 17
   <211> 36
   <212> DNA
   <213> Artificial Sequence
<220>
   <223> Description of Artificial Sequence:primer
<400> 17
   aaagctagcc tsctgcygyt ctkkttwycw ggtryc 36
<210> 18
   <211> 46
   <212> DNA
   <213> Artificial Sequence
<220>
   <223> Description of Artificial Sequence:primer
<400> 18
   tacgttacct gagcggccgc gctgggctca agttttttgt ccaccg 46
<210> 19
   <211> 38
   <212> DNA
   <213> Artificial Sequence
<220>
   <223> Description of Artificial Sequence:primer
<400> 19
   tttgctagct tactaacact cattcctgtt gaagctct 38
<210> 20
   <211> 102
   <212> DNA
   <213> Artificial Sequence
<220>
   <223> Description of Artificial Sequence:primer
<400> 20
   gcactaggtc aatctagaat ggtaagcgct attgttttat atgtgctttt ggcggcggcg 60
   cattctgcct ttgcggcggt tgtgatgacc ccaaacccac tc 102
<210> 21
   <211> 738
   <212> DNA
   <213> mouse
<220>
   <221> CDS
   <222> (37)..(693)
<400> 21
   accctcctcc tgttcnnnnn ntcagntgtc ancagt gat gtt gtg atg acc cca    54
                                           Asp Val Val Met Thr Pro
                                            1               5
   aac cca ctc tcc ctg cct gtc agt ctt gga gat caa gcc tcc atc tct   102
   Asn Pro Leu Ser Leu Pro Val Ser Leu Gly Asp Gln Ala Ser Ile Ser
               10                  15                  20
   tgc aga tct agt cag agc ctt tta cac agt aat gga atc acc tat tta   150
   Cys Arg Ser Ser Gln Ser Leu Leu His Ser Asn Gly Ile Thr Tyr Leu
           25                  30                  35
   cat tgg tac ctg cag aag cca ggc cag tct cca aag ctc ctg atc tac   198
   His Trp Tyr Leu Gln Lys Pro Gly Gln Ser Pro Lys Leu Leu Ile Tyr
       40                  45                  50
   aaa gtt tcc aac cga ttt tct ggg gtc cca gac agg ttc agt ggc agt   246
   Lys Val Ser Asn Arg Phe Ser Gly Val Pro Asp Arg Phe Ser Gly Ser
    55                  60                  65                  70
   gga tca ggg aca gat ttc aca ctc aag atc aac aga gtg gag gct gag   294
   Gly Ser Gly Thr Asp Phe Thr Leu Lys Ile Asn Arg Val Glu Ala Glu
                            75                   80                   85
   gat ctg gga gtt tat ttc tgc tct caa agt aca cat gtt ccg tac acg      342
   Asp Leu Gly Val Tyr Phe Cys Ser Gln Ser Thr His Val Pro Tyr Thr
               90                  95                  100
   ttc gga ggg ggg acc aag ctg gaa ata aaa cgg gct gat gct gca cca      390
   Phe Gly Gly Gly Thr Lys Leu Glu Ile Lys Arg Ala Asp Ala Ala Pro
           105                 110                 115
   act gta tcc atc ttc cca cca tcc agt gag cag tta aca tct gga ggt      438
   Thr Val Ser Ile Phe Pro Pro Ser Ser Glu Gln Leu Thr Ser Gly Gly
       120                 125                 130
   gcc tca gtc gtg tgc ttc ttg aac aac ttc tac ccc aaa gac atc aat      486
   Ala Ser Val Val Cys Phe Leu Asn Asn Phe Tyr Pro Lys Asp Ile Asn
   135                 140                 145                 150
   gtc aag tgg aag att gat ggc agt gaa cga caa aat ggc gtc ctg aac      534
   Val Lys Trp Lys Ile Asp Gly Ser Glu Arg Gln Asn Gly Val Leu Asn
                   155                 160                 165
   agt tgg act gat cag gac acc aaa gac agc acc tac agc atg agc agc      582
   Ser Trp Thr Asp Gln Asp Thr Lys Asp Ser Thr Tyr Ser Met Ser Ser
               170                 175                 180
   acc ctc acg ttg acc aag gac gag tat gaa cga cat aac agc tat acc      630
   Thr Leu Thr Leu Thr Lys Asp Glu Tyr Glu Arg His Asn Ser Tyr Thr
           185                 190                 195
   tgt gag gcc act cac aag aca tca act tca ccc att gtc aag agc ttc      678
   Cys Glu Ala Thr His Lys Thr Ser Thr Ser Pro Ile Val Lys Ser Phe
       200                 205                 210
   aac agg aat gag tgt tagtaagcta gcacgcccga tggtgggacg gtatgaataa      733
   Asn Arg Asn Glu Cys
   215
   tccgg                                                                 738
<210> 22
   <211> 336
   <212> DNA
   <213> mouse
<220>
   <221> CDS
   <222> (1)..(336)
<400> 22
   gat gtt gtg atg acc cca aac cca ctc tcc ctg cct gtc agt ctt gga     48
   Asp Val Val Met Thr Pro Asn Pro Leu Ser Leu Pro Val Ser Leu Gly
    1               5                  10                  15
   gat caa gcc tcc atc tct tgc aga tct agt cag agc ctt tta cac agt     96
   Asp Gln Ala Ser Ile Ser Cys Arg Ser Ser Gln Ser Leu Leu His Ser
               20                  25                  30
   aat gga atc acc tat tta cat tgg tac ctg cag aag cca ggc cag tct    144
   Asn Gly Ile Thr Tyr Leu His Trp Tyr Leu Gln Lys Pro Gly Gln Ser
           35                  40                  45
   cca aag ctc ctg atc tac aaa gtt tcc aac cga ttt tct ggg gtc cca    192
   Pro Lys Leu Leu Ile Tyr Lys Val Ser Asn Arg Phe Ser Gly Val Pro
       50                  55                  60
   gac agg ttc agt ggc agt gga tca ggg aca gat ttc aca ctc aag atc    240
   Asp Arg Phe Ser Gly Ser Gly Ser Gly Thr Asp Phe Thr Leu Lys Ile
   65                  70                  75                  80
   aac aga gtg gag gct gag gat ctg gga gtt tat ttc tgc tct caa agt    288
   Asn Arg Val Glu Ala Glu Asp Leu Gly Val Tyr Phe Cys Ser Gln Ser
                   85                  90                  95
   aca cat gtt ccg tac acg ttc gga ggg ggg acc aag ctg gaa ata aaa    336
   Thr His Val Pro Tyr Thr Phe Gly Gly Gly Thr Lys Leu Glu Ile Lys
               100                 105                 110
<210> 23
   <211> 108
   <212> DNA
   <213> Artificial Sequence
<220>
   <223> Description of Artificial Sequence:primer
<400> 23
   gcactaggtc aatctagaat ggtaagcgct attgttttat atgtgctttt ggcggcggcg 60
   gcgcattctg cctttgcggc ggttcaactg cagcagtctg gggctgag              108
<210> 24
   <211> 882
   <212> DNA
   <213> mouse
<220>
   <221> CDS
   <222> (25)..(837)
<220>
   <221> mat_peptide
   <222> (79)..(837)
<400> 24
   ctgcagggta cggccatatt ggcc atg act atc ctt tgc tgg ctg gcc ctt    51
                              Met Thr Ile Leu Cys Trp Leu Ala Leu
                                          -15                 -10
   ctg tca act ctg act gcc gtc aac act gcg gtt gtg atg acc cca aac    99
   Leu Ser Thr Leu Thr Ala Val Asn Thr Ala Val Val Met Thr Pro Asn
                   -5              -1   1               5
   cca ctc tcc ctg cct gtc agt ctt gga gat caa gcc tcc atc tct tgc   147
   Pro Leu Ser Leu Pro Val Ser Leu Gly Asp Gln Ala Ser Ile Ser Cys
           10                  15                  20
   aga tct agt cag agc ctt tta cac agt aat gga atc acc tat tta cat   195
   Arg Ser Ser Gln Ser Leu Leu His Ser Asn Gly Ile Thr Tyr Leu His
       25                  30                  35
   tgg tac ctg cag aag cca ggc cag tct cca aag ctc ctg atc tac aaa     243
   Trp Tyr Leu Gln Lys Pro Gly Gln Ser Pro Lys Leu Leu Ile Tyr Lys
    40                 45                  50                  55
   gtt tcc aac cga ttt tct ggg gtc cca gac agg ttc agt ggc agt gga     291
   Val Ser Asn Arg Phe Ser Gly Val Pro Asp Arg Phe Ser Gly Ser Gly
                   60                  65                  70
   tca ggg aca gat ttc aca ctc aag atc agc aga gtg gag gct gag gat     339
   Ser Gly Thr Asp Phe Thr Leu Lys Ile Ser Arg Val Glu Ala Glu Asp
               75                  80                  85
   ctg gga gtt tat ttc tgc tct caa agt aca cat gtt ccg tac acg ttc     387
   Leu Gly Val Tyr Phe Cys Ser Gln Ser Thr His Val Pro Tyr Thr Phe
           90                  95                  100
   gga ggg ggg acc aag ctg gaa ata aaa ggc agc acc agc ggc agc ggc     435
   Gly Gly Gly Thr Lys Leu Glu Ile Lys Gly Ser Thr Ser Gly Ser Gly
       105                 110                 115
   aag ccg ggc agc ggc gag ggc agc acc aag ggc cat gtt caa ctg cag     483
   Lys Pro Gly Ser Gly Glu Gly Ser Thr Lys Gly His Val Gln Leu Gln
   120                 125                 130                 135
   cag tct ggg gct gag ctg gtg agg cct ggg gct tca gtg acg ctg tcc     531
   Gln Ser Gly Ala Glu Leu Val Arg Pro Gly Ala Ser Val Thr Leu Ser
                   140                 145                 150
   tgc aag gct tcg ggc tac aca ttt act gac tat gaa ata cac tgg gtg     579
   Cys Lys Ala Ser Gly Tyr Thr Phe Thr Asp Tyr Glu Ile His Trp Val
               155                 160                 165
   agg cag aca cct gtg cat ggc ctg gaa tgg att gga gct att gat cct     627
   Arg Gln Thr Pro Val His Gly Leu Glu Trp Ile Gly Ala Ile Asp Pro
           170                 175                 180
   gaa act ggt ggt act gcc tac aat cag aag ttc aag gac aag gcc ata     675
   Glu Thr Gly Gly Thr Ala Tyr Asn Gln Lys Phe Lys Asp Lys Ala Ile
       185                 190                 195
   gtg act gta gac aaa tcc tcc agc aca gcc tac atg gag ctc cgc agc     723
   Val Thr Val Asp Lys Ser Ser Ser Thr Ala Tyr Met Glu Leu Arg Ser
   200                 205                 210                 215
   ctg aca tct gaa gac tct gcc gtc tat tac tat aca aga tgg ttt gag     771
   Leu Thr Ser Glu Asp Ser Ala Val Tyr Tyr Tyr Thr Arg Trp Phe Glu
                   220                 225                 230
   gac tgg ggc caa ggg act ctg gtc act gtc tct gca atg cgg ggt tct     819
   Asp Trp Gly Gln Gly Thr Leu Val Thr Val Ser Ala Met Arg Gly Ser
               235                 240                 245
   cat cat cat cat cat cat tagggcctct ctggccgatc ccccgaattt            867
   His His His His His His
           250
   ccccgatcgt tcaaa                                                    882
<210> 25
   <211> 339
   <212> DNA
   <213> mouse
<220>
   <221> CDS
   <222> (1) .. (339)
<400> 25
   cag gtt gtg atg acc cca aac cca ctc tcc ctg cct gtc agt ctt gga    48
   Gln Val Val Met Thr Pro Asn Pro Leu Ser Leu Pro Val Ser Leu Gly
     1               5                  10                  15
   gat caa gcc tcc atc tct tgc aga tct agt cag agc ctt tta cac agt    96
   Asp Gln Ala Ser Ile Ser Cys Arg Ser Ser Gln Ser Leu Leu His Ser
                20                  25                  30
   aat gga atc acc tat tta cat tgg tac ctg cag aag cca ggc cag tct    144
   Asn Gly Ile Thr Tyr Leu His Trp Tyr Leu Gln Lys Pro Gly Gln Ser
            35                  40                  45
   cca aag ctc ctg atc tac aaa gtt tcc aac cga ttt tct ggg gtc cca    192
   Pro Lys Leu Leu Ile Tyr Lys Val Ser Asn Arg Phe Ser Gly Val Pro
        50                  55                  60
   gac agg ttc agt ggc agt gga tca ggg aca gat ttc aca ctc aag atc    240
   Asp Arg Phe Ser Gly Ser Gly Ser Gly Thr Asp Phe Thr Leu Lys Ile
    65                  70                  75                  80
   agc aga gtg gag gct gag gat ctg gga gtt tat ttc tgc tct caa agt    288
   Ser Arg Val Glu Ala Glu Asp Leu Gly Val Tyr Phe Cys Ser Gln Ser
                    85                  90                  95
   aca cat gtt ccg tac acg ttc gga ggg ggg acc aag ctg gaa ata aaa    336
   Thr His Val Pro Tyr Thr Phe Gly Gly Gly Thr Lys Leu Glu Ile Lys
               100                 105                 110
   ggc                                                                339
   Gly
<210> 26
   <211> 64
   <212> DNA
   <213> Artificial Sequence
<220>
   <223> Description of Artificial Sequence:primer
<400> 26
   cccgggtttg gattcggagc cagatcctga ggatttaccc tctgcagaga cagtgaccag 60
   agtc                                                              64
<210> 27
   <211> 63
   <212> DNA
   <213> Artificial Sequence
<220>
   <223> Description of Artificial Sequence:primer
<400> 27
   gagggtaaat cctcaggatc tggctccgaa tccaaacccg gggatgttgt gatgacccca 60
   aac 63
<210> 28
   <211> 78
   <212> DNA
   <213> Artificial Sequence
<220>
   <223> Description of Artificial Sequence:primer
<400> 28
   gcactaggtc aagcggccgc ttactaattc agatcctctt ctgagatgag tttttcttct 60
   tttatttcca gcttggtc 78
<210> 29
   <211> 459
   <212> DNA
   <213> mouse
<220>
   <221> CDS
   <222> (19)..(459)
<220>
   <221> mat_peptide
   <222> (79)..(459)
<400> 29
   gcactaggtc aatctaga atg gta agc gct att gtt tta tat gtg ctt ttg    51
                       Met Val Ser Ala Ile Val Leu Tyr Val Leu Leu
                       -20                 -15                 -10
   gcg gcg gcg gcg cat tct gcc ttt gcg gcg gtt caa ctg cag cag tct    99
   Ala Ala Ala Ala His Ser Ala Phe Ala Ala Val Gln Leu Gln Gln Ser
                   -5              -1   1               5
   ggg gct gag ctg gtg agg cct ggg gct tca gtg acg ctg tcc tgc aag   147
   Gly Ala Glu Leu Val Arg Pro Gly Ala Ser Val Thr Leu Ser Cys Lys
           10                  15                  20
   gct tcg ggc tac aca ttt act gac tat gaa ata cac tgg gtg agg cag   195
   Ala Ser Gly Tyr Thr Phe Thr Asp Tyr Glu Ile His Trp Val Arg Gln
       25                  30                  35
   aca cct gtg cat ggc ctg gaa tgg att gga gct att gat cct gaa act   243
   Thr Pro Val His Gly Leu Glu Trp Ile Gly Ala Ile Asp Pro Glu Thr
   40                  45                  50                  55
   ggt ggt act gcc tac aat cag aag ttc aag gac aag gcc ata gtg act   291
   Gly Gly Thr Ala Tyr Asn Gln Lys Phe Lys Asp Lys Ala Ile Val Thr
                   60                  65                  70
   gta gac aaa tcc tcc agc aca gcc tac atg gag ctc cgc agc ctg aca   339
   Val Asp Lys Ser Ser Ser Thr Ala Tyr Met Glu Leu Arg Ser Leu Thr
               75                  80                  85
   tct gaa gac tct gcc gtc tat tac tat aca aga tgg ttt gag gac tgg   387
   Ser Glu Asp Ser Ala Val Tyr Tyr Tyr Thr Arg Trp Phe Glu Asp Trp
           90                  95                  100
   ggc caa ggg act ctg gtc act gtc tct gca gag ggt aaa tcc tca gga   435
   Gly Gln Gly Thr Leu Val Thr Val Ser Ala Glu Gly Lys Ser Ser Gly
       105                 110                 115
   tct ggc tcc gaa tcc aaa ccc ggg                                   459
   Ser Gly Ser Glu Ser Lys Pro Gly
   120                 125
<210> 30
   <211> 438
   <212> DNA
   <213> mouse
<220>
   <221> CDS
   <222> (1)..(408)
<400> 30
   gag ggt aaa tcc tca gga tct ggc tcc gaa tcc aaa ccc ggg gat gtt    48
   Glu Gly Lys Ser Ser Gly Ser Gly Ser Glu Ser Lys Pro Gly Asp Val
     1               5                  10                  15
   gtg atg acc cca aac cca ctc tcc ctg cct gtc agt ctt gga gat caa    96
   Val Met Thr Pro Asn Pro Leu Ser Leu Pro Val Ser Leu Gly Asp Gln
                20                  25                  30
   gcc tcc atc tct tgc aga tct agt cag agc ctt tta cac agt aat gga   144
   Ala Ser Ile Ser Cys Arg Ser Ser Gln Ser Leu Leu His Ser Asn Gly
            35                  40                  45
   atc acc tat tta cat tgg tac ctg cag aag cca ggc cag tct cca aag   192
   Ile Thr Tyr Leu His Trp Tyr Leu Gln Lys Pro Gly Gln Ser Pro Lys
        50                  55                  60
   ctc ctg atc tac aaa gtt tcc aac cga ttt tct ggg gtc cca gac agg   240
   Leu Leu Ile Tyr Lys Val Ser Asn Arg Phe Ser Gly Val Pro Asp Arg
    65                  70                  75                  80
   ttc agt ggc agt gga tca ggg aca gat ttc aca ctc aag atc agc aga   288
   Phe Ser Gly Ser Gly Ser Gly Thr Asp Phe Thr Leu Lys Ile Ser Arg
                    85                  90                  95
   gtg gag gct gag gat ctg gga gtt tat ttc tgc tct caa agt aca cat   336
   Val Glu Ala Glu Asp Leu Gly Val Tyr Phe Cys Ser Gln Ser Thr His
               100                 105                 110
   gtt ccg tac acg ttc gga ggg ggg acc aag ctg gaa ata aaa gaa gaa   384
   Val Pro Tyr Thr Phe Gly Gly Gly Thr Lys Leu Glu Ile Lys Glu Glu
           115                 120                 125
   aaa ctc atc tca gaa gag gat ctg aattagtaag ggccgccttt gacctagtgc  438
   Lys Leu Ile Ser Glu Glu Asp Leu
           130                 135
<210> 31
   <211> 830
   <212> DNA
   <213> mouse
<220>
   <221> CDS
   <222> (7)..(813)
<220>
   <221> mat_peptide
   <222> (67)..(813)
<400> 31
   tctaga atg gta agc gct att gtt tta tat gtg ctt ttg gcg gcg gcg      48
          Met Val Ser Ala Ile Val Leu Tyr Val Leu Leu Ala Ala Ala
          -20                 -15                 -10
   gcg cat tct gcc ttt gcg gcg gtt caa ctg cag cag tct ggg gct gag     96
   Ala His Ser Ala Phe Ala Ala Val Gln Leu Gln Gln Ser Gly Ala Glu
       -5                  -1   1              5                  10
   ctg gtg agg cct ggg gct tca gtg acg ctg tcc tgc aag gct tcg ggc    144
   Leu Val Arg Pro Gly Ala Ser Val Thr Leu Ser Cys Lys Ala Ser Gly
                   15                  20                  25
   tac aca ttt act gac tat gaa ata cac tgg gtg agg cag aca cct gtg    192
   Tyr Thr Phe Thr Asp Tyr Glu Ile His Trp Val Arg Gln Thr Pro Val
               30                  35                  40
   cat ggc ctg gaa tgg att gga gct att gat cct gaa act ggt ggt act    240
   His Gly Leu Glu Trp Ile Gly Ala Ile Asp Pro Glu Thr Gly Gly Thr
           45                  50                  55
   gcc tac aat cag aag ttc aag gac aag gcc ata gtg act gta gac aaa    288
   Ala Tyr Asn Gln Lys Phe Lys Asp Lys Ala Ile Val Thr Val Asp Lys
       60                  65                  70
   tcc tcc agc aca gcc tac atg gag ctc cgc agc ctg aca tct gaa gac    336
   Ser Ser Ser Thr Ala Tyr Met Glu Leu Arg Ser Leu Thr Ser Glu Asp
   75                  80                  85                  90
   tct gcc gtc tat tac tat aca aga tgg ttt gag gac tgg ggc caa ggg    384
   Ser Ala Val Tyr Tyr Tyr Thr Arg Trp Phe Glu Asp Trp Gly Gln Gly
                   95                 100                 105
   act ctg gtc act gtc tct gca gag ggt aaa tcc tca gga tct ggc tcc    432
   Thr Leu Val Thr Val Ser Ala Glu Gly Lys Ser Ser Gly Ser Gly Ser
               110                 115                 120
   gaa tcc aaa ccc ggg gat gtt gtg atg acc cca aac cca ctc tcc ctg    480
   Glu Ser Lys Pro Gly Asp Val Val Met Thr Pro Asn Pro Leu Ser Leu
           125                 130                 135
   cct gtc agt ctt gga gat caa gcc tcc atc tct tgc aga tct agt cag    528
   Pro Val Ser Leu Gly Asp Gln Ala Ser Ile Ser Cys Arg Ser Ser Gln
       140                 145                 150
   agc ctt tta cac agt aat gga atc acc tat tta cat tgg tac ctg cag    576
   Ser Leu Leu His Ser Asn Gly Ile Thr Tyr Leu His Trp Tyr Leu Gln
   155                 160                 165                 170
   aag cca ggc cag tct cca aag ctc ctg atc tac aaa gtt tcc aac cga    624
   Lys Pro Gly Gln Ser Pro Lys Leu Leu Ile Tyr Lys Val Ser Asn Arg
                   175                 180                 185
   ttt tct ggg gtc cca gac agg ttc agt ggc agt gga tca ggg aca gat    672
   Phe Ser Gly Val Pro Asp Arg Phe Ser Gly Ser Gly Ser Gly Thr Asp
               190                 195                 200
   ttc aca ctc aag atc agc aga gtg gag gct gag gat ctg gga gtt tat    720
   Phe Thr Leu Lys Ile Ser Arg Val Glu Ala Glu Asp Leu Gly Val Tyr
           205                 210                 215
   ttc tgc tct caa agt aca cat gtt ccg tac acg ttc gga ggg ggg acc    768
   Phe Cys Ser Gln Ser Thr His Val Pro Tyr Thr Phe Gly Gly Gly Thr
       220                 225                 230
   aag ctg gaa ata aaa gaa gaa aaa ctc atc tca gaa gag gat ctg        813
   Lys Leu Glu Ile Lys Glu Glu Lys Leu Ile Ser Glu Glu Asp Leu
   235                 240                 245
   aattagtaag cggccgc                                                 830
<210> 32
   <211> 269
   <212> PRT
   <213> mouse
<400> 32
   Met Val Ser Ala Ile Val Leu Tyr Val Leu Leu Ala Ala Ala Ala His
   -20                 -15                 -10                  -5
   Ser Ala Phe Ala Ala Val Gln Leu Gln Gln Ser Gly Ala Glu Leu Val
                -1   1               5                  10
   Arg Pro Gly Ala Ser Val Thr Leu Ser Cys Lys Ala Ser Gly Tyr Thr
            15                  20                  25
   Phe Thr Asp Tyr Glu Ile His Trp Val Arg Gln Thr Pro Val His Gly
        30                  35                  40
   Leu Glu Trp Ile Gly Ala Ile Asp Pro Glu Thr Gly Gly Thr Ala Tyr
    45                  50                  55                  60
   Asn Gln Lys Phe Lys Asp Lys Ala Ile Val Thr Val Asp Lys Ser Ser
                    65                  70                  75
   Ser Thr Ala Tyr Met Glu Leu Arg Ser Leu Thr Ser Glu Asp Ser Ala
                80                  85                  90
   Val Tyr Tyr Tyr Thr Arg Trp Phe Glu Asp Trp Gly Gln Gly Thr Leu
            95                 100                 105
   Val Thr Val Ser Ala Glu Gly Lys Ser Ser Gly Ser Gly Ser Glu Ser
       110                 115                 120
   Lys Pro Gly Asp Val Val Met Thr Pro Asn Pro Leu Ser Leu Pro Val
   125                 130                 135                 140
   Ser Leu Gly Asp Gln Ala Ser Ile Ser Cys Arg Ser Ser Gln Ser Leu
                   145                 150                 155
   Leu His Ser Asn Gly Ile Thr Tyr Leu His Trp Tyr Leu Gln Lys Pro
               160                 165                 170
   Gly Gln Ser Pro Lys Leu Leu Ile Tyr Lys Val Ser Asn Arg Phe Ser
           175                 180                 185
   Gly Val Pro Asp Arg Phe Ser Gly Ser Gly Ser Gly Thr Asp Phe Thr
       190                 195                 200
   Leu Lys Ile Ser Arg Val Glu Ala Glu Asp Leu Gly Val Tyr Phe Cys
   205                 210                 215                 220
   Ser Gln Ser Thr His Val Pro Tyr Thr Phe Gly Gly Gly Thr Lys Leu
                   225                 230                 235
   Glu Ile Lys Glu Glu Lys Leu Ile Ser Glu Glu Asp Leu
               240                 245
<210> 33
   <211> 30
   <212> DNA
   <213> Artificial Sequence
<220>
   <223> Description of Artificial Sequence:c-myc tag
<400> 33
   gaagaaaaac tcatctcaga agaggatctg 30
<210> 34
   <211> 60
   <212> DNA
   <213> Artificial Sequence
<220>
   <223> Description of Artificial Sequence:p67 leader
<400> 34
   atggtaagcg ctattgtttt atatgtgctt ttggcggcgg cggcgcattc tgcctttgcg 60
<210> 35
   <211> 84
   <212> DNA
   <213> Artificial Sequence
<220>
   <223> Description of Artificial Sequence:primer
<400> 35
   gcactaggtc aatctagaat gactatcctt tgctggctgg cccttctgtc aactctgact 60
   gccgtcaacg ctgcggttgt gatg 84
<210> 36
   <211> 70
   <212> DNA
   <213> Artificial Sequence
<220>
   <223> Description of Artificial Sequence:primer
<400> 36
   gcccttggtg ccctcgccgc tgcccggctt gccgctgccg ctggtgctgc cttttatttc 60
   cagcttggtc 70
<210> 37
   <211> 74
   <212> DNA
   <213> Artificial Sequence
<220>
   <223> Description of Artificial Sequence:primer
<400> 37
   ggcagcacca gcggcagcgg caagccgggc agcggcgagg gcagcaccaa gggccaggtt 60
   caactgcagc agtc 74
<210> 38
   <211> 72
   <212> DNA
   <213> Artificial Sequence
<220>
   <223> Description of Artificial Sequence:primer
<400> 38
   gcactaggtc aagcggccgc atgatgatga tgatgatgag aaccccgcat tgcagagaca 60
   gtgaccagag tc 72
<210> 39
   <211> 41
   <212> DNA
   <213> Artificial Sequence
<220>
   <223> Description of Artificial Sequence:primer
<400> 39
   aaaatttgcg gccgcctaat gatgatgatg atgatgagaa c 41
<210> 40
   <211> 462
   <212> DNA
   <213> mouse
<220>
   <221> CDS
   <222> (19)..(462)
<220>
   <221> mat_peptide
   <222> (73)..(462)
<400> 40
   gcactaggtc aatctaga atg act atc ctt tgc tgg cta gcc ctt ctg tca    51
                       Met Thr Ile Leu Cys Trp Leu Ala Leu Leu Ser
                                   -15                 -10
   act ctg act gcc gtc aac gct gcg gtt gtg atg acc cca aac cca ctc    99
   Thr Leu Thr Ala Val Asn Ala Ala Val Val Met Thr Pro Asn Pro Leu
           -5                  -1   1                   5
   tcc ctg cct gtc agt ctt gga gat caa gcc tcc atc tct tgc aga tct   147
   Ser Leu Pro Val Ser Leu Gly Asp Gln Ala Ser Ile Ser Cys Arg Ser
    10                  15                  20                  25
   agt cag agc ctt tta cac agt aat gga atc acc tat tta cat tgg tac   195
   Ser Gln Ser Leu Leu His Ser Asn Gly Ile Thr Tyr Leu His Trp Tyr
                    30                  35                  40
   ctg cag aag cca ggc cag tct cca aag ctc ctg atc tac aaa gtt tcc   243
   Leu Gln Lys Pro Gly Gln Ser Pro Lys Leu Leu Ile Tyr Lys Val Ser
                45                  50                  55
   aac cga ttt tct ggg gtc cca gac agg ttc agt ggc agt gga tca ggg   291
   Asn Arg Phe Ser Gly Val Pro Asp Arg Phe Ser Gly Ser Gly Ser Gly
            60                  65                  70
   aca gat ttc aca ctc aag atc agc aga gtg gag gct gag gat ctg gga   339
   Thr Asp Phe Thr Leu Lys Ile Ser Arg Val Glu Ala Glu Asp Leu Gly
        75                  80                  85
   gtt tat ttc tgc tct caa agt aca cat gtt ccg tac acg ttc gga ggg   387
   Val Tyr Phe Cys Ser Gln Ser Thr His Val Pro Tyr Thr Phe Gly Gly
    90                  95                 100                 105
   ggg acc aag ctg gaa ata aaa ggc agc acc agc ggc agc ggc aag ccg   435
   Gly Thr Lys Leu Glu Ile Lys Gly Ser Thr Ser Gly Ser Gly Lys Pro
                   110                 115                 120
   ggc agc ggc gag ggc agc acc aag ggc                               462
   Gly Ser Gly Glu Gly Ser Thr Lys Gly
               125                 130
<210> 41
   <211> 443
   <212> DNA
   <213> mouse
<220>
   <221> CDS
   <222> (1)..(423)
<400> 41
   ggc agc acc agc ggc agc ggc aag ccg ggc agc ggc gag ggc agc acc    48
   Gly Ser Thr Ser Gly Ser Gly Lys Pro Gly Ser Gly Glu Gly Ser Thr
   1               5                   10                  15
   aag ggc cat gtt caa ctg cag cag tct ggg gct gag ctg gtg agg cct     96
   Lys Gly His Val Gln Leu Gln Gln Ser Gly Ala Glu Leu Val Arg Pro
               20                      25                  30
   ggg gct tca gtg acg ctg tcc tgc aag gct tcg ggc tac aca ttt act    144
   Gly Ala Ser Val Thr Leu Ser Cys Lys Ala Ser Gly Tyr Thr Phe Thr
           35                  40                  45
   gac tat gaa ata cac tgg gtg aag cag aca cct gtg cat ggc ctg gaa    192
   Asp Tyr Glu Ile His Trp Val Lys Gln Thr Pro Val His Gly Leu Glu
       50                  55                  60
   tgg att gga gct att gat cct gaa act ggt ggt act gcc tac aat cag    240
   Trp Ile Gly Ala Ile Asp Pro Glu Thr Gly Gly Thr Ala Tyr Asn Gln
   65                  70                  75                  80
   aag ttc aag gac aag gcc ata gtg act gta gac aaa tcc tcc agc aca    288
   Lys Phe Lys Asp Lys Ala Ile Val Thr Val Asp Lys Ser Ser Ser Thr
                   85                  90                  95
   gcc tac atg gag ctc cgc agc ctg aca tct gaa gac tct gcc gtc tat    336
   Ala Tyr Met Glu Leu Arg Ser Leu Thr Ser Glu Asp Ser Ala Val Tyr
               100                 105                 110
   tac tat aca aga tgg ttt gag gac tgg ggc caa ggg act ctg gtc act    384
   Tyr Tyr Thr Arg Trp Phe Glu Asp Trp Gly Gln Gly Thr Leu Val Thr
           115                 120                 125
   gtc tct gca atg cgg ggt tct cat cat cat cat cat cat gcggccgctt     433
   Val Ser Ala Met Arg Gly Ser His His His His His His
       130                 135                 140
   gacctagtgc                                                          443
<210> 42
   <211> 851
   <212> DNA
   <213> mouse
<220>
   <221> CDS
   <222> (19)..(831)
<220>
   <221> mat_peptide
   <222> (73)..(831)
<400> 42
   gcactaggtc aatctaga atg act atc ctt tgc tgg cta gcc ctt ctg tca   51
                       Met Thr Ile Leu Cys Trp Leu Ala Leu Leu Ser
                                   -15                 -10
   act ctg act gcc gtc aac gct gcg gtt gtg atg acc cca aac cca ctc   99
   Thr Leu Thr Ala Val Asn Ala Ala Val Val Met Thr Pro Asn Pro Leu
           -5                  -1   1               5
   tcc ctg cct gtc agt ctt gga gat caa gcc tcc atc tct tgc aga tct   147
   Ser Leu Pro Val Ser Leu Gly Asp Gln Ala Ser Ile Ser Cys Arg Ser
    10                  15                  20                  25
   agt cag agc ctt tta cac agt aat gga atc acc tat tta cat tgg tac     195
   Ser Gln Ser Leu Leu His Ser Asn Gly Ile Thr Tyr Leu His Trp Tyr
                   30                  35                  40
   ctg cag aag cca ggc cag tct cca aag ctc ctg atc tac aaa gtt tcc     243
   Leu Gln Lys Pro Gly Gln Ser Pro Lys Leu Leu Ile Tyr Lys Val Ser
               45                  50                  55
   aac cga ttt tct ggg gtc cca gac agg ttc agt ggc agt gga tca ggg     291
   Asn Arg Phe Ser Gly Val Pro Asp Arg Phe Ser Gly Ser Gly Ser Gly
           60                  65                  70
   aca gat ttc aca ctc aag atc agc aga gtg gag gct gag gat ctg gga     339
   Thr Asp Phe Thr Leu Lys Ile Ser Arg Val Glu Ala Glu Asp Leu Gly
       75                  80                  85
   gtt tat ttc tgc tct caa agt aca cat gtt ccg tac acg ttc gga ggg     387
   Val Tyr Phe Cys Ser Gln Ser Thr His Val Pro Tyr Thr Phe Gly Gly
   90                  95                  100                 105
   ggg acc aag ctg gaa ata aaa ggc agc acc agc ggc agc ggc aag ccg     435
   Gly Thr Lys Leu Glu Ile Lys Gly Ser Thr Ser Gly Ser Gly Lys Pro
                   110                 115                 120
   ggc agc ggc gag ggc agc acc aag ggc cat gtt caa ctg cag cag tct     483
   Gly Ser Gly Glu Gly Ser Thr Lys Gly His Val Gln Leu Gln Gln Ser
               125                 130                 135
   ggg gct gag ctg gtg agg cct ggg gct tca gtg acg ctg tcc tgc aag     531
   Gly Ala Glu Leu Val Arg Pro Gly Ala Ser Val Thr Leu Ser Cys Lys
           140                 145                 150
   gct tcg ggc tac aca ttt act gac tat gaa ata cac tgg gtg aag cag     579
   Ala Ser Gly Tyr Thr Phe Thr Asp Tyr Glu Ile His Trp Val Lys Gln
       155                 160                 165
   aca cct gtg cat ggc ctg gaa tgg att gga gct att gat cct gaa act     627
   Thr Pro Val His Gly Leu Glu Trp Ile Gly Ala Ile Asp Pro Glu Thr
   170                 175                 180                 185
   ggt ggt act gcc tac aat cag aag ttc aag gac aag gcc ata gtg act     675
   Gly Gly Thr Ala Tyr Asn Gln Lys Phe Lys Asp Lys Ala Ile Val Thr
                   190                 195                 200
   gta gac aaa tcc tcc agc aca gcc tac atg gag ctc cgc agc ctg aca     723
   Val Asp Lys Ser Ser Ser Thr Ala Tyr Met Glu Leu Arg Ser Leu Thr
               205                 210                 215
   tct gaa gac tct gcc gtc tat tac tat aca aga tgg ttt gag gac tgg     771
   Ser Glu Asp Ser Ala Val Tyr Tyr Tyr Thr Arg Trp Phe Glu Asp Trp
           220                 225                 230
   ggc caa ggg act ctg gtc act gtc tct gca atg cgg ggt tct cat cat     819
   Gly Gln Gly Thr Leu Val Thr Val Ser Ala Met Arg Gly Ser His His
       235                 240                 245
   cat cat cat cat gcggccgctt gacctagtgc                               851
   His His His His
   250
<210> 43
   <211> 867
   <212> DNA
   <213> mouse
<220>
   <221> CDS
   <222> (31)..(893)
<220>
   <221> mat_peptide
   <222> (85)..(843)
<400> 43
   aatttaaacg gatcccgggt accttctaga atg act atc ctt tgc tgg cta gcc   54
                                    Met Thr Ile Leu Cys Trp Leu Ala
                                                -15
   ctt ctg tca act ctg act gcc gtc aac gct gcg gtt gtg atg acc cca   102
   Leu Leu Ser Thr Leu Thr Ala Val Asn Ala Ala Val Val Met Thr Pro
   -10                     -5                  -1   1              5
   aac cca ctc tcc ctg cct gtc agt ctt gga gat caa gcc tcc atc tct   150
   Asn Pro Leu Ser Leu Pro Val Ser Leu Gly Asp Gln Ala Ser Ile Ser
               10                  15                  20
   tgc aga tct agt cag agc ctt tta cac agt aat gga atc acc tat tta   198
   Cys Arg Ser Ser Gln Ser Leu Leu His Ser Asn Gly Ile Thr Tyr Leu
           25                  30                  35
   cat tgg tac ctg cag aag cca ggc cag tct cca aag ctc ctg atc tac   246
   His Trp Tyr Leu Gln Lys Pro Gly Gln Ser Pro Lys Leu Leu Ile Tyr
       40                  45                  50
   aaa gtt tcc aac cga ttt tct ggg gtc cca gac agg ttc agt ggc agt   294
   Lys Val Ser Asn Arg Phe Ser Gly Val Pro Asp Arg Phe Ser Gly Ser
    55                     60                  65                  70
   gga tca ggg aca gat ttc aca ctc aag atc agc aga gtg gag gct gag   342
   Gly Ser Gly Thr Asp Phe Thr Leu Lys Ile Ser Arg Val Glu Ala Glu
                       75                  80                  85
   gat ctg gga gtt tat ttc tgc tct caa agt aca cat gtt ccg tac acg   390
   Asp Leu Gly Val Tyr Phe Cys Ser Gln Ser Thr His Val Pro Tyr Thr
               90                  95                  100
   ttc gga ggg ggg acc aag ctg gaa ata aaa ggc agc acc agc ggc agc   438
   Phe Gly Gly Gly Thr Lys Leu Glu Ile Lys Gly Ser Thr Ser Gly Ser
           105                 110                 115
   ggc aag ccg ggc agc ggc gag ggc agc acc aag ggc cat gtt caa ctg   486
   Gly Lys Pro Gly Ser Gly Glu Gly Ser Thr Lys Gly His Val Gln Leu
       120                 125                 130
   cag cag tct ggg gct gag ctg gtg agg cct ggg gct tca gtg acg ctg   534
   Gln Gln Ser Gly Ala Glu Leu Val Arg Pro Gly Ala Ser Val Thr Leu
   135                     140                 145                 150
   tcc tgc aag gct tcg ggc tac aca ttt act gac tat gaa ata cac tgg   582
   Ser Cys Lys Ala Ser Gly Tyr Thr Phe Thr Asp Tyr Glu Ile His Trp
                   155                 160                 165
   gtg aag cag aca cct gtg cat ggc ctg gaa tgg att gga gct att gat      630
   Val Lys Gln Thr Pro Val His Gly Leu Glu Trp Ile Gly Ala Ile Asp
               170                 175                 180
   cct gaa act ggt ggt act gcc tac aat cag aag ttc aag gac aag gcc      678
   Pro Glu Thr Gly Gly Thr Ala Tyr Asn Gln Lys Phe Lys Asp Lys Ala
           185                 190                 195
   ata gtg act gta gac aaa tcc tcc agc aca gcc tac atg gag ctc cgc      726
   Ile Val Thr Val Asp Lys Ser Ser Ser Thr Ala Tyr Met Glu Leu Arg
       200                 205                 210
   agc ctg aca tct gaa gac tct gcc gtc tat tac tat aca aga tgg ttt      774
   Ser Leu Thr Ser Glu Asp Ser Ala Val Tyr Tyr Tyr Thr Arg Trp Phe
   215                 220                 225                 230
   gag gac tgg ggc caa ggg act ctg gtc act gtc tct gca atg cgg ggt      822
   Glu Asp Trp Gly Gln Gly Thr Leu Val Thr Val Ser Ala Met Arg Gly
                   235                 240                 245
   tct cat cat cat cat cat cat taggcggccg ctgcagatct gatc               867
   Ser His His His His His His
               250
<210> 44
   <211> 39
   <212> DNA
   <213> Artificial Sequence
<220>
   <223> Description of Artificial Sequence:primer
<400> 44
   tttaaaggcc atattggcca tgactatcct ttgctggct 39
<210> 45
   <211> 43
   <212> DNA
   <213> Artificial Sequence
<220>
   <223> Description of Artificial Sequence:primer
<400> 45
   tttaaaggcc atattggcca tggatgttgt gatgacccca aac 43
<210> 46
   <211> 54
   <212> DNA
   <213> Artificial Sequence
<220>
   <223> Description of Artificial Sequence:primer
<400> 46
   tttaaaggcc agagaggccc taatgatgat gatgatgatg agaaccccgc attg 54
<210> 47
   <211> 882
   <212> DNA
   <213> mouse
<220>
   <221> CDS
   <222> (25)..(837)
<220>
   <221> mat_peptide
   <222> (79)..(837)
<400> 47
   ctgcagggta cggccatatt ggcc atg act atc ctt tgc tgg ctg gcc ctt      51
                              Met Thr Ile Leu Cys Trp Leu Ala Leu
                                          -15                 -10
   ctg tca act ctg act gcc gtc aac act gcg gtt gtg atg acc cca aac      99
   Leu Ser Thr Leu Thr Ala Val Asn Thr Ala Val Val Met Thr Pro Asn
                   -5                  -1   1               5
   cca ctc tcc ctg cct gtc agt ctt gga gat caa gcc tcc atc tct tgc     147
   Pro Leu Ser Leu Pro Val Ser Leu Gly Asp Gln Ala Ser Ile Ser Cys
               10                  15                  20
   aga tct agt cag agc ctt tta cac agt aat gga atc acc tat tta cat     195
   Arg Ser Ser Gln Ser Leu Leu His Ser Asn Gly Ile Thr Tyr Leu His
       25                  30                  35
   tgg tac ctg cag aag cca ggc cag tct cca aag ctc ctg atc tac aaa     243
   Trp Tyr Leu Gln Lys Pro Gly Gln Ser Pro Lys Leu Leu Ile Tyr Lys
   40                  45                  50                  55
   gtt tcc aac cga ttt tct ggg gtc cca gac agg ttc agt ggc agt gga     291
   Val Ser Asn Arg Phe Ser Gly Val Pro Asp Arg Phe Ser Gly Ser Gly
                   60                  65                  70
   tca ggg aca gat ttc aca ctc aag atc agc aga gtg gag gct gag gat     339
   Ser Gly Thr Asp Phe Thr Leu Lys Ile Ser Arg Val Glu Ala Glu Asp
               75                  80                  85
   ctg gga gtt tat ttc tgc tct caa agt aca cat gtt ccg tac acg ttc     387
   Leu Gly Val Tyr Phe Cys Ser Gln Ser Thr His Val Pro Tyr Thr Phe
           90                  95                  100
   gga ggg ggg acc aag ctg gaa ata aaa ggc agc acc agc ggc agc ggc     435
   Gly Gly Gly Thr Lys Leu Glu Ile Lys Gly Ser Thr Ser Gly Ser Gly
       105                 110                 115
   aag ccg ggc agc ggc gag ggc agc acc aag ggc cat gtt caa ctg cag     483
   Lys Pro Gly Ser Gly Glu Gly Ser Thr Lys Gly His Val Gln Leu Gln
   120                 125                 130                 135
   cag tct ggg gct gag ctg gtg agg cct ggg gct tca gtg acg ctg tcc     531
   Gln Ser Gly Ala Glu Leu Val Arg Pro Gly Ala Ser Val Thr Leu Ser
                   140                 145                 150
   tgc aag gct tcg ggc tac aca ttt act gac tat gaa ata cac tgg gtg     579
   Cys Lys Ala Ser Gly Tyr Thr Phe Thr Asp Tyr Glu Ile His Trp Val
               155                 160                 165
   agg cag aca cct gtg cat ggc ctg gaa tgg att gga gct att gat cct     627
   Arg Gln Thr Pro Val His Gly Leu Glu Trp Ile Gly Ala Ile Asp Pro
           170                 175                 180
   gaa act ggt ggt act gcc tac aat cag aag ttc aag gac aag gcc ata     675
   Glu Thr Gly Gly Thr Ala Tyr Asn Gln Lys Phe Lys Asp Lys Ala Ile
       185                 190                 195
   gtg act gta gac aaa tcc tcc agc aca gcc tac atg gag ctc cgc agc     723
   Val Thr Val Asp Lys Ser Ser Ser Thr Ala Tyr Met Glu Leu Arg Ser
   200                 205                 210                 215
   ctg aca tct gaa gac tct gcc gtc tat tac tat aca aga tgg ttt gag     771
   Leu Thr Ser Glu Asp Ser Ala Val Tyr Tyr Tyr Thr Arg Trp Phe Glu
                   220                 225                 230
   gac tgg ggc caa ggg act ctg gtc act gtc tct gca atg cgg ggt tct     819
   Asp Trp Gly Gln Gly Thr Leu Val Thr Val Ser Ala Met Arg Gly Ser
               235                 240                 245
   cat cat cat cat cat cat tagggcctct ctggccgatc ccccgaattt           867
   His His His His His His
           250
   ccccgatcgt tcaaa                                                    882
<210> 48
   <211> 846
   <212> DNA
   <213> mouse
<220>
   <221> CDS
   <222> (25)..(786)
<400> 48
   ctgcagggta cggccatatt ggcc atg gat gtt gtg atg acc cca aac cca     51
                              Met Asp Val Val Met Thr Pro Asn Pro
                               1               5
   ctc tcc ctg cct gtc agt ctt gga gat caa gcc tcc atc tct tgc aga    99
   Leu Ser Leu Pro Val Ser Leu Gly Asp Gln Ala Ser Ile Ser Cys Arg
    10                  15                  20                  25
   tct agt cag agc ctt tta cac agt aat gga atc acc tat tta cat tgg    147
   Ser Ser Gln Ser Leu Leu His Ser Asn Gly Ile Thr Tyr Leu His Trp
                    30                  35                  40
   tac ctg cag aag cca ggc cag tct cca aag ctc ctg atc tac aaa gtt    195
   Tyr Leu Gln Lys Pro Gly Gln Ser Pro Lys Leu Leu Ile Tyr Lys Val
                45                  50                  55
   tcc aac cga ttt tct ggg gtc cca gac agg ttc agt ggc agt gga tca    243
   Ser Asn Arg Phe Ser Gly Val Pro Asp Arg Phe Ser Gly Ser Gly Ser
            60                  65                  70
   ggg aca gat ttc aca ctc aag atc agc aga gtg gag gct gag gat ctg    291
   Gly Thr Asp Phe Thr Leu Lys Ile Ser Arg Val Glu Ala Glu Asp Leu
        75                  80                  85
   gga gtt tat ttc tgc tct caa agt aca cat gtt ccg tac acg ttc gga    339
   Gly Val Tyr Phe Cys Ser Gln Ser Thr His Val Pro Tyr Thr Phe Gly
    90                  95                 100                 105
   ggg ggg acc aag ctg gaa ata aaa ggc agc acc agc ggc agc ggc aag    387
   Gly Gly Thr Lys Leu Glu Ile Lys Gly Ser Thr Ser Gly Ser Gly Lys
                   110                 115                 120
   ccg ggc agc ggc gag ggc agc acc aag ggc cat gtt caa ctg cag cag    435
   Pro Gly Ser Gly Glu Gly Ser Thr Lys Gly His Val Gln Leu Gln Gln
               125                 130                 135
   tct ggg gct gag ctg gtg agg cct ggg gct tca gtg acg ctg tcc tgc    483
   Ser Gly Ala Glu Leu Val Arg Pro Gly Ala Ser Val Thr Leu Ser Cys
           140                 145                 150
   aag gct tcg ggc tac aca ttt act gac tat gaa ata cac tgg gtg agg    531
   Lys Ala Ser Gly Tyr Thr Phe Thr Asp Tyr Glu Ile His Trp Val Arg
       155                 160                 165
   cag aca cct gtg cat ggc ctg gaa tgg att gga gct att gat cct gaa    579
   Gln Thr Pro Val His Gly Leu Glu Trp Ile Gly Ala Ile Asp Pro Glu
   170                 175                 180                 185
   act ggt ggt act gcc tac aat cag aag ttc aag gac aag gcc ata gtg    627
   Thr Gly Gly Thr Ala Tyr Asn Gln Lys Phe Lys Asp Lys Ala Ile Val
                   190                 195                 200
   act gta gac aaa tcc tcc agc aca gcc tac atg gag ctc cgc agc ctg    675
   Thr Val Asp Lys Ser Ser Ser Thr Ala Tyr Met Glu Leu Arg Ser Leu
               205                 210                 215
   aca tct gaa gac tct gcc gtc tat tac tat aca aga tgg ttt gag gac    723
   Thr Ser Glu Asp Ser Ala Val Tyr Tyr Tyr Thr Arg Trp Phe Glu Asp
           220                 225                 230
   tgg ggc caa ggg act ctg gtc act gtc tct gca atg cgg ggt tct cat    771
   Trp Gly Gln Gly Thr Leu Val Thr Val Ser Ala Met Arg Gly Ser His
       235                 240                 245
   cat cat cat cat cat tagggcctct ctggccgatc ccccgaattt ccccgatcgt    826
   His His His His His
   250
   tcaaacattt ggcaataaag                                              846
<210> 49
   <211> 738
   <212> DNA
   <213> mouse
<220>
   <221> CDS
   <222> (19)..(687)
   <223> mature peptide is coded by nucleotides 49 to 687
<400> 49
   cccgggtacc cttctaga ctc gtg aca gtt gtt gat ggt gcc caa tcc cag     51
                       Leu Val Thr Val Val Asp Gly Ala Gln Ser Gln
                        1               5                  10
   gtt caa ctg cag cag tct ggg gct gag ctg gtg agg cct ggg gct tca     99
   Val Gln Leu Gln Gln Ser Gly Ala Glu Leu Val Arg Pro Gly Ala Ser
               15                  20                  25
   gtg acg ctg tcc tgc aag gct tcg ggc tac aca ttt act gac tat gaa    147
   Val Thr Leu Ser Cys Lys Ala Ser Gly Tyr Thr Phe Thr Asp Tyr Glu
           30                  35                  40
   ata cac tgg gtg agg cag aca cct gtg cat ggc ctg gaa tgg att gga    195
   Ile His Trp Val Arg Gln Thr Pro Val His Gly Leu Glu Trp Ile Gly
       45                  50                  55
   gct att gat cct gaa act ggt ggt act gcc tac aat cag aag ttc aag    243
   Ala Ile Asp Pro Glu Thr Gly Gly Thr Ala Tyr Asn Gln Lys Phe Lys
   60                  65                  70                  75
   gac aag gcc ata gtg act gta gac aaa tcc tcc agc aca gcc tac atg    291
   Asp Lys Ala Ile Val Thr Val Asp Lys Ser Ser Ser Thr Ala Tyr Met
                   80                  85                  90
   gag ctc cgc agc ctg aca tct gaa gac tct gcc gtc tat tac tat aca    339
   Glu Leu Arg Ser Leu Thr Ser Glu Asp Ser Ala Val Tyr Tyr Tyr Thr
               95                 100                 105
   aga tgg ttt gag gac tgg ggc caa ggg act ctg gtc act gtc tct gca    387
   Arg Trp Phe Glu Asp Trp Gly Gln Gly Thr Leu Val Thr Val Ser Ala
           110                 115                 120
   gcc aaa aca aca ccc cca tca gtc tat cca ctg gcc cct ggg tgt gga    435
   Ala Lys Thr Thr Pro Pro Ser Val Tyr Pro Leu Ala Pro Gly Cys Gly
       125                 130                 135
   gat aca act ggt tcc tct gtg act ctg gga tgc ctg gtc aag ggc tac    483
   Asp Thr Thr Gly Ser Ser Val Thr Leu Gly Cys Leu Val Lys Gly Tyr
   140                 145                 150                 155
   ttc cct gag tca gtg act gtg act tgg aac tct gga tcc ctg tcc agc    531
   Phe Pro Glu Ser Val Thr Val Thr Trp Asn Ser Gly Ser Leu Ser Ser
                   160                 165                 170
   agt gtg cac acc ttc cca gct ctc ctg cag tct gga ctc tac act atg    579
   Ser Val His Thr Phe Pro Ala Leu Leu Gln Ser Gly Leu Tyr Thr Met
               175                 180                 185
   agc agc tca gtg act gtc ccc tcc agc acc tgg cca agt cag acc gtc    627
   Ser Ser Ser Val Thr Val Pro Ser Ser Thr Trp Pro Ser Gln Thr Val
           190                 195                 200
   acc tgc agc gtt gct cac cca gcc agc agc acc acg gtg gac aaa aaa    675
   Thr Cys Ser Val Ala His Pro Ala Ser Ser Thr Thr Val Asp Lys Lys
       205                 210                 215
   ctt gag ccc agc gcggccgctg caggtcttga tcctttcctg ggacccggca        727
   Leu Glu Pro Ser
   220
   agaaccaaaa a 738
<210> 50
   <211> 42
   <212> DNA
   <213> mouse
<400> 50
   gcactaggtc aagcggccgc ttactaacac tcattcctgt tg 42
<210> 51
   <211> 753
   <212> DNA
   <213> mouse
<220>
   <221> CDS
   <222> (7)..(705)
<220>
   <221> mat_peptide
   <222> (67)..(705)
<400> 51
   tctaga atg gta agc gct att gtt tta tat gtg ctt ttg gcg gcg gcg      48
          Met Val Ser Ala Ile Val Leu Tyr Val Leu Leu Ala Ala Ala
          -20                 -15                 -10
   gcg cat tct gcc ttt gcg gcg gtt caa ctg cag cag tct ggg gct gag     96
   Ala His Ser Ala Phe Ala Ala Val Gln Leu Gln Gln Ser Gly Ala Glu
       -5                  -1   1               5                  10
   ctg gtg agg cct ggg gct tca gtg acg ctg tcc tgc aag gct tcg ggc    144
   Leu Val Arg Pro Gly Ala Ser Val Thr Leu Ser Cys Lys Ala Ser Gly
                    15                  20                  25
   tac aca ttt act gac tat gaa ata cac tgg gtg agg cag aca cct gtg    192
   Tyr Thr Phe Thr Asp Tyr Glu Ile His Trp Val Arg Gln Thr Pro Val
                30                  35                  40
   cat ggc ctg gaa tgg att gga gct att gat cct gaa act ggt ggt act    240
   His Gly Leu Glu Trp Ile Gly Ala Ile Asp Pro Glu Thr Gly Gly Thr
            45                  50                  55
   gcc tac aat cag aag ttc aag gac aag gcc ata gtg act gta gac aaa    288
   Ala Tyr Asn Gln Lys Phe Lys Asp Lys Ala Ile Val Thr Val Asp Lys
        60                  65                  70
   tcc tcc agc aca gcc tac atg gag ctc cgc agc ctg aca tct gaa gac    336
   Ser Ser Ser Thr Ala Tyr Met Glu Leu Arg Ser Leu Thr Ser Glu Asp
    75                  80                  85                  90
   tct gcc gtc tat tac tat aca aga tgg ttt gag gac tgg ggc caa ggg    384
   Ser Ala Val Tyr Tyr Tyr Thr Arg Trp Phe Glu Asp Trp Gly Gln Gly
                    95                 100                 105
   act ctg gtc act gtc tct gca gcc aaa aca aca ccc cca tca gtc tat    432
   Thr Leu Val Thr Val Ser Ala Ala Lys Thr Thr Pro Pro Ser Val Tyr
               110                 115                 120
   cca ctg gcc cct ggg tgt gga gat aca act ggt tcc tct gtg act ctg   480
   Pro Leu Ala Pro Gly Cys Gly Asp Thr Thr Gly Ser Ser Val Thr Leu
           125                 130                 135
   gga tgc ctg gtc aag ggc tac ttc cct gag tca gtg act gtg act tgg   528
   Gly Cys Leu Val Lys Gly Tyr Phe Pro Glu Ser Val Thr Val Thr Trp
       140                 145                 150
   aac tct gga tcc ctg tcc agc agt gtg cac acc ttc cca gct ctc ctg   576
   Asn Ser Gly Ser Leu Ser Ser Ser Val His Thr Phe Pro Ala Leu Leu
   155                 160                 165                 170
   cag tct gga ctc tac act atg agc agc tca gtg act gtc ccc tcc agc   624
   Gln Ser Gly Leu Tyr Thr Met Ser Ser Ser Val Thr Val Pro Ser Ser
                   175                 180                 185
   acc tgg acc tgc agc gtt gct cac cca gcc agc agc cca agt cag acc   672
   Thr Trp Thr Cys Ser Val Ala His Pro Ala Ser Ser Pro Ser Gln Thr
               190                 195                 200
   gtc acc acg gtg gac aaa aaa ctt gag ccc agc tagtaatgag cggccgctgc 725
   Val Thr Thr Val Asp Lys Lys Leu Glu Pro Ser
           205                 210
   agatctgatc ctttcctggg acccggca                                    753
<210> 52
   <211> 19
   <212> DNA
   <213> Artificial Sequence
<220>
   <223> Description of Artificial Sequence:nt 2253 to 2271 of pDAB439
<400> 52
   tgcatgtgtt ctccttttt 19
<210> 53
   <211> 44
   <212> DNA
   <213> Artificial Sequence
<220>
   <223> Description of Artificial Sequence:nt 4256 to 4308 of pDAB439
<400> 53
   ggtacggcca tattggccga gctcggcctc tctggccgat cccc 44
<210> 54
   <211> 63
   <212> DNA
   <213> Artificial Sequence
<220>
   <223> Description of Artificial Sequence:nt 4744 to 4807 of pDAB439
<400> 54
   gcggccgctt taacgcccgg gcatttaaat ggcgcgccgc gatcgcttgc agatctgcat 60
   ggg 63
<210> 55
   <211> 20
   <212> DNA
   <213> Artificial Sequence
<220>
   <223> Description of Artificial Sequence:nt 5417 to 5436 of pDAB439
<400> 55
   ggggactcta gaggatccag 20
<210> 56
   <211> 24
   <212> PRT
   <213> Zea mays
<400> 56
   Pro Gly Ser Pro Ala Pro Ala Ala Pro Lys Asn Gly Leu Gly Glu Arg
     1               5                  10                  15
   Pro Glu Ser Leu Asp Val Arg Gly
                20

## Description

### FIELD OF THE INVENTION

This invention relates to the preparation and use of nucleic acid fragments or genes encoding monoclonal antibodies immunologically reactive to transit peptides and the use of said genes to create transgenic plants having altered protein levels.

### BACKGROUND OF THE INVENTION

The modification of plants by genetic engineering has been of considerable interest in recent years. To date, several different plant species have been modified by introducing new genes encoding proteins having enzymatic activity. In essence, presence of these enzymes encoded by transgenes results in the production of proteins which can alter existing plant properties and/or produce new and enhanced characteristics. When said proteins are expressed, new or modified enzymatic activities not previously endogenous to the plant can be observed, or levels of proteins found therein can be increased.

Another genetic approach that has been used to modify plant species is down-regulation of a particular plant protein. Down-regulation can result in either partial or complete elimination of a particular enzymatic activity of choice by decreasing overall steady state levels of the protein associated therewith. A common down-regulation approach used to modify a desired plant enzyme level or activity is antisense RNA technology. Antisense RNA results in down-regulation at the RNA translational level. Down-regulation by antisense RNA, as described in US Patent 5,107,065 to Calgene, has been shown effective with a variety of plant genes as described by Shimada et al., (1993) Theor. Appl. Genet. 86:665-672; Kull, et al., (1995) J. Genet. Breed. 49:67-76; Slabas and Elborough, (1997) WO 97/07222 to Zeneca, published 2/27/97; and Knutzon et al., (1992) Proc. Natl. Acad. Sci USA 89:2624-2628.

Another down regulation approach involves the use of ribozyme technology as described by Hasselhoff and Gerlach, (1988) Nature 342:76-79. Ribozyme technology, like antisense methodologies, also works at the RNA translational level and involves making catalytic RNA molecules which bind to and cleave the mRNA of interest. Ribozymes have recently been demonstrated as an effective method for the down-regulation of plant proteins as described in WO97/10328 to Ribozyme Pharmaceuticals and Dow AgroSciences, LLC, formally DowElanco.

Co-suppression, as described by Seymour et al., (1993, Plant Mol. Biol. 23:1-9) is another approach applicable for down-regulation of plant gene expression. At present, the precise mechanism of down-regulation via co-suppression is not known. However, it has been used extensively to produce transgenic plants having modified gene expression levels as described in Brusslan et al., (1993) Plant Cell 5:667-677; Vaucheret et al., (1995) Mol. Gen. Genet. 248:311-317; and Jorgensen et al., (196) Plant Mol. Biol. 31:957-973.

As disclosed herein, Applicants have invented an alternative approach to down-regulate proteins in plants relying on the use of monoclonal antibodies (MAb) and functional fragments thereof, such as single chain antibodies (SCAb) that recognize and bind transit peptides. As a result, steady-state levels of corresponding passenger proteins can be reduced. The proposed approach is further exemplified, as shown in the non-limiting examples, through down-regulation of maize stearoyl-ACP Δ-9 desaturase.

In many situations it will be desirable to modify an existing trait of a plant cell rather than introduce a new trait. Thus, one may wish to modify the overall activity levels of a particular enzyme, provide for preferential accumulation of one allele as compared to another, one isozyme as compared to another or the like. In other instances, one may only wish to reduce the amount of expression of a protein encoded by a gene rather than inhibit expression entirely. It is therefore of interest to use the invention disclosed herein which will allow for directed modification of specific proteins in particular plant cells, plant tissues, or plants.

### SUMMARY OF THE INVENTION

In the present invention, monoclonal antibodies have been generated against transit peptides that direct plant passenger proteins to organelles. Steady state levels of passenger proteins in plant cells can be altered by expressing antibodies which immunologically react to said transit peptides found attached to said passenger proteins in the precursor form.

Thus, according to one aspect the present invention provides a nucleic acid construct comprising in the 5' to 3' direction of transcription, a promoter functional in a plant cell, a nucleic acid sequence that encodes an antibody or fragment thereof having the ability to bind to a transit peptide that directs a passenger protein associated with the transit peptide to an organelle of a plant cell, and a termination region functional in a plant cell.

According to another aspect, the invention provides a method of decreasing the steady state level of a passenger protein in a plant cell which comprises placing a nucleic acid construct of the first aspect in the plant cell.

According to a further aspect, a plant cell is provided comprising a nucleic acid construct according to the first aspect, and in which cell the steady state level of a passenger protein found therein has thereby been decreased. In addition, the present invention provides plants or progeny thereof derived from such a plant cell.

In the above aspects, the plant cell may be a dicotyledon, or a monocotyledon. In a preferred embodiment, the plant is maize, and the transit peptide is the transit peptide for maize stearoyl-ACP Δ-9 desaturase or maize palmitoyi-ACP thioesterase.

The organelle may be selected from the group consisting of chloroplast, amyloplast, chromoplast, leucoplast, mitochrondria, and the nucleus.

In a preferred embodiment, the antibody or fragment thereof is a single chain antibody (SCAb). SCAbs can be obtained by modifying the genes encoding a Monoclonal antibody to express a SCAb derived therefrom. Said SCAbs can exhibit binding and antigen affinity similar to said monoclonal antibodies, but are encoded by a single gene.

Preferably, the transit peptide epitope to be recognized by the antibody or fragement thereof comprises at least 6 amino acids, said amino acids being continuously adjacent to each other in said transit peptide.

In a preferred embodiment, the nucleic acid sequence, that encodes an antibody or fragment thereof, is selected from the group consisting of SEQ ID NO:21, SEQ ID NO:22, SEQ ID NO:24, SEQ ID NO:25, SEQ ID NO:31, SEQ ID NO:43, and SEQ ID NO:48.

According to a further aspect, the present invention provides a hybridoma cell line designated 10E10, having ATTC Designation HB-12544. In addition, the present invention provides for antibodies produced by said hybridoma cell line.

### Detailed Description of the Invention

The present invention relates to methods and compositions for obtaining transgenic plants having altered steady state passenger protein levels due to expression of antibodies being immunologically reactive to transit peptides. The following phrases and terms are defined below:

By "altered steady state" is meant that the total level of a particular passenger plant protein having a transit peptide in a modified plant is different from that of a normal or non-modified plant under similar conditions. Decreased steady state levels can be achieved by expressing antibodies immunologically reactive to said transit peptide.

By "antibody" is meant a protein molecule having functional activity comprising two identical polypeptide chains of about 600 amino acid residues (usually referred to as heavy chains, H) covalently attached to each other by disulfide bonds, and two identical shorter polypeptide chains of about 220 amino acid residues (usually referred to as the light chains, L) each light chain being covalently attached to each other by disulfide bonds. The term antibody also includes immunologically-active fragments of the above described tetramer, as well as fragments, segments, proteolytically cleaved portions or recombinantly-prepared portions that still maintain antigen binding capacity as disclosed herein.

By "cDNA" is meant DNA that is complementary to and derived from a mRNA.

By "chimeric DNA construction" is meant a recombinant DNA containing genes or portions thereof from one or more species.

By "constitutive promoter" is meant promoter elements that direct continuous gene expression in all cell types and at all times (i.e., actin, ubiquitin, CaMV 35S, 35T, and the like).

By "cosuppression" is meant the introduction of a foreign gene having substantial homology to an endogenous gene, and in a plant cell can cause reduction in activity of the foreign gene and/or the endogenous protein product.

By "developmental specific" promoter is meant promoter elements responsible for gene expression at specific plant developmental stages, such as in early or late embryogenesis.

By "enhancer" is meant nucleotide sequence elements which can stimulate promoter activity such as those from maize streak virus (MSV) and alcohol dehydrogenase intron 1.

By "expression" as used herein, is meant the transcription and stable accumulation of the antibody RNA inside a plant cell.

By "foreign" or "heterologous gene" is meant a gene encoding a protein whose exact amino acid sequence is not normally found in the host cell, but is introduced therein.

By "functional" fragments, and "functional antibodies" is meant that the antibody or fragments thereof retain their ability to bind epitopes used in the immunization process and production of said antibody.

By "gene" is meant to include all genetic material involved in protein expression including chimeric DNA constructions, genes, plant genes and portions thereof.

By "genome" is meant genetic material contained in each cell of an organism and/or virus.

By "indigenous" gene is meant the gene encoding the precursor protein, passenger protein, or transit peptide as found in the native organism. Typically, the transit peptide and passenger protein are found together in the source from which the gene was isolated, although not required to be within the scope of the invention. A non-limiting example of an indigenous gene would be the gene encoding the precursor protein maize Δ-9 desaturase, said gene being found in maize.

By "inducible promoter" is meant promoter elements which are responsible for expression of genes in response to a specific signal, such as: physical stimuli (heat shock genes); light (RUBP carboxylase); hormone (Em); metabolites and stress.

By "mature protein" or "passenger protein" is meant the protein which is found after processing and passing into an organelle. Passenger proteins are originally made in a precursor form that includes a transit peptide and said passenger protein. Upon entry into an organelle, the transit peptide portion is cleaved, thus leaving the "passenger" or "mature" protein. Passenger protein are the proteins typically obtained upon purification from an homogenate, as described herein. The sequence of passenger proteins can be determined as described herein. Passenger proteins can be indigenous in that they are naturally found in the organelle of interest. Passenger proteins can also be non-indigenous in that a plant may be transformed using a heterologous gene encoding a passenger protein not naturally found therein.

By "modified plant" is meant a plant wherein the protein levels or total protein specific activity levels of a passenger protein have been altered relative to that seen in a unmodified plant due to functional activity of an antibody.

By "nucleic acids" is meant to include all forms of DNA and RNA.

By "organelle" or "plant organelle" is meant to include chloroplasts, chromoplast, leucoplast, amyloplast, mitochondria, the nucleus, and the like.

By "phenotypic change" is meant to include alterations in steady state protein levels in a transformed or modified plant due to the expression of an antibody or fragments thereof binding to a organeller transit peptide.

By "plant" is meant a photosynthetic organism including both eukaryotes and prokaryotes.

By "precursor" protein is meant a protein having a transit peptide and a passenger protein covalently attached to each other. The passenger protein and transit peptide can be homologous to each other in that they are encoded in a manner isolated from nature. A non-limiting example could be the native maize transit peptide of Δ-9 desaturase covalently linked to the native maize Δ-9 desaturase passenger protein. In addition, the transit peptide and passenger protein can be heterologous to each other. A non-limiting example could be the native transit peptide for Δ-9 desaturase heterologously attached to the passenger protein gene encoding chlorophyll a/b binding protein.

By "promoter regulatory element" is meant nucleotide sequence elements within a nucleic fragment or gene which control the expression of that nucleic acid fragment or gene. Promoter sequences provide the recognition for RNA polymerase and other transcriptional factors required for efficient transcription. Promoter regulatory elements are also meant to include constitutive, tissue-specific, developmental-specific, inducible promoters and the like. Promoter regulatory elements may also include certain enhancer sequence elements that improve transcriptional efficiency. In addition, promoters and promoter regulatory elements, as used herein, are meant to include subgenomic promoters found in +sense, single-stranded RNA viruses such as tobacco mosaic virus and the like.

By "tissue-specific" promoter is meant promoter elements responsible for gene expression in specific cell or tissue types, such as the leaves or seeds (i.e., zein, oleosin, napin, ACP, globulin and like).

By "transgenic plant" is meant a plant expressing a chimeric gene introduced through transformation efforts.

By "transit peptide" or "signal peptide" is meant those amino acids that direct a passenger protein to a particular organelle as defined herein. It is within the scope of this invention that the transit peptide can be either indigenous or non-indigenous to the plant cell of interest. In addition, as further described herein, the transit peptide can be either homologous or heterologous to the passenger protein of interest in that it is natively found with said passenger protein as isolated from nature (homologous) or not (heterologous).

The present invention is directed to down regulation of organeller proteins by expression of antibodies or functional fragments thereof that are immunologically reactive to transit peptides. Down regulation can result in decreased steady state levels of the passenger proteins or enzymatic activity levels of said passenger proteins. Passenger protein may be homologous or heterologous with respect to the transit peptide. Furthermore, transit peptides may be homologous or heterologous with respect to passenger proteins. Both transit peptides and passenger proteins may be, either together or separately, indigenous or non-indigenous to the plant cell in which they are found.

In plants, chloroplast, mitochondria, the nucleus and other organelles as defined herein, use transit peptides to direct passenger proteins to the appropriate organelle. Transit peptides can be used as antigens, as disclosed herein, in the production of functional antibodies immunoreactive thereto. Said functional antibodies can then be expressed within the cell whereby they can bind to said transit peptides and prevent accumulation of the passenger protein in the organelle and hence the cell. While chloroplast, mitochondria, and the nucleus are the primary organelles found in plant tissue, cells are also likely to have other kinds of organelles including amyloplasts, chromoplast, leucoplast and the like, all of which use transit peptides for protein localization. Therefore, protein uptake can be inhibited in plant organelles by practicing the invention as disclosed herein.

As further disclosed herein, expression of functional antibodies within plant cells which can bind transit peptides can result in decreased steady-state levels of the passenger protein associated therewith when compared to non-transformed plants. Preferably, steady state levels of passenger proteins can be decreased from about 10% to about 90%; more preferably from about 50% to about 90%; with steady state levels of passenger proteins being decreased about 90% compared to non-modified being most preferred.

As further described herein, amino acid, gene, and nucleic acid fragment sequence encoding transit peptides can be used to produced antibodies which can then be used in transgenic applications to produce plants having altered steady-state protein levels. The invention is exemplified by determining the transit peptide sequence of maize stearoyl-ACP Δ-9 desaturase (Δ-9 desaturase) followed by producing, cloning, and expressing functional antibodies having an affinity to the same. In some cases, transit peptide sequences can be determined by comparison of the gene and amino acid sequence encoding for the complete precursor protein as described herein. Preferably, genes and nucleic acid fragments encoding for passenger proteins and the use of said genes to determine nucleic acid fragments and genes encoding transit peptides are isolated from plants. More preferred are those amino acid, gene, and nucleic acid fragments disclosed herein as SEQ ID NO:1, SEQ ID NO:2, SEQ ID NO:3, SEQ ID NO: 4, SEQ ID NO: 5, SEQ ID NO:6, SEQ ID NO: 7, SEQ ID NO:8, SEQ ID NO: 9, SEQ ID NO:10, SEQ ID NO:11, SEQ ID NO:12, SEQ ID NO:13 SEQ ID NO:14 and SEQ ID NO:15.

Hybridoma cell lines producing monoclonal antibodies can be obtained as disclosed herein. Said cell line most preferred is 10E10 (Mab-Tp1), which was deposited in accordance with the terms of the Budapest Treaty at the American Type Culture Collection, 10801 University Boulevard, Manassas, Virginia 20110-2209, on June 18, 1998(ATTC Designation HB-12544). Monoclonal antibodies which are immunoreactive to the transit peptides disclosed herein can be produced from hybridoma cell lines. Preferably, the amino acid sequence, gene sequence and nucleic acid fragments encoding precursor proteins, transit peptides, and passenger proteins can be determined. Most preferred are those amino acid, gene and nucleic acid sequences disclosed herein as SEQ ID NO:21, SEQ ID NO:22, SEQ ID NO:24, SEQ ID NO:25, SEQ ID NO:26, SEQ ID NO:27 SEQ ID NO:28 SEQ ID NO:29, SEQ ID NO:30, SEQ ID NO:31, SEQ ID NO:32, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50 and SEQ ID NO:51.

As disclosed herein, functional antibodies against transit peptides which direct passenger proteins to organelles can be identified and produced. The amino acid sequence and hence the nucleic acid sequence of a transit peptide can be determined in a variety of ways available to the skilled artisan. For example, passenger proteins of interest can be purified using a variety of techniques as disclosed herein. Once purified, amino terminal sequencing of said protein can be determined. The gene encoding said protein can then be cloned. Comparison of the amino acid sequence determined by and from the cDNA to that obtained from amino terminal sequence of the passenger protein will allow determination of the transit peptide sequence. In addition, many transit peptide sequences are available in the art and can easily be obtained from GenBank located at the National Center for Biotechnology Information web site. In plants, transit peptides are typically cleaved and degraded upon entry of the passenger protein into the organelle of interest, therefore, purification of transit peptides from plant tissues is typically not possible.

Transit peptides may provide intracellular transport to plastids and mitochondria. The subject of transit peptides in plants has been extensively reviewed by Keegstra et al., (1989) Cell, 56:247-253). Typically, there is very little primary amino acid sequence homology between different plant transit peptides. In addition, even though passenger proteins may have amino acid and nucleic acid sequence similarities between cultivars, lines, and species, transit peptides obtained therefrom may show very little amino acid or DNA sequence homology. Furthermore, the length of transit peptides can vary, with some passenger proteins having transit peptides with as little as about 30 amino acid while other can be about 150 amino acids or longer. Nearly all transit peptide amino acid sequences nearly all start with Met-Ala at the amino terminus. The carboxy terminus can often be determined by inspection in that cleavage most often occurs at or near the motif Val-Ala-Val-Val , Val-Ala-Ala-Val or variations thereof. In many cases, transit peptides may also contain further information for organeller targeting. Depending of the source of the transit peptide, precursor proteins may display differences in import behavior, activity and efficiency. These differences are thought not to be only due solely to the transit peptide but also to passenger proteins and interaction associated therewith. Additional descriptions of transit peptide characteristics in plants and mechanisms associated therewith can be found in Pfanner et al., (1988) Eur. J. Biochem, 175:205-212; Ko and Ko, (1992) J. Biol. Chem. 267, 13910-13916; Pfanner et al., (1988) TIBS 13:165-167; Bascomb et al. (1992) Plant Microb. Biotechnol. Res. Ser. 1:142-163; and Bukau et al., (1996) Trends in Cell Biol. 6:480-486.

Transit peptides may also be used to import proteins into the nucleus. Antibodies can be made against said peptides and used to lower steady state levels of the passenger proteins as disclosed herein. Nuclear transit peptides are typically comprised of short polypeptide regions and can be classified as one of three distinct types. For example, one type possesses a single short region enriched in basic amino acids. Another type is composed of two basic regions separated by a spacer. Finally, a third type possesses both hydrophobic and basic amino acids. Nuclear transit peptides in plants are further described in Silver (1991) Cell 64:489-497; Hicks et al., (1995) Plant Physiol. 107:1055-1058; Ballas and Citovsky, (1997) Proc. Natl. Acad. Sci. USA, 94:10723-10728; Citovsky et al., (1994) Proc. Natl. Acad. Sci. USA (91): 3210-3214; Lyck et al., (1997) Planta, 202:117-125; Reiss et al., (1997) Mol. Gen. Genet. 253:695-702; Sakamoto et al., (1996) Plant J., 10:859-868; and Raikhel (1992) Plant Physiol. 100:1627-1632.

As further described herein, transit peptides once identified can be produced using a variety of methods. Such methods can include heterologous expression in an appropriate system such as *E*. *coli,* Baculovirus, yeast and the like. The preferred method of peptide production is through chemical synthesis using either *t-*butyloxycarbonyl(*t*-BOC) or 9-fluorenylmethoxycarbonyl (FMOC) as described in Baraby and Merrifield (1980) In The Peptides Vol. 2 (E. Gross and Meienhofer, eds) pp.1-284, Academic Press, San Diego, CA; and Stewart and Young, (1984) Solid Phase Peptide Synthesis, 2nd ed. Pierce Chemical Co., Rockford, IL.

It is not necessary for the entire transit peptide to be used in antibody production to be within the scope of this invention. Generally, peptides with a length of about 10 to about 15 residues can be used; however, peptides with as few as about 6 and up to about 150 amino acids or more are considered to be within the scope of the invention. Typically, polypeptide fragments rich in hydrophilic amino acids induce the best immunological response. Said hydrophilic amino acids include arginine, aspartic acid, glutamic acid, lysine, serine, asparagine, glutamine, glycine, and proline. However, polypeptide fragments with other amino acids can be used as well.

Antibodies bind to epitope sites based on amino acid/amino acid interactions and are thus highly specific for individual epitope sites. However, as stated herein, there is a distinct lack of amino acid and DNA homology between different transit peptides found in plants. Therefore, the use of antibodies immunologically reactive thereto in one genus, species, cultivar, or line may or may not be immunologically reactive to said transit peptides for the same passenger protein found in another genus, species, cultivar, or line. To ensure function, transit peptides from which the passenger protein of interest is derived are typically sequenced from a variety of species, cultivars, or lines to see if common amino acid motifs can be determined. If so, these common amino acid motifs can be chosen for synthetic peptide and antibody production. If said sites do not exist, individual hybridoma lines for the transit peptide of each passenger protein of interest may be required. This feature is especially important to consider in the production of hybrid crops, said crops being derived from transgenic and/or other parental lines.

Once a transit peptide is synthesized, it can be purified prior to conjugation. A variety of purification methods are available to the artisan including gel filtration, dialysis, reverse phase high performance liquid chromatography, ion exchange chromatography and the like.

After the transit peptide is synthesized and purified it may be desirable to conjugate said peptide with an appropriate carrier protein to increase the immunogenic response. A carrier protein should be a good immunogen and have a sufficient number of amino acid residues with reactive side chains for coupling to the synthetic peptide. Keyhole Limpet haemocyanin (KLH) is commonly used because of its proven efficacy. Other proteins useful as carrier molecules include thyroglobulin, bovine serum albumin, tetanus toxin, and the like.

In addition to the choice of carrier protein, a method of coupling the synthetic peptide (antigen) to the carrier protein can also be selected. Most coupling methods rely on the presence of free amino, sulfhydryl, phenolic, or carboxylic groups. The chosen coupling method can link the transit peptide to the carrier either through the carboxy- or amino terminal residue. A variety of coupling reagents are available to the artisan including glutaraldehyde, *Bis*-imido esters, *bis*-diaotized benzidine, *m*-maleimidobenzoyl-*N*-hydroxysuccinimide and the like.

In accordance with the present invention, there is provided material suitable for eliciting an immunogenic response in a system of mammalian origin. Preferably, the mammal can be of rat, mouse, horse, goat, rabbit, and the like. Most preferred is wherein a mouse is used for antibody production. Elicitation of said response is typically induced by immunization. Immunization protocols are well known in the art and can vary considerably yet remain effective as described in Current Protocols in Immunology, Edited by John E. Coligan et al., John Wiley & Sons, Inc. (1991). Typically, about 1 µg to 50 µg of antigen are given in emulsions of about 1 mL to about 2 mL for a rat and about one-tenth as much for mice. The first injection of antigen into a host animal may be subcutaneous, intramuscular, intraperitoneal or intravasular. Thereafter, about 1 to about 10 or more booster immunizations may be given at intervals of between about 2 and about 8 weeks. The route of immunization may be varied according to protocol.

Immune response elicited following injection is typically monitored by performing western analysis to determine the level of antibody present in the animal's serum using techniques commonly available to one having ordinary skill in the art of immunology. Injections of antigen are typically continued until the immunological response reaches a plateau of maximal response.

After immunization is complete, immune lymphocytes from the immunized animal are typically fused to myeloma cells to generate hybridoma cell lines, which are immortal and produce monoclonal antibodies. Lymphocytes are generally taken from either lymph node tissue or spleen tissue and are fused to plasmocytoma cells, which are specialized myeloma cells available to the artisan from the American Type Culture Collection, Manassas, VA. In a typical fusion, a suspension of lymphocyte cells is added to the myeloma cells in the presence of a fusogen such as polyethylene glycol (PEG). However, other methods of cell transformation are possible including transformation of myeloma cells with viruses such as Epstein-Barr virus and the like.

After fusion, cells are harvested, diluted, and cultured for a week or more in separate wells containing the appropriate selective agent such as hypoxanthine, aminopterin, and thymidine (HAT) which is capable of extinguishing non-fused cells. Cultured supernatants from said wells can be screened to determine hybridoma growth as well as determine the selectivity and affinity of a given antibody to the antigen. The cells may be screened for the presence of antibodies capable of recognizing selected antigenic determinants by using methods available to the skilled artisan such as solid phase radioimmunoassay, enzyme-linked immunosorbent assays, western analysis, and the like. The cells so selected can then be placed in individual wells as single cell colonies by limiting dilution. Feeder cells (e.g. thymocytes, peritoneal exudate cells, and the like) may be added as necessary. After growing, supernatants therefrom can be characterized for antibody production by rescreening as described herein.

Antibodies may be categorized by class or subclass, depending on which of a number of heavy chain constant domains they possess. Heavy chains are classified according to their constant region: IgA, IgD, IgE, IgG or IgM, with some subclasses among them. Light chains are classified as either kappa or lambda. Categorization of antibodies may be performed using commercially available isotyping kits as disclosed herein.

Once hybridoma cell lines have been established and antibodies have been produced and characterized, the antibodies can be purified for further analysis using a variety of techniques. Such techniques can include the use of Sepharose-protein G columns wherein the antibodies are bound followed by elution with 0.5 M ammonium acetate (pH 3.0) and neutralization with 1 M Tris. Other purification techniques can include the use ammonium sulfate precipitation/size exclusion chromatography, affinity chromatography using protein-A Sepharose, as well as standard ion exchange chromatographic techniques available to the artisan skill in the area of protein biochemistry. For purifying engineered antibodies, affinity tags can be encoded by the DNA so that amino acids are expressed which allow binding to a predetermined matrix. Such tags can include the 6X Histidine motif which can bind to a Ni-Sepharose column, a c-myc tag, and the like.

The amino acid sequence of said antibody can be determined using a variety of methods available to the artisan. Such methods can involve direct protein sequencing using a variety of techniques including Edman degradation, mass spectroscopy, nuclear magnetic resonance, and the like. Other methods to determine amino acid sequence can involve the use of molecular biological techniques whereby the gene encoding said antibodies are cloned and sequenced, as disclosed herein. The determination of amino acid sequence allows the antibody or derivatives thereof to be synthesized using a variety of peptide synthesis methods available to the artisan including solid phase t-butyloxycarbonyl (t-BOC), solid phase 9-fluorenylmethyloxycarbonyl (t-FMOC) methods, as well as methods developed by Geysen et al., (1987, J. Immunnol. Methods, 102:259-274); Houghton et al., (1985, Proc. Natl. Acad. Sci. USA, 82:5131-5135); and the like.

In a preferred embodiment, antibodies can be prepared in vitro from chromosomal DNA, cDNA or synthetic oligonucleotides using molecular biology techniques and heterologous expression systems such as E. *coli,* yeast, Baculoviruses, and the like. Cells or other source material containing DNA or RNA coding for the desired antibody may be isolated. In some instances, it may be desirable to isolate the genomic DNA or mRNA (to generate cDNA) from such source material as described in Goldfein et al., (1987, J. Immunology, 138:940-944). When significant levels of nucleic acids are not available, one can use polymerase chain reactions (PCR) to isolate nucleic acid fragments encoding the antibody of choice, as disclosed herein.

If the amino acid sequence has been previously determined using methods disclosed herein or others available to the skilled artisan, the genetic code can be used to reverse translate said amino acid sequence into a DNA sequence. A series of oligonucleotides ranging from about 20 to about 50 bases or more can then be synthesized in order to provide a series of overlapping fragments. The synthesized oligonucleotides can be annealed and ligated to create the gene encoding said antibody.

The complete genomic sequence of a particular antibody may be obtained by screening of a genomic or cosmid library with a probe. Probes can be considerably shorter than the entire gene sequence, but should be at least about 10 nucleotides, preferably at least about 15 nucleotides, more preferably at least about 20 or so nucleotides in length. Longer oligonucleotides up to the full length of the gene encoding the polypeptide of interest are also useful. Both DNA and RNA can be used as probes. In use, probes are typically labeled with ³²P, biotinylated, and the like in a manner that allows for detection thereof. Said probes are often incubated with single stranded DNA from the source of which the gene is desired. Hybridization, or the act of the probe binding to the DNA, is detected usually after binding using nitrocellulose paper or nylon membranes by means of the label on said probe. Hybridization techniques are well known to the person skilled in the art of molecular biology. Thus, antibody genes may be so isolated.

In a preferred embodiment, mRNA can easily be isolated from hybridoma cell lines expressing the antibody of interest using a variety of well known methods. Continually culturing said lines in conjunction with limiting dilutions as disclosed herein effectively results in a monoculture whereby cDNA obtained therefrom essentially encodes for only one antibody. After isotyping said antibody, use of oligonucleotide primers corresponding to constant regions found therein allows for the cloning thereof without necessarily proceeding through the steps common to other cloning procedures such as creation of libraries in phagemids, cosmids, and the like. Once cloned, the cDNA can be sequenced and the amino acid sequence can be determined via the genetic code.

One may engineer an antibody heavy chain or light chain variable region effectively homologous to the antibody heavy chain or light chain variable region of a known antibody. The term "effectively homologous" refers to the concept that the primary amino acid sequence of the variable region may be altered yet the antibody may retain a functional binding site with the capacity to bind to the same antigen or epitope. Accordingly, such variations and derivations are considered to be within the scope of the present invention. Antibodies so engineered may be experimentally determined using cross-blocking techniques wherein binding of one antibody prevents binding of a second antibody to the same epitope through either local or distant steric hindrance.

Modifications contemplated by the present invention include the addition, deletion, or conservative substitution of a limited number of various amino acids. Generally, an amino acid sequence is effectively homologous to a second amino acid sequence if at least about 70 percent, preferably at least about 80 percent, and most preferably, at least about 90 percent of the amino acid sequence of the variable portions of the two antibodies in question are homologous. Conservative substitutions are those exemplified such as glutamic acid and aspartic acid; valine, leucine, and isoleucine; asparagine and glutamine; threonine and serine; glycine and alanine; phenylalanine, tyrosine, and tryptophan; and lysine and arginine. Accordingly, such variations and derivations are considered to be within the scope of the present invention.

Binding affinities between the original and homologous antibody to the antigen of choice can also be used to determine "effective homology" Effectively homologous antibodies would be expected to have antibody:antigen binding constants that are at least within about 3 orders of magnitude compared to the original antibody, and more preferably binding constants that are within about 2 orders of magnitude relative to the original antibody, with binding constants essentially the same as the original antibody being most preferred. Determination of antibody binding constants is well known in the art.

Fragments of antibodies which maintain their ability to bind to the antigen are considered functional in accordance with the present invention. Said fragments include segments which are generated upon proteolytic cleavage or recombinantly prepared portions thereof. Thus, for example, portions of variable and constants domains of the antibody may be specifically altered or partially or completely omitted. Nonlimiting examples of such recombinant fragments include Fab, F(ab)₂, and Fv fragments as described in U.S. Patent 4,642,334 to Moore and U.S. Patent 4,816,567 to Genentech. In addition, antibodies may be modified to form single chain Fv molecules as described in U.S. Patent 4,946,778 to Genex Corp. and as disclosed herein. Said fragments and modifications thereto can also be created by manipulating genes encoding said fragments as disclosed herein. Recombinant constructs containing nucleic acid sequences encoding a functional antibody of interest or fragments thereof and heterologous nucleic acid sequences may be prepared. By heterologous is meant any sequence which is not naturally found joined to the antibody sequence. Hence, by definition, a DNA sequence joined to a sequence not naturally found in a gene encoding an antibody is considered to be heterologous.

Constructs may be designed to produce antibodies of interest or functional fragments thereof in either prokaryotic or eukaryotic cells. The expression of antibodies in a plant cells is of special interest. Moreover, the nucleic acid sequences encoding said antibodies may be integrated into a plant host genome. By transcribing and translating nucleic acid sequences encoding said antibodies or functional fragments thereof in a plant host, said plant is expected to exhibit properties wherein steady state passenger protein levels associated with a gene transit peptide are lower than that observed in the non-transformed plant under similar conditions.

The compositions and methods of the present invention as disclosed herein provide for decreased steady state levels of passenger proteins being targeted to organelles. A wide variety of modifications may be made in numerous types of plants. Preferred monocotyledonous plants, for example, include rice, corn, wheat, barley, oats, rye and sorghum. Examples of preferred dicotyledonous plants included canola, pea, soybean, sunflower, tobacco, cotton, sugar beet, petunia, tomato, broccoli, lettuce, apple, plum, orange, and lemon.

Modification to plants using the invention as disclosed herein may include varying the steady state protein levels involved in fatty acid distribution of a fatty acid source, such as rapeseed, Cuphea, corn, soybean, canola or any oilseed crop of interest. Antibodies can also be made against the transit peptide of passenger proteins such as Δ-9 desaturase, palmitoyl-ACP thioesterase, β-ketoacyl-ACP synthase, oleyl-ACP thioesterase, and the like. Antibodies can also be made against the transit peptide of proteins such as chlorophyll a/b binding proteins as a way to reduce chlorophyll content. Other organeller localized proteins having transit peptides, of which antibodies can be made using said transit peptide as an antigen as disclosed herein, include: NADPH+ dependent glyceraldehyde-3-phosphate dehydrogenase, early light inducible protein, clip protease regulatory protease, pyruvate orthophosphate dikinase, chlorophyll a/b binding protein, triose phosphate-3-phosphoglycerate phosphate translocator, 5-enol pyruval shikimate-3-phosphate synthase, dihydrofolate reductase, thymidylate synthase, acetyl-coenzyme A carboxylase, Cu/Zn superoxide dismutase, cysteine synthase, rubisco activase, ferritin, granule bound starch synthase, pyrophosphate, glutamine synthase, aldolase, glutathione reductase, nitrite reductase, 2-oxoglutarate/malate translocator, ADP-glucose pyrophosphorylase, ferrodoxin, carbonic anhydrase, polyphenol oxidase, ferrodoxin NADP= oxidoreductase, platocyannin, glycerol-3-phosphate dehydrogenase, lipoxygenase, o-acetylserine (thiol)-lysase, acyl carrier protein, 3-deoxy-D-arabino-heptulosonate 7-phosphate synthase, chloroplast-localized heat shock protein, starch phosphorylase, pyruvate orthophosphate dikinase, starch glycosyltransferase, and the like.

It is within the scope of the present invention that the steady state levels of passenger proteins can be decreased by transforming a plant cell with a gene encoding an antibody directed to the transit peptide associated with said passenger protein in a precursor protein form. It is also within the scope of this invention that transit peptides can be homologous or heterologous to the passenger protein as described herein. As further disclosed herein, transit peptides can be either indigenous or non-indigenous to the transformed plants. In a like manner, passenger proteins can be either indigenous or non-indigenous to the transformed plant cell.

Transit peptides and passenger proteins can be heterologous to each other in that they may not be associated in a manner found in nature. For example, the transit peptide from the small subunit of Rubisco (SSU) can be used to place a variety of proteins in the chloroplast of a plant cell thus causing proteins not normally found to accumulate therein. Antibodies reactive against SSU transit peptide would be expected to prevent accumulation and decrease steady state levels of passenger proteins associated therewith relative to non-transformed controls.

To obtain high expression of heterologous genes in plants it may be preferred to reengineer said genes so that they are more efficiently expressed in the cytoplasm of plant cells. Maize is one such plant where it may be preferred to reengineer the heterologous gene(s) prior to transformation to increase the expression level thereof in said plant. Therefore, an additional step in the design of genes encoding antibodies for plant expression may involve reengineering of a heterologous gene for optimal expression.

One reason for the reengineering an antibody gene for expression in maize is to ensure the optimal G+C content of the native gene as well as eliminate any sequences mimicking or duplicating plant gene control sequences. The presence of some A+T-rich sequences within the DNA of gene(s) introduced into plants (e.g., TATA box regions normally found in gene promoters) may result in aberrant transcription of the gene(s). On the other hand, the presence of other regulatory sequences residing in the transcribed mRNA (e.g., polyadenylation signal sequences (AAUAAA), or sequences complementary to small nuclear RNAs involved in pre-mRNA splicing) may lead to RNA instability. Therefore, one aspect in the design of genes encoding an antibody gene for maize expression, more preferably referred to as plant optimized gene(s), is to generate a DNA sequence having a higher G+C content, and preferably one close to that of maize genes coding for metabolic enzymes. Another goal in the design of the plant optimized gene(s) is to generate a DNA sequence in which the sequence modifications do not hinder translation.

The table below (Table 1) illustrates how high the G+C content is in maize. For the data in Table 1, coding regions of the genes were extracted from GenBank (Release 71) entries, and base compositions were calculated using the MacVector™ program (IBI, New Haven, CT). Intron sequences were ignored in the calculations.

Due to the plasticity afforded by the redundancy of the genetic code (i.e., some amino acids are specified by more than one codon), evolution of the genomes in different organisms or
classes of organisms has resulted in differential usage of redundant codons. This "codon bias" is reflected in the mean base composition of protein coding regions. For example,
organisms with relatively low G+C contents utilize codons having A or T in the third position of redundant codons, whereas those having higher G+C contents utilize codons having
G or C in the third position. It is thought that the presence of "minor" codons within a mRNA may reduce the absolute translation rate of that mRNA, especially when the relative abundance of the charged tRNA corresponding to the minor codon is low. An extension of this is that the diminution of translation rate by individual minor codons would be at least additive for multiple minor codons. Therefore, mRNAs having high relative contents of minor codons would have correspondingly low translation rates. This rate would be reflected by subsequent low levels of the encoded protein.

In reengineering genes encoding an antibody for maize expression, the codon bias of the plant has been determined. The codon bias for maize is the statistical codon distribution that the plant uses for coding its proteins and the preferred codon usage is shown in Table 2. After determining the bias, the percent frequency of the codons in the gene(s) of interest is determined. The primary codons preferred by the plant should be determined as well as the second and third choice of preferred codons. Afterwards, the amino acid sequence of the gene of interest is reverse translated so that the resulting nucleic acid sequence codes for exactly the same protein as the native gene wanting to be heterologously expressed. The new

**Table 1. Compilation of G+C contents of protein coding regions of maize genes.**

| Protein Class^{a} | Range %G+C | Mean %G+C^{b} |
|---|---|---|
| Metabolic Enzymes (76) | 44.4-75.3 | 59.0 ( ± 8.0) |
| Structural Proteins (18) | 48.6-70.5 | 63.6 ( ± 6.7) |
| Regulatory Proteins (5) | 57.2-68.9 | 62.0 ( ± 4.9) |
| Uncharacterized Proteins (9) | 41.5-70.3 | 64.3 ( ± 7.2) |
| **All Proteins (108)** | **44.4-75.3** | **60.8 ( ± 5.2)** |

| | | |
|---|---|---|
| ^{a}number of genes in class given in parentheses. ^{b}standard deviations given in parentheses. ^{c}Combined groups mean ignored in mean calculation. | | |

DNA sequences are designed using codon bias information so that they correspond to the most preferred codons of the desired plant. The new sequences are then analyzed for restriction enzyme sites that might have been created by the modifications. The identified sites are further modified by replacing the codons with second or third choice with preferred codons. Other sites in the sequence which could affect transcription or translation of the gene of interest are the exon:intron 5' or 3' junctions, poly A addition signals, or RNA polymerase termination signals. The sequence is further analyzed and modified to reduce the frequency of TA or GC doublets. In addition to the doublets, G or C sequence blocks that have more than about four residues that are the same can affect transcription of the sequence. Therefore, these blocks are also modified by replacing the codons of first or second choice, etc. with the next preferred codon of choice.

It is preferred that the plant optimized gene(s) encoding an antibody contain about 63% of first choice codons, between about 22% to about 37% second choice codons, and between about 15% to about 0% third choice codons, wherein the total percentage is 100%. Most preferred the plant optimized gene(s) contains about 63% of first choice codons, at least about 22% second choice codons, about 7.5% third choice codons, and about 7.5% fourth choice codons, wherein the total percentage is 100%. The preferred codon usage for engineering genes for maize expression are shown in Table 2. The method described above enables one skilled in the art to modify gene(s) that are foreign to a particular plant so that the genes are optimally expressed in plants. The method is further illustrated in pending PCT application WO 97/13402.

In order to design plant optimized genes, the amino acid sequence of the antibody of interest is reverse translated into a DNA sequence utilizing a non-redundant genetic code established from a codon bias table compiled for the gene sequences for the particular plant, as shown in Table 2. The resulting DNA sequence, which is completely homogeneous in codon usage, is further modified to establish a DNA sequence that besides having a higher degree of codon diversity, also contains strategically placed restriction enzyme recognition sites, desirable base composition, and a lack of sequences that might interfere with transcription of the gene or translation of the product mRNA.

In designing an antibody gene for optimal plant expression, it may be necessary to add nucleic acid sequences encoding amino acids that have been proven to increase the expression levels thereof. For example, Schouten et al., (1996, Plant Molecular Biology, 30:781-793) have shown that the carboxy-terminal amino acid sequence KDEL increased antibody accumulation in transgenic tobacco. Other strategies for increasing expression of antibody genes have been explored by Owen et al., (1992, Chem. Ind., 11:406-408); Ma (1995, ACS Symp. Ser., 604:56-59); Schouten et al., (1997 FEBS Lett. 415:235-241); Conrad and Fiedler, (1994, Plant Mol. Biol. 26:1023-1030): Fiedler et al., (1997, Immunotechnology 3:205-216); van Engelen et al., (1994, Plant Mol. Biol. 26:1701-1710); Hiatt et al., (1989, Nature 342: 76-78); Firek et al., (1993, Plant Mol. Biol. 23:861-870); Ma et al., (1994, Eur. J. Immunol. 24:131-138).

In another aspect of the invention, genes encoding the antibodies are expressed from transcriptional units inserted into the plant genome. Preferably, said transcriptional units are recombinant vectors capable of stable integration into the plant genome and selection of transformed plant lines expressing mRNA encoding for said antibodies are expressed either by constitutive or inducible promoters in the plant cell. Once expressed, mRNA is translated into proteins, thereby incorporating amino acids of interest. Genes encoding antibodies expressed in plant cells can be under the control of a constitutive promoter, a tissue-specific promoter, an inducible promoter, and the like. Several techniques exist for introducing foreign recombinant vectors into plant cells, and for obtaining plants that stably maintain and express the introduced gene. Such techniques include acceleration of genetic material coated onto microparticles directly into cells (U.S. Patents 4,945,050 to Cornell and 5,141,131 to DowElanco, now Dow AgroSciences, LLC). In addition, plants may be transformed using *Agrobacterium* technology, see U.S. Patent 5,177,010 to University of Toledo, 5,104,310 to Texas A&M, European Patent Application 0131624B1, European Patent Applications 120516, 15941881 and 176,112 to Schilperoot, U.S. Patents 5,149,645, 5,969,976, 5,464,763 and 4,940,838 and 4,693,976 to Schilperoot, European Patent Applications 116718, 290799, 320500 all to Max Planck, European Patent Applications 604662,627752 and US Patent 5,591,616 to Japan Tobacco, European Patent Applications 0267159, and 0292435 and U.S. Patent 5,231,019 all to Ciba Geigy, now Novartis, U.S. Patents 5,463,174 and 4,762,785 both to Calgene, and U.S. Patents 5,004,863 and 5,159,135 both to Agracetus. Other transformation technology includes whiskers technology, see U.S. Patents 5,302,523 and 5,464,765 both to Zeneca. Electroporation technology has also been used to transform plants, see WO 87/06614 to Boyce Thompson Institute, 5,472,869 and 5,384,253 both to Dekalb, WO9209696 and WO9321335 both to Plant Genetic Systems. Furthermore, viral vectors can also be used in produce transgenic plants expressing the protein of interest. For example, monocotyledonous plant can be transformed with a viral vector using the methods described in U.S. Patents 5,569,597 to Mycogen and Ciba-Giegy, now Novartis, as well as U.S. Patents 5,589,367 and 5,316,931, both to Biosource.

As mentioned previously, the manner in which the DNA construct is introduced into the plant host is not critical to this invention. Any method which provides for efficient transformation may be employed. For example, various methods for plant cell transformation are described herein and include the use of Ti or Ri-plasmids and the like to perform Agrobacterium mediated transformation. In many instances, it will be desirable to have the construct used for transformation bordered on one or both sides by T-DNA borders, more specifically the right border. This is particularly useful when the construct uses *Agrobacterium tumefaciens* or *Agrobacterium rhizogenes* as a mode for transformation, although T-DNA borders may find use with other modes of transformation.

Where Agrobacterium is used for plant cell transformation, a vector may be used which may be introduced into the host for homologous recombination with T-DNA or the Ti or Ri plasmid present in the host. Introduction of the vector may be performed via electroporation, tri-parental mating and other techniques for transforming gram-negative bacteria which are known to those skilled in the art. The manner of vector transformation into the Agrobacterium host is not critical to this invention. The Ti or Ri plasmid containing the T-DNA for recombination may be capable or incapable of causing gall formation, and is not critical to said invention so long as the vir genes are present.

**Table 2. Preferred amino acid codons for proteins expressed in maize.**

| **Amino Acid** | **Codon*** |
|---|---|
| Alanine | GCC/GCG |
| Cysteine | TGC/TGT |
| Aspartic Acid | GAC/GAT |
| Glutamic Acid | GAG/GAA |
| Phenylalanine | TTC/TTT |
| Glycine | GGC/GGG |
| Histidine | CAC/CAT |
| Isoleucine | ATC/ATT |
| Lysine | AAG/AAA |
| Leucine | CTG/CTC |
| Methionine | ATG |
| Asparagine | AAC/AAT |
| Proline | CCG/CCA |
| Glutamine | CAG/CAA |
| Arginine | AGG/CGC |
| Serine | AGC/TCC |
| Threonine | ACC/ACG |
| Valine | GTG/GTC |
| Tryptophan | TGG |
| Tryrosine | TAC/TAT |
| Stop | TGA/TAG |
| * The first and second preferred codons for maize. | |

In some cases where Agrobacterium is used for transformation, the expression construct being within the T-DNA borders will be inserted into a broad spectrum vector such as pRK2 or derivatives thereof as described in Ditta et al., (PNAS USA (1980) 77:7347-7351 and EPO 0 120 515, which are incorporated herein by reference. Included within the expression construct and the T-DNA will be one or more markers as described herein which allow for selection of transformed Agrobacterium and transformed plant cells. The particular marker employed is not essential to this invention, with the preferred marker depending on the host and construction used.

For transformation of plant cells using Agrobacterium, explants may be combined and incubated with the transformed Agrobacterium for sufficient time to allow transformation thereof. After transformation, the agrobacteria are killed by selection with the appropriate antibiotic and plant cells are cultured with the appropriate selective medium. Once calli are formed, shoot formation can be encouraged by employing the appropriate plant hormones according to methods well known in the art of plant tissue culturing and plant regeneration. However, a callus intermediate stage may not always be necessary. After shoot formation, said plant cells can be transferred to medium which encourages root formation thereby completing plant regeneration. The plants may then be grown to seed and said seed can be used to establish future generations. Regardless of transformation technique, the antibody gene is preferably incorporated into a gene transfer vector adapted to express said gene in a plant cell by including in the vector a plant promoter regulatory element, as well as 3' non-translated transcriptional termination regions such as Nos and the like.

In addition to numerous technologies for transforming plants, the type of tissue which is contacted with the foreign genes may vary as well. Such tissue would include but would not be limited to embryogenic tissue, callus tissue types I, II, and III, hypocotyl, meristem, root tissue and the like. Almost all plant tissues may be transformed during cellular dedifferentiation using appropriate techniques described herein.

Another variable is the choice of a selectable marker. Preference for a particular marker is at the discretion of the artisan, but any of the following selectable markers may be used along with any other gene not listed herein which could function as a selectable marker. Such selectable markers include but are not limited to aminoglycoside phosphotransferase gene of transposon Tn5 (Aph II) which encodes resistance to the antibiotics kanamycin, neomycin and G418, as well as those genes which encode for resistance or tolerance to glyphosate; hygromycin; methotrexate; phosphinothricin (bialophos); imidazolinones, sulfonylureas and triazolopyrimidine herbicides, such as chlorsulfuron; bromoxynil, dalapon and the like.

In addition to a selectable marker, it may be desirous to use a reporter gene. In some instances a reporter gene may be used with or without a selectable marker. Reporter genes are genes which are typically not present in the recipient organism or tissue and typically encode for proteins resulting in some phenotypic change or enzymatic property. Examples of such genes are provided in K. Wising et al. (1988) Ann. Rev. Genetics, 22:421, which is incorporated herein by reference. Preferred reporter genes include the beta-glucuronidase (GUS) of the uidA locus of *E. coli*, the chloramphenicol acetyl transferase gene from Tn9 of *E. coli,* the green fluorescent protein from the bioluminescent jellyfish *Aequorea victoria,* and the luciferase genes from firefly *Photinus pyralis.* An assay for detecting reporter gene expression may then be performed at a suitable time after said gene has been introduced into recipient cells. A preferred such assay entails the use of the gene encoding beta-glucuronidase (GUS) of the uidA locus of *E. coli* as described by Jefferson et al., (1987 Biochem. Soc. Trans. 15, 17-19) to identify transformed cells.

In addition to plant promoter regulatory elements, promoter regulatory elements from a variety of sources can be used efficiently in plant cells to express foreign genes. For example, promoter regulatory elements of bacterial origin, such as the octopine synthase promoter, the nopaline synthase promoter, the mannopine synthase promoter; promoters of viral origin, such as the cauliflower mosaic virus (35S and 19S), 35T (which is a re-engineered 35S promoter, see WO 97/13402 published April 17, 1997) and the like may be used. Plant promoter regulatory elements include but are not limited to ribulose-1,6-bisphosphate (RUBP) carboxylase small subunit (ssu), beta-conglycinin promoter, beta-phaseolin promoter, ADH promoter, heat-shock promoters and tissue specific promoters. Other elements such as matrix attachment regions, scaffold attachment regions, introns, enhancers, polyadenylation sequences, and the like may be present and thus may improve the transcription efficiency or DNA integration. Such elements may or may not be necessary for DNA function, although they can provide better expression or functioning of the DNA by affecting transcription, mRNA stability, and the like. Such elements may be included in the DNA as desired to obtain optimal performance of the transformed DNA in the plant. Typical elements include but are not limited to Adh-intron 1, Adh-intron 6, the alfalfa mosaic virus coat protein leader sequence, the maize streak virus coat protein leader sequence, as well as others available to a skilled artisan.

Constitutive promoter regulatory elements may also be used thereby directing continuous gene expression in all cells types and at all times (e.g., actin, ubiquitin, CaMV 35S, and the like). Tissue specific promoter regulatory elements are responsible for gene expression in specific cell or tissue types, such as the leaves or seeds (e.g., zein, oleosin, napin, ACP, globulin, and the like) and these may also be used.

Promoter regulatory elements may also be active during a certain stage of the plants' development as well as active in plant tissues and organs. Examples of such include but are not limited to pollen-specific, embryo specific, corn silk specific, cotton fiber specific, root specific, seed endosperm, fruit-specific, specific promoter regulatory elements and the like. Under certain circumstances it may be desirable to use an inducible promoter regulatory element, which is responsible for expression of genes in response to a specific signal, such as: physical stimulus (heat shock genes); light (RUBP carboxylase); hormone (Em); metabolites; chemical; and stress. Other desirable transcription and translation elements that function in plants may be used.

The present invention also contemplates the use of transformed plants which are selfed to produce an inbred plant. The inbred plant can produce seed containing genes encoding antibodies. The inbred lines can also be crossed with other inbred lines to produce hybrids. Parts obtained from the regenerated plant, as flowers, seeds, leaves branches, fruit and the like are covered by the invention provided that said parts contain genes encoding and/or express the antibody of interest. Progeny and variants, and mutants of the regenerated plants are also included within the scope of the invention.

In diploid plants, typically one parent may be transformed and the other parent maybe wild type. After crossing the parents, the first generation hybrids (F₁) can be selfed to produce second generation hybrids (F₂). Those plants expressing the highest levels of the antibody or having the desired phenotype can be chosen.

Another way to generate plants expressing active antibodies is to transform two plant lines, one with a gene encoding either a heavy chain or light chain and the other plant with the complimentary chain. The two or more plant lines can then be crossed to create progeny contain both heavy chain and light chain components. Functional antibodies can be obtained in this manner as described by Ma et al., (1995) Science 268:716-719.

Plant RNA viral based systems can also be used to express the antibody genes for the purposes as disclosed herein. In so doing, the gene can be inserted into the coat protein region of a suitable plant virus which will infect the host plant of interest. The antibody can then be expressed thus modifying steady state passenger protein levels. Plant RNA viral based systems are described in U.S. Patents 5,500,360 to Mycgoen Plant Sciences, Inc. and U.S. Patents 5,316,931 and 5,589,367 to Biosource Genetics Corp.

Standard and molecular biology techniques may be used to clone and sequence the toxins described herein. Additional information may be found in Sambrook, J., Fritsch, E. F., and Maniatis, T. (1989), Molecular Cloning, A Laboratory Manual, Cold Spring Harbor Press.

The invention is illustrated in further details by the following non-limiting examples. The examples are for the purpose of illustration only and are not to be construed as limiting the scope of the present invention.

### EXAMPLE 1

### ISOLATION AND CLONING OF STEAROYL-ACP Δ-9 DESATURASE (Δ-9 DESATURASE) FROM MAIZE

A cDNA clone encoding maize stearoyl-ACP Δ-9 desaturase (Δ-9 desaturase) was obtained from a cDNA library derived from maize kernels of inbred CS608 (Mycogen Seeds, San Diego, CA) that had been grown in a greenhouse and hand pollinated. The cDNA library was prepared from said kernels harvested at 20 days after pollination, hereinafter 20-DAP. Upon harvest, embryos were immediately collected, frozen on dry ice, and stored at - 70° C. RNA was extracted by grinding 2.5 g to a fine powder in liquid nitrogen. Afterwards, 10 mL of extraction buffer [50 mM Tris-HC1, pH 8.0, 4% para-amino salicyclic acid (Sigma Chemical Co), 1% tri-iso-propylnaphtalenesulfonic acid (Eastman Kodak Co., Rochester, NY), 10 mM DTT, and 10 mM sodium meta-bisulfite (Sigma Chemical Co., St. Louis, MO)] was added and the mixture was homogenized for 1 min using a TEKMAR TISSUMIZER (Tekmar Co., Cincinnati, OH). The homogenate was extracted with an equal volume of phenol equilibrated with 0.1 M Tris-HCl, pH 8.0. Organic and aqueous phases were separated by centrifugation at 4° C. The aqueous phase was removed and extracted with an equal volume of chloroform/octanol (24:1). The supernatant was then transferred, centrifuged, transferred again, and a one-half volume of 7.5 M ammonium acetate (pH 8.0) was added. RNA was then precipitated on ice for 30 min.

Precipitated RNA was collected by centrifugation and dissolved in 1 mL of diethylpyrocarbonate-treated water (0.1% v/v), hereinafter DEPC-water. One-half volume of 7.5 M ammonium acetate (pH 8.0) and two volumes of 100% ethanol were added and the RNA was allowed to precipitate at -20° C for 30 min. The precipitate was collected by centrifugation, washed in ice-cold 70% ethanol, air dried, and dissolved in 0.5 mL DEPC-treated water.

PolyA⁺ mRNA was purified on oligo dT-cellulose (Collaborative Biomedical Products, Bedford, MA) columns. Type 3 oligo-dT cellulose (0.1 g) was equilibrated in 5 mL of buffer 1 for 30 min, where buffer 1 was loading buffer with 0.5 M NaCl and loading buffer was 20 mM Tris-HC1, pH 7.6, 1 mM EDTA, and 0.1% sodium lauryl sulfate (SDS). The column was washed with 3 volumes of DEPC-water, 3 volumes of wash buffer [0.1 N NaOH, 5 mM EDTA], 3 volumes of DEPC-water, and 5 volumes of buffer 1. The dissolved RNA pellet was heated at 65° C for 5 min, diluted 2x with buffer 2 [2x loading buffer] and then applied to the oligo-dT column. The flow through material was then collected, reheated, and reapplied to the column. Following, the column was washed with 10 volumes of buffer 1 followed by 10 volumes of buffer 3 [loading buffer having 0.1 M NaCl]. PolyA⁺ RNA was eluted with 3 volumes of elution buffer [10 mM Tris-HCl, pH 7.5, 1 mM EDTA, 0.05% SDS] and collected in 0.5 mL fractions. RNA fractions were combined, buffered to 0.3 M sodium acetate pH 5.2, and precipitated at -20° C for 16 h after addition of 2.2 volumes of 100% ethanol. The precipitate was collected by centrifugation, washed with 70% ethanol, dried, and dissolved in 50 µL DEPC-treated water. This material was then repurified on a fresh oligo-dT column as described herein to produce highly-enriched polyA⁺ mRNA. RNA concentrations were determined by measuring OD_{260 nm}.

Five µg of polyA⁺ RNA was converted to cDNA and cloned into the LAMBDA UNI-ZAP vector using the Lambda ZAP-cDNA synthesis and cloning kit according to the manufacturers protocols (Stratagene, La Jolla, CA). The resulting library had an original titer of 3.38 x 10¹⁰ plaque forming units/mL (pfu/mL), greater than 95% recombinants and an average insert size of 1.35 kb. The cDNA library was amplified according to Sambrook et al., (Molecular Cloning, A Laboratory Manual, 2nd Ed. (1989) Cold Spring Harbor Laboratory Press) and had a titer of 6.0 x 10⁶ pfu/mL. Total library cDNA was batch rescued and isolated as follows: 5 mL of XL1 Blue *E. coli* cells (Stratagene) at OD₆₀₀ₙₘ = 1.0 in 10 mM MgSO₄ were mixed with 8.3 µL (5x10⁸ pfu) of amplified embryo cDNA library phage-stock, and 100 µL EXASSIST helper phage (Stratagene) and incubated at 37° C for 20 min. Twenty-five mL of TY medium [8.0 g/L tryptone, 5.0 g/L yeast extract, and 2.5 g/L NaCl, pH 7.8] were added and cells were incubated at 37° C for 3 h while shaking. Afterwards, bacterial cells were heat killed at 68° C for 15 min and the supernatant was recovered. Five hundred µL supernatant was mixed with 14.5 mL of SOLR cells (Stratagene) (OD₆₀₀ₙₘ = 1.5), incubated at 37° C for 15 min, added to 500 mL LB [10 g/L tryptone, 10 g/L NaCl, and 5 g/L yeast extract containing 50 µg/mL Ampicillin], and grown overnight. Afterwards, plasmid DNA was obtained by alkaline lysis/CsCl purification, according to Sambrook et al (Molecular Cloning, A Laboratory Manual, 2nd Ed. (1989) Cold Spring Harbor Laboratory Press), which is incorporated by reference, and analyzed by agarose gel electrophoresis following digestion with EcoRI/XhoI. A smear ranging from 0.5 to 3.0 kb was observed following electrophoresis.

To isolate a clone encoding maize Δ-9 desaturase, a DNA fragment was amplified using polymerase chain reaction technology, hereinafter PCR, to produce a probe which could be used to isolate a full length cDNA. A 5' primer with 128-fold degeneracy and a 3' primer with 128-fold degeneracy, entered herein as SEQ ID NO:1 and SEQ ID NO:2, respectively, were synthesized on an Applied Biosystems High-Throughput DNA Synthesizer Model 394 (Foster City, CA). Double stranded cDNA was used as template. PCR amplification was performed as follows: 10 ng template DNA, 5 µL 10x reaction buffer, hereinafter 10X RB, [100 mM Tris-HCl pH 8.3, 500 mM KCl, 15 mM MgCl₂, 0.01% (w/v) gelatin], 5 µL of 2 mM deoxyribose nucleotides triphosphate (dNTPs), 50 pmole primers (SEQ ID NO:1 and SEQ ID NO:2), 2.5 units AMPLITAQ DNA Polymerase (Perkin-Elmer, Norwalk, CT) and water for a total volume of 50 µL. A DNA Thermal Cycler (Perkin-Elmer Cetus Model #9600, Norwalk, CT) was programmed as follows: 96° C for 1 min; followed by [94° C (1 min), 37° C (2 min), and 72° C (3 min)] for 35 cycles; followed by 7 min at 72°C. A DNA product of 276 base pairs (bp) was obtained, sequenced as described, infra, and entered herein as SEQ ID NO:3.

The PCR fragment was cloned directly into the pBC vector (Stratagene, LaJolla, CA) and transformed into One Shot™ INVaF' competent cells (Invitrogen) according to manufacturers specifications. The DNA was extracted using the Qiawell Plasmid Purification System (Qiagen, Santa Clarita, CA) according to the manufacturer's instructions. Recombinant clones were sequenced by dideoxy chain termination using PRISM AMPLITAQ READY REACTION DYEDEOXY Terminator cycle sequencing kit #401384 according to the manufacturer (Perkin-Elmer Applied Biosystems Division, Foster City, CA). Samples were run on an ABI373A automated DNA sequencer (Perkin-Elmer, Applied Biosystems Division). DNA sequence analysis of SEQ ID NO:3 was performed using MACVECTOR v. 4.1.4 (Oxford Molecular, Campbell, KY), which gave theoretical translations and alignments thus generating the amino acid sequence entered herein as SEQ ID NO:4.

The CS608 embryo cDNA library described herein was screened using a DNA fragment which was essentially SEQ ID NO:3. This fragment was used to obtain full length clones encoding maize Δ-9 desaturase. Probe DNA was obtained by digesting the cloned PCR fragment (SEQ ID NO:3) with the appropriate restriction enzymes. This material was then run on a preparative 1% agarose gel, the band was excised and the DNA was extracted with QIAEX (Qiagen). An α³²P-deoxyribocytosine triphosphate (dCTP)-labeled probe was generated using QUICKPRIME Random Labeling kit (Stratagene, LaJolla, CA) according to the manufacturer instructions using 5 µL of [α³²P]-dCTP (3000 Ci/mmole, 10 µCi/µL, DuPont, NEN Life Science Products, Boston, MA). Afterwards, the labeling reaction was applied to a NucTrap column (Stratagene) equilibrated with TE [10 mM Tris-HCl, pH 8.0, 1 mM EDTA]. Labeled DNA was eluted with 2 volumes of TE (400 µL each). The probe was heat denatured before being added to hybridization buffer as described herein.

Methods for phage titering, plating, coring and rescuing were as described in the LAMBDA ZAP II Library (Stratagene) instruction manual. The cDNA library was plated (100,000 pfu/plate) on five 22.5 x 22.5 cm NUNC assay plates (Nunc Inc. Roskilde, Denmark). Duplicate phage lifts were taken from each plate using 0.45 µm Hybond N+ nylon membranes (Amersham, Arlington Heights, IL). Following transfer of the phage to the filters, cells and phage were denatured and DNA was fixed to the membranes by autoclaving for 8 min at 125° C followed by UV-crosslinking using a STRATALINKER UV Crosslinker (Stratagene).

Prior to hybridization, filters were extensively washed in 2x SSC [1x SSC is 150 mM NaCl, 15 mM sodium citrated, pH 7.0] 0.1% sodium dodecyl sulfate (SDS) at 65° C. Filter prehybridization was performed at 42° C for 1 h in 200 mL hybridization buffer containing 50% (v/v) formamide, 3x SSC, 10x Denhardt's solution [1x Denhardt's solution is 0.02% Ficoll (Type 400, Pharmacia), 0.02% polyvinylpyrollidone, and 0.02% bovine serum albumin], 0.1% (w/v) SDS, and 100 µg/mL sheared and denatured salmon sperm DNA. Afterwards, used hybridization buffer was replaced with 100 mL of fresh hybridization buffer containing labeled probe (final activity = 6.1 x 10⁶ dpm/mL). Hybridization continued for 18-20 h at 42° C with gentle rotation. Afterwards, filters were washed twice at room temperature for 40 min in 100 mL of wash solution containing 0.1x SSC and 0.1% SDS, followed by 3 - 1 h washes with 1 L of the same at 60° C, followed by 2 - 1 h washes with 2 L of the same at 60° C. Filters were then exposed to Kodak XOMAT-AR Film (Eastman Kodak Company, Rochester, NY) with intensifying screens (Lightening Plus, DuPont CRONEX, DuPont, Wilmington DE) for 16 h at -70° C. Examination of films allowed the identification of positive plaques.

Some of the positive plaques were cored out and stored in 1 mL SM buffer [5.8 g/L NaCl, 2 g/L MgSO₄, 20 mM Tris-HCl, pH 7.5, 5 mL/L of 2% (w/v) gelatin] with 50 µL chloroform. Phage were plated for secondary screening using 50 µL of a 1:1000 dilution of the primary phage stock. Positive plaques from the secondary screening were cored out and stored in 500 µL of SM buffer. Phage from these stocks were then plated for tertiary screenings using amounts ranging from 5 µL of undiluted secondary stock to 20 µL of 1:100 dilution in SM buffer. All subsequent hybridizations were performed as described, *supra.* Isolates were rescued into phagemid form per the LAMBDA-ZAP II Library Instruction Manual (Stratagene). Rescued phagemid were plated by combining 200 µL SOLR cells (Stratagene) grown to OD₆₀₀ₙₘ = 0.5 to 1.0 with 50-100 µL phagemid and incubating for 15 min at 37° C. Cells containing phagemid were streaked on LB agar containing Ampicillin (75 µg/mL) and grown overnight at 37° C. DNA was extracted from 4 mL liquid cultures grown overnight at 37° C in TB [1.2% tryptone, 2.4% yeast extract, 0.4% glycerol, 0.17 M KH₂PO₄, and 0.72 M K₂HPO₄] using the alkaline lysis/polyethylene glycol protocol described in the PRISM READY REACTION DYEDEOXY Terminator Cycle Sequencing Kit Protocol#401388 Rev. B (Perkin-Elmer, Applied Biosystems Division). A sequence of the full length maize Δ-9 desaturase cDNA and corresponding amino acid sequence is entered herein as SEQ ID NO:5 and SEQ ID NO:6, respectively.

### EXAMPLE 2

### EXPRESSION OF THE GENES ENCODING MAIZE STEAROYL-ACP Δ-9 DESATURASE (Δ-9 DESATURASE) MATURE PROTEIN AND PRECURSOR PROTEIN

The coding sequence of maize Δ9 desaturase was cloned in expression vector pET9d DN using standard molecular cloning techniques. pET9d DN is a modified version of expression vector pET9d (Novagen Inc., Madison, WI) allowing subcloning of fragments in a unique NheI site downstream from the Shine & Dalgarno sequence and translation initiation ATG codon. To facilitate subcloning the cDNA clone (SEQ ID NO:5) was amplified with primers having the sequence entered herein as SEQ ID NO:7 and SEQ ID NO:8. Following amplification, the PCR product was digested with an excess of NheI, purified through agarose gel and directly ligated into vector pET9d DN which had been cut with NheI and dephosphorylated with calf intestinal phosphatase. The ligation was transformed into *E. coli* DH5α cells and recombinant plasmids containing inserts were selected by miniprep screening and digestion with NheI. One clone with the appropriate sized insert in the correct orientation relative to the T7 promoter was identified by separate digestion with NheI, NcoI or XhoI. By subcloning fragment in the NheI site of pET9d DN, the putative precursor polypeptide was joined to the ATG codon downstream from the T7 promoter and bacterial Shine & Dalgarno sequence provided by pET9d DN. This NheI site changed the nucleotide sequence of the putative N-terminus of the precursor protein from ATG GCG CTC CGC to ATG GCT AGC CTC CGC, which changed the encoded Met-Ala-Leu-Arg amino acid sequence to Met-Ala-Ser-Leu-Arg. The insert of this clone was sequenced entirely and the clone was assigned number pDAB432D

The precursor maize Δ9 desaturase was expressed in *E. coli* BL21(DE3) (Novagen Inc., Madison, WI). For small scale expression, 1 µg of plasmid pDAB432D was transformed into 200 µL CaCl₂ competent cells and plated on two LB plates containing kanamycin at 25 µg/mL. Following overnight incubation at 37° C colonies were scraped off the plate and resuspended in 10 mL of LB Broth containing kanamycin (50 µg/mL) and isopropyl-β-D-thiogalactoside (IPTG) at 1.0 mM. Cells were allowed to express proteins for 3 hr during vigorous shaking at 37° C. Cells were harvested by centrifugation at 3000 rpm for 10 min at 4° C. The cell pellet was frozen and thawed twice in dry ice-ethanol to improve cell lysis. Next, the cell pellet was resuspended in 1.0 mL of lysis buffer (10 mM Tris HCl pH 8.0, 1.0 mM EDTA, 150 mM NaCl, 0.1% Triton X 100, 100 µg/mL DNaseI, 100 µg/mL RNaseH, 1.0 mg/mL lysozyme) and incubated at 37° C until it was no longer viscous. Soluble proteins were separated from aggregated denatured proteins by centrifugation at 4° C for 10 min. The insoluble pellet was resuspended in about 300 µL of the above lysis buffer. Both fractions had an approximate final volume of 0.5 mL.

Protein expression experiments were analyzed by electrophoresis of small aliquots (1.0 to 2.0 µL) of the soluble and pellet fractions on 10-15% gradient SDS polyacrylamide Phast Gels (Pharmacia LKB Biotechnology, Piscataway, NJ). Protein bands were visualized by staining with Coomassie Brilliant Blue in the Phast Gel apparatus, and destained overnight by shaking in a small volume of 10% glycerol, 10% acetic acid. Molecular size of bands was determined by comparison to Bio-Rad Low Molecular Weight markers (Bio-Rad Laboratories, Hercules, CA). Western blot analysis was performed using antibodies produced as described herein specific to the mature portion of maize Δ9-desaturase. Expression of plasmid pDAB432D in *E.coli* resulted in the production of a 42 kDa protein in the cell pellet, indicating that the precursor protein produced in E. *coli* was insoluble.

In production of mature Δ-9 desaturase protein, the portion of the cDNA clone encoding the mature desaturase protein was subcloned into pETd DAN. The amino terminus of the mature maize Δ-9 desaturase protein determined as described herein. In order to express the mature protein, DNA corresponding to SEQ ID NO:11 was cloned into the expression vector pET9dΔN which is a modified version of pET9d (Novagen, Inc.). The vector pET9dΔN allowed subcloning of fragments in a unique NheI site downstream from the Shine and Dalgarno sequence and translation initiation ATG codon.

To facilitate subcloning, DNA corresponding to SEQ ID NO:5 was amplified with primers entered herein as SEQ ID NO:9 and SEQ ID NO:10. The DNA product had the sequence entered herein as SEQ ID NO:11 and encoded a protein having the amino acid sequence entered herein as SEQ ID NO:12. Following amplification, the PCR product was digested with an excess of NheI, gel purified through agarose gel and directly ligated into vector pET9dΔN which had been cut with NheI and dephosphorylated with calf intestinal phosphatase. The ligation was transformed into *E. coli* DH5α cells, and recombinant plasmid containing inserts were selected by miniprep screening and digestion with NheI. Clones with the insert in the correct orientation relative to the T7 promoter were identified by separate digestions with NcoI or XhoI. The plasmid having the DNA construct in the correct orientation was named pDAB428. Expression of protein from said plasmid was performed in a manner similar to that described for plasmid pDAB432D.

### EXAMPLE 3

### PURIFICATION AND CHARACTERIZATION OF NATIVE AND E. coli EXPRESSED MAIZE STEAROYL-ACP Δ-9 DESATURASE (Δ-9 DESATURASE)

An *E*. *coli* produced supernatant containing mature maize Δ-9 desaturase, as described above, was adjusted to 25 mM sodium phosphate buffer (pH 6.0) and chilled on ice for 1 h. Afterwards, the resulting flocculant precipitant was removed by centrifugation. The ice incubation step was repeated twice after which the solution remained clear. The clarified solution was loaded onto a Mono S HR10/10 column (Pharmacia) that had been equilibrated in 25 mM sodium phosphate buffer (pH 6.0). Proteins bound to the column matrix were eluted using a 0-500 mM NaCl gradient over 1 h (2 mL/min; 2 mL fractions). Fractions containing Δ-9 desaturase activity as described herein were pooled and separated based on size using a Superose 12B HR16/50 (Pharmacia) column) at a flow rate of 1.2 mL/min that had been equilibrated in 50 mM Tris-HCl (pH 7.5). Fractions were collected (1.2 mL/fraction) and samples were assayed for the presence of Δ-9 desaturase protein via SDS-PAGE. Molecular size calculations were based on column calibration using the following standards: ribonuclease A (13.7 kDa); ovalbumin (43.0 kDa); bovine serum albumin (67.0 kDa); aldolase (158.0 kDa); catalase (232.0 kDa); ferritin (440.0 kDa) and blue dextran (2,000 kDa).

The putative protein of interest was subjected to SDS-PAGE, blotted onto ProBlott membrane (Amersham), visualized with 0.02% amido black, excised, and sent to Harvard Microchem, (Boston, MA) for amino-terminal sequence analysis thus yielding the sequence entered herein as SEQ ID NO:13. Spectrophotometric analysis of the diiron-oxo component associated with the expressed protein (Fox et al., 1993, Proc. Natl. Acad. Sci. USA 90:2486-2490), as well as identification using a stain specific for nonheme iron (Leong et al., 1992, Anal. Biochem. 207:317-320) were also used to confirmed that the protein was mature Δ-9 desaturase.

The E. *coli* produced mature Δ-9 desaturase, as determined by amino terminal sequencing, was gel purified via SDS-PAGE and sent in the gel matrix to Berkeley Antibody Co. (Richmond, CA) for production of polyclonal antiserum in rabbits. Inoculations of antigen were initiated with 200 µg protein followed by three boosting injections of 100 µg each at three week intervals. Evaluations of antibody titers against mature Δ-9 desaturase were performed via western analysis using the ECL detection system (Amersham, Inc.; Arlington Heights, IL) .

Polyclonal antiserum produced above were used for western analysis as follows: purified or partially purified proteins were subjected to SDS-PAGE analysis on a 10-20% polyacrylamide gel (Integrated Separation Systems, Natick, MA) using the method described by Laemmli (1970, Nature 277:680). Proteins were then transferred to nitrocellulose paper using a Pharmacia Semi-Dry Blotter and the nonspecific binding sites were blocked with BLOTTO (5% dry milk in phosphate buffered saline [PBS]/0.05% Tween-20). The membrane was incubated in the polyclonal media (polyclonal antibody diluted 1:1000 in BLOTTO) for 1 h at room temperature while agitating on an orbital shaker. Afterwards, membranes were washed three times for 0.5 h each with excess BLOTTO followed by incubation for 0.5 h with a 1:2000 dilution of horseradish peroxidase linked goat-anti-rabbit polyclonal serum (BioRad, Hercules, CA). Following incubation, blots were washed three times for 10 min each with an excess of BLOTTO and then developed using the ECL Detection System (Amersham Inc.) as per the manufacturer's instructions.

Δ-9 desaturase was purified from corn kernels following homogenization using a Warring blender in 25 mM sodium phosphate buffer (pH 7.0) containing 25 mM sodium bisulfite and 2.5% polyvinylpolypyrrolidone. The crude homogenate was filtered through cheesecloth, centrifuged (10,000 x g) for 0.25 h and the resulting supernatant was filtered once more through cheesecloth. In some cases, the supernatant was fractionated via saturated ammonium sulfate precipitation at 20% (v/v). Upon centrifugation, the resulting supernatant was then precipitated with 80% (v/v) ammonium sulfate. Resulting pellets were dialyzed extensively against 25 mM sodium phosphate buffer (pH 6.0). After dialysis, the material was clarified via centrifugation and applied to a Mono S HR10/10 column (Pharmacia, Inc. Piscataway, NJ) equilibrated in 25 mM sodium phosphate buffer (pH 6.0). After extensive column washing, proteins bound to the column matrix were eluted using a 0-500 mM NaCl gradient over 1 h (2 mL/min; 2 mL fractions). Upon dialysis, this material was further fractionated by acyl carrier protein (ACP)-sepharose and phenyl Superose chromatography.

ACP-sepharose matrix was prepared as follows: ACP was purchased from Sigma Chemical Co. (St. Louis, MO) and purified by repeated precipitation at pH 4.1 followed by resolubilization (Rock and Cronan, 1981, Methods in Enzymology 71:341-351). Attachment of ACP to cyanogen bromide-activated Sepharose 4-B beads was performed essentially as described by Pharmacia, Inc., in the package insert. After linkage and blocking of the remaining sites with glycine, the ACP-sepharose material was packed into a HR 5/5 column (Pharmacia, Inc.) and equilibrated in 25 mM sodium phosphate buffer (pH 7.0). Mono-S fractions identified as having Δ-9 desaturase activity were then loaded onto the ACP-Sepharose column as described previously (McKeon and Stumpf, 1982, J. Biol. Chem. 254:7116-7122; Thompson et al., 1991, J. Biol. Chem. 257:12141-12147). After extensive column washing, ACP-binding proteins were eluted using 1M NaCl.

Fractions containing Δ-9 desaturase activity obtained from the ACP-sepharose column were adjusted to 0.4 M ammonium sulfate (25 mM sodium phosphate, pH 7.0) and loaded onto a Pharmacia Phenyl Superose column (HR 10/10). Proteins were eluted by running a gradient (0.4 - 0.0 M ammonium sulfate) at 2 mL/min for 1 hr. Δ-9 desaturase activity typically eluted between 60- and 30 mM ammonium sulfate as determined by enzymatic and western analysis. Proteins were dialyzed extensively and concentrated using a Centricon-10 concentrator (Amicon, Inc., Beverly, MA). Samples were then blotted to PVDF membrane as described herein and sent to Harvard Microchem for amino terminal analysis. The amino terminal sequence is entered herein as SEQ ID NO:14.

Production of substrate for Δ-9 desaturase activity assays was performed as follows. Cells harboring a plasmid encoding acyl-ACP synthetase (Jackowski et al., 1994, J. Biol. Chem. 269:2921-2928) were grown, heat shock induced, and the acyl-ACP synthetase protein produced therein was partially purified essentially as described by Rock and Cronan, (1979, J. Biol. Chem. 254:7116-7122). [9,10 (n)- ³H] stearic acid (specific activity = 49 Ci/mmol) was custom synthesized by Amersham Life Sciences, Inc. Radioactive and non radioactive ACP-stearate was synthesized essentially as described by Rock et al., (1981, Methods in Enzymology 71:341-351). Substrate production reactions were allowed to proceed for 16 h at 37° C before termination and purification. Non-radioactive substrate was made by using nonlabeled stearic acid. All reagents, unless otherwise specified, were obtained from Sigma Chemical Co.

Analysis of Δ-9 desaturase enzymatic activity was performed essentially as described by Thompson et al., (1991, Proc. Natl. Acad. Sci USA 88:2578-2582). Reactions were typically performed in triplicate and at three different enzyme concentrations to ensure linearity. Non-radioactive ACP-stearate was spiked with ³H-substrate and used at 60 µM final concentration (total volume = 500 µL; total radioactivity = 5.1 x 10⁴ dpm/sample). Reactions were allowed to proceed for 10 min at 25° C and were terminated by adding 0.5 mL of 12% (w/v) trichloroacetic acid (TCA). After incubation for 5 min, the resulting precipitate was centrifuged and an aliquot of the remaining supernatant was removed (0.5 mL) and pH adjusted by adding 500 µL of 1M Tris (pH 8.0). The Δ-9 desaturase enzymatic activity was quantitated by measuring the amount of ³H released by liquid scintillation counting. A unit of activity was defined as the production of 1 µmole of oleate/min as determined by the release of ³H₂O (McKeon and Stumpf, 1982, J. Biol. Chem. 257, 12141-12147). Testing of the maize purified and E. *coli* expressed Δ-9 desaturase protein proved that both protein preparations had the appropriate desaturase enzymatic activity.

### EXAMPLE 4

### PRODUCTION OF SYNTHETIC Δ-9 DESATURASE TRANSIT PEPTIDE (Δ-9TP) AND PRODUCTION OF POLYCLONAL ANTIBODIES SPECIFIC THERETO

As described herein, the maize Δ-9 desaturase gene was cloned and the amino acid sequence of the putative transit peptide (Δ-9TP) was determined and entered herein as SEQ ID NO:15. This sequence information was sent to Bio-Synthesis, Inc., (Lewisville, TX) for peptide synthesis. Once received, the peptide was dissolved in sterile water and aliquots thereof were analyzed by gel electrophoresis on a 4-27% gradient gel (Integrated Separation Science, Inc., Natick, MA). By comparing to the molecular weight markers, the synthetic Δ-9TP was determined to be about 4.0 kDa. The synthetic Δ-9TP was then conjugated to Keyhole Limpet hemacyanin (KLH) and injected into rabbits for polyclonal antibody production by Biosynthesis, Inc. Serum was taken at 10 weeks and 13 weeks after the initial injection. Polyclonal antibodies derived from this production were so named Pab-TP.

Immunospecificity of Pab-TP to the Δ-9TP peptide was determined by Western blot analysis using E. *coli* produced precursor (pDAB432D) and mature Δ-9 desaturase protein (pDAB428), as well as the synthetically made Δ-9TP polypeptide. Proteins were loaded onto a SDS-PAGE gel as described herein and were then blotted to PVDF membrane for western analysis as previously described. In testing Pab-TP antibodies, it was observed that said antibodies were immunologically reactive to both the precursor Δ-9 desaturase and the Δ-9TP polypeptide but did not recognize the mature protein. These data indicated that Pab-TP antibodies were specific for the transit peptide portion only of maize Δ-9 desaturase.

### EXAMPLE 5

### PRODUCTION AND SCREENING OF HYBRIDOMA CELL LINES

Approximately 100 to 200 µg of KLH conjugated Δ-9TP or Δ-9 desaturase mature protein was mixed with 1.0 mL of 1:1 mixture of Freund's Complete adjuvant and Freund's Incomplete adjuvant (Sigma Chemical Co.) for the initial immunizations. Samples were mixed only with Freund's incomplete adjuvant for subsequent immunization. Approximately 150 mL of the antigen was injected intraperitoneally into individual Balb/c mice at two week intervals. After 5 injections, immunogenic response was determined by Western analysis of sera from said mice. Injections were continued if the immunogenic response was weak, otherwise, mice were used in the production of hybridoma cell lines.

The cell fusion protocol was based on the procedure described by Galfre and Milstein (1981, Methods in Enzymology 73:3-46). After the spleen was aseptically removed from an individual mouse, spleen cells were separated and washed twice with RPMI 1640 medium (Cellgro™, Fisher Scientific, Pittsburgh, PA). A viable cell count was determined after addition of an equal volume of Red Blood Cell Lysis Buffer (Sigma, St. Louis, MO), and 2 volumes of Trypan blue (Sigma, St. Louis, MO). Myeloma cells (X63Ag8) originally purchased from American Tissue Culture Collection (ATCC) were used as the fusion partner.

Myeloma cells were grown at a density of 7x10⁵ cells/mL. Two days prior to cell fusion, cultures were diluted to 2x10⁵ cells/mL and washed twice with RPMI 1640 medium. Approximately 10⁸ spleen cells and 3.5 x10⁷ myeloma cells (a ratio of 3:1 spleen cells:myeloma cells) were used for each fusion. These cells were washed together once and the wash medium was discarded. One mL of pre-melted PEG 4000 (5.0 mg in 2.5 mL RPMI 1640 medium, 40° C) was added to the cell pellet. After 1 min, 20 mL of RPMI 1640 medium was gently added and the mixture was incubated at 37° C for 20 min. The medium was removed by centrifugation. Cell pellets were then resuspended in 200 mL of selection medium containing: complete medium [500 mL RPMI 1640, 50 mL fetal bovine serum (Sigma Chemical Co.), 6 mL 200 mM L-glutamine (Cellgro™), 6 mL 100 mM sodium pyruvate (Cellgro™), 5000 units penicillin-streptomycin (Cellgro™)], 10% Origen Hybridoma Cloning Factor (HCF; Origen, Fisher Scientific), and 1x HAT solution (hypoxanthine, aminopterin, and thymidine (Cellgro™). Cells were incubated for 1 h at 37° C and 200 µL thereof were distributed into each well of 96-well plates. After 10 to 14 days, media from each well were screened for antibody-producing hybridomas.

ELISA techniques were used to screen for Δ-9TP specific monoclonal antibody (Mab) producing hybridoma cell lines. The assay involved first applying the antigen (10-50 ng of synthetic Δ-9TP or precursor protein produced from pDAB432D) in 0.1 M sodium carbonate into wells of microtiter plates (Dynatech Laboratories, Chantilly, Virginia) and incubating overnight at 4° C. Afterwards, the excess antigen solution was discarded and the wells were blocked with 1% BSA (bovine serum albumin) in PBS (phosphate buffer saline, Sigma) at 37° C for at least 1 h. The blocking solution was discarded, and 100 µL of hybridoma cell medium was added to each well. Plates were incubated at 37° C for at least 1 h, after which they were washed 3 times with wash solution (0.025% Tween 20 in PBS). A 100 µL volume of a 1:1000 dilution of alkaline phosphatase conjugated anti-IgG + IgM antibodies (Kirkegaard & Perry, Gaithersburg, MD) in 1% BSA in PBS was added to each well. The plates were incubated at 37° C for 1 h and washed 3 times with the wash solution. Finally, the phosphatase substrate p-NPP (para-nitrophenyl phosphate; Kirkegaard & Perry, Gaithersburg, MD) prepared according to the manufacturer's procedure was added to the wells. The color was allowed to develop for 10 to 30 min before the A₄₀₅ₙₘ was determined using a plate reader (Molecular Devices, Sunnyvale, CA).

Cell lines determined positive by ELISA were increased by transferring the cells to 6-well plates containing fresh complete medium supplemented with 5% HCF. Once established, cell lines were re-screened by standard Western Blot analysis. Typically, 20 to 50 ng of precursor Δ-9 desaturase protein (pDAB432D) was applied to each well of a SDS polyacrylamide gel. After gel electrophoresis was completed, proteins were electroblotted onto Hybond-ECL nitrocellulose membrane (Amersham, Arlington Heights, IL) and western analysis was performed as described previously. Hybridoma cell medium obtained from test lines was used as the primary antibody. Secondary antibodies were typically goat-anti-mouse IgG conjugated to horseradish peroxidase (BioRad). Detection of immunological binding was performed using an ECL detection kit (Amersham) according to manufacturer's instruction.

Antibodies of interest were purified from the cell media using a Pure I antibody purification kit (Sigma). Two antibodies obtained from monoclonal cell lines (Mab-TP1 and Mab-Δ9M) were further characterized by isotyping. Antibody isotype was classified using a Mouse Isotyping Kit (Boehringer Mannheim, Indianapolis, IN). Mab-TP1 and Mab-Δ9M were determined to be IgG 2b Kappa Light Chain, and IgGl Kappa Light Chain, respectively.

To determine binding specificity of the selected MAbs, a Western blot analysis was performed using the following as antigen: black mexican sweet corn (BMS) callus tissue, Type II corn callus tissue, maize seed extract, *E.coli* produced precursor protein (pDAB432D), and purified mature Δ-9 desaturase protein. Duplicate blots were made and each blot was incubated with MAb-TP1, or MAb-Δ9M. The result showed that MAb-TP1 could only recognize Δ-9 desaturase precursor protein and did not recognize mature Δ9-desaturase or only other proteins in callus tissues or seeds. These data indicated that the Mabs generated against Δ-9TP were specific thereto.

### EXAMPLE 6

### CLONING OF GENES FROM MONOCLONAL CELL LINES

Hybridoma cell lines of interest were grown in two 150 mL flasks containing 60 mL of hybridoma medium. Seven days after inoculation, about 1 x 10⁸ cells were harvested into 50 mL centrifuge tubes and pelleted by centrifugation at 800 rpm using a table-top centrifuge. The medium was discarded and the cells were washed twice with sterile PBS. Tubes containing washed cells were placed on dry ice for 15 min prior to mRNA isolation.

PolyA+ mRNA was isolated using a Fast Track mRNA Isolation Kit (Invitrogen, Carlsbad, CA). Hybridoma cells were resuspended in 15 mL of lysis buffer provided therein. Cells were sheared by passing through a sterile 60 cc syringe fitted with an 18-21 gauge needle. This process was repeated 4 times. Lysates were incubated at 45° C for 15 min while shaking after which 950 µL of 5M NaCl was added. Upon mixing thoroughly, the solution was passed through the syringe an additional 4 times in order to cause DNA shearing. Seventy-five mg of powdered Oligo (dt) Cellulose was then added and the tube was rocked gently at room temperature for 1 h. Afterwards, lysates were centrifuge at 2000-4000 x g in a table top centrifuge for 5 min, supernatants were discarded, and pellets were washed twice with binding buffer and low salt buffer as provided. Pellets were then resuspended in 800 µL of low salt buffer. Cellulose suspensions were transferred into a spin column and washed 3 times by centrifugation and by addition of low salt buffer. After the last centrifugation, pellets were resuspended in 200 µL of Elution Buffer, the solution was collected by centrifugation and the process was repeated for a total volume of 400 µL. To precipitate the RNA, 60 µL of 2M sodium acetate and 1000 µL of absolute ethanol were added to the collected solution followed by freezing on dry ice. Following, tubes were centrifuged at 14,000 x g for 15 min and the mRNA pellet was resuspended in 20 to 40 µL of sterile filtered water.

The mRNA obtained above was used to produce cDNA by reverse transcriptase reactions using cDNA Cycling^{®} Kit (Invitrogen, Carlsbad, CA) according manufacturer's instructions. Typically, the total reaction volume was 20 µL with 8 µL of mRNA added thereto. Reactions were started by adding mRNA and water followed by heating for 10 min at 65° C and then followed by cooling for 2 min at room temperature. Afterwards, RNase inhibitor, buffer, nucleotide triphosphates, sodium pyrophosphate and reverse transcriptase were added. Ingredients were mixed and tubes were spun briefly before incubating at 42° C for 1 h. Tubes were then incubated at 95° C for 2 min before being placed on ice. cDNA synthesis was repeated again by adding additional reverse transcriptase followed by incubation for 1 h, followed by phenol extraction with 1 µL 0.5 M EDTA, pH 8.0 and 20 µL of Phenol/Chloroform/Isoamyl Alcohol (25:24:1). The tube was vortexed, centrifuged and the aqueous solution was recovered. To the aqueous solution was added 22 µL of ammonium acetate and 88 µL of absolute ethanol followed by freezing on dry ice and collection of pellets by centrifugation. Pellets were dissolved in sterile water and stored until further use.

Degenerate primers were designed and synthesized as disclosed herein. The 5'primers were designed to anneal to the signal peptide of the heavy (SEQ ID NO:16) or light chain genes (SEQ ID NO:17) based on published sequences (Kabat et al., 1987, Sequences of Proteins of Immunological Interest; 4th Ed. U.S. Department of Health and Human Services, Public Health Service, NIH). The 3' primers were designed to anneal to the constant region of the heavy (SEQ ID NO:18) and light chain genes (SEQ ID NO:19).

Genes of interest were obtained by PCR. The reaction (100 µL total volume) was prepared using a PCR Core Kit (Boehringer Mannheim, Corp., Indianapolis, IN) and typically contained 0.5 µg cDNA template and 100 pm of each primer. The heavy and light chain cDNAs were PCR amplified in separate experiments. PCR conditions were as follows: [95° C (1 min); 55° C (0. 5 min) ; 72° C (0.5 min)] for 30 cycles followed by 1 cycle of 95° C (1 min); 55° C (0.5 min); 72° C for 2 min. PCR products were observed using agarose gel electrophoresis and ethidium bromide staining. Amplification of the heavy and light chains resulted in DNA fragments of about 730 bp each entered herein as SEQ ID NO:24 and SEQ ID NO:21, respectively. Products were then digested with the NheI, cloned into pBlueBac1 (Invitrogen, Carlsbad, CA) and transformed into DH5α strain of *E. coli* using standard procedures. Clones were recovered and digested with NheI to verify the DNA insert. Plasmid DNA was produced, isolated, and sequenced as previously described using several external and internal primers. The DNA encoding the light chain (SEQ ID NO:50) was modified as follows: a 5' primer, entered herein as SEQ ID NO:20, was designed to replace the native mouse signal peptide with a Baculovirus gp67 and (p67) leader (Murphy et al., 1993, Protein Expression and Purification, 4:349-357). SEQ ID NO:50 was used as the 3' primer. After performing PCR as described herein, the DNA was digested with *Xba* I *and Not* I enzymes and inserted into a Baculovirus transfer plasmid, pAcMP3 (PharMingen, San Diego, CA). The cloned plasmid (AcMP3/TpLCp67) was transformed into the DH5α strain of E. *coli* (Gibco, Gaithersburg, MD). Several clones were recovered on the selection plates. Plasmid DNA was isolated from a selected clone and re-sequenced. Said sequence is entered herein as SEQ ID NO:49. The DNA encoding the hypervariable region of said clone is entered herein as SEQ ID NO:22. Comparison of sequence information indicated that the cloned Kappa chain was the authentic light chain of the Mab-Δ-9TP1 gene and not the endogenous non-functional light chain of myeloma cells (X63Ag8).

The DNA encoding the heavy chain (SEQ ID NO:24), obtained by using PCR primers SEQ ID NO: 16 and SEQ ID NO:18, was purified, digested with *Xba I* and Not *I* and ligated into pAcMP3. Sequencing data indicated that said clone was an authentic IgG2B heavy chain gene. The gene was re-amplified by PCR using primers with a sequence according to SEQ ID NO:23 and SEQ ID NO:18 to add a p67 Baculovirus leader, cloned into the AcMP3 Baculovirus transfer plasmid (AcMP3/TpHCp67) and transformed into E. *coli* as described herein. Colonies were screened and an appropriate clone was sequenced. The sequence (SEQ ID NO:51) matched that previously determined for the Mab-TP1 heavy chain. Comparison to known heavy chains revealed that the two CH1 sequences were identical indicating that the cloned heavy chain gene is an authentic IgG2B sequence. The sequence of the hypervariable region is entered herein as SEQ ID NO:25.

### EXAMPLE 7

### GENERATION OF RECOMBINANT BACULOVIRUS EXPRESSING Mab-TP1

SF9 (Invitrogen) cells were cultured at 27° C in spinner flasks with Sf900II (Gibco, Gaithersburg, MD) serum free complete medium supplemented with penicillin/streptomycin/fungizone at 1/2 rate. Cells were split to 3 x 10⁵ cells/mL every four days. SF9 cells were seeded at 8 x 10⁵ cells/well in a six well plate and allowed to attach for 1 h at 27° C. The medium was discarded and cells were washed twice with fresh medium without antibiotics. After the last wash, 1.5 mL of fresh medium without antibiotics was added. In a polystyrene tube, 0.1 µg of linearized parental Baculovirus DNA (Baculogold, PharMingen, San Diego, CA) was mixed with 0.5 µg of the transfer vector (AcMP3/TpLCp67 or AcMP3/TpHCp67 plasmid), followed by transfection mix as described in the standard cationic lipisome mediated transfection protocol as provided by CLONTECH (Palo Alto, CA). Transfection medium was harvested after four days and samples of medium and cell pellet were analyzed for protein expression by western analysis as described herein. A 1:1000 dilution of goat anti-mouse FAb-specific IgG conjugated to horseradish peroxidase (Sigma Chemical Co.) was used with a ECL detection kit (Amersham). The results indicated that light chain and heavy chain proteins could be produced and recognized by appropriate antibodies, thus authenticity was confirmed.

To determine if functional antibodies could be produced, recombinant virus, AcMP3/TpLCp67 and AcMP3/TpHCp67 were co-infected in SF9 cells. The cell medium was harvested and used as the primary antibody in Western analysis using precursor Δ-9 desaturase (pDAB432D) as antigen. The expected sized band (42 kDa) was observed indicating that the recombinant antibody produced was indeed functional.

### EXAMPLE 8

### CONSTRUCTION OF THE SINGLE CHAIN ANTIBODY-TP1 (SCAB-TP1) GENE

Generation of the *SCAb-Tp1* gene was accomplished via a two step PCR reaction using the PCR core kit (Boehringer Mannheim). Standard reactions contained 200µM dNTPs, 10.0 pM primer, SEQ ID NO:24 or SEQ ID NO:49 as template (0.5 µg), PCR buffer and 4 units of Taq polymerase as described previously. A sample of each reaction was analyzed in a 1% agarose gel. Approximately equal molar amounts of light and heavy chain product were then added to a second reaction using the same conditions as above except that different primers were used. The resulting PCR products were then isolated and purified from 1% agarose gels using the Qaiex II kit (Qiagen).

Amplification of the heavy chain variable region of SEQ ID NO:24 (which is SEQ ID NO:25) with primers according to SEQ ID NO:23 and SEQ ID NO:26 resulted in a PCR product of -450 bp entered herein as SEQ ID NO:29. Amplification of the light chain variable region of SEQ ID NO: 21 (which is SEQ ID NO:22) with primers according to SEQ ID NO:27 and SEQ ID NO:28 resulted in PCR product of -440 bp entered herein as SEQ ID NO:30. A sewing reaction using equal molar amounts DNA according to SEQ ID NO:29 and SEQ ID NO:30 using primers according to SEQ ID NO:23 and SEQ ID NO:28 yielded the SCAb-Tp1 gene product of -830 bp and having a DNA and amino acid sequence entered herein as SEQ ID NO:31 and SEQ ID NO:32, respectively. The DNA was then digested with Xba I and Not I and ligated into the AcMP3 Baculovirus transfer vector (PharMingen). A positive clone was identified and sequenced (SEQ ID NO:31), a c-myc tag (SEQ ID NO:33) was added to the 3' end of the gene and the p67 leader sequence (SEQ ID NO:34) was added at the 5' terminus.

The SCAb-TP1 gene (SEQ ID NO:31) was reconstructed by PCR using the reaction conditions described above. Amplification of DNA according to SEQ ID NO:22 with primers according to SEQ ID NO:35 and SEQ ID NO:36 resulted in a product of -450 bp, having the sequence entered herein as SEQ ID NO:40. Amplification of DNA according to SEQ ID NO:25 with primers according to SEQ ID NO:37 and SEQ ID NO:38 (TABLE V) resulted in a product of -440 bp having a DNA sequence according to SEQ ID NO:41. Approximately equal molar amounts of each product (SEQ ID NO:40 and SEQ ID NO:41) were added to another reaction with primers according to SEQ ID NO:35 and SEQ ID NO:38 yielding a product of -846 bp (SEQ ID NO:42). An additional PCR reaction was performed with DNA according to SEQ ID NO:42 using the primer pair having a DNA sequence according to SEQ ID NO:35 and SEQ ID NO:39 to add a stop codon to the 3' end of the gene. The -849bp product (SEQ ID NO:43) was isolated from a 1% agarose gel, digested with Xba I/Not I and ligated into the AcMP3 transfer vector (AcMP3/TPSCegt). An appropriate clone was identified and sequenced.

AcMP3/TPSCegt and BacPAK6 Bsu36 I linear parental DNA (Clontech) were co-transfected into SF9 insect cells to create recombinant virus. Cationic lipisome mediated transfection was performed as described in the instructions included with the BacPAK6 DNA. The transfection media was harvested after 48 h and 0.5 mL was used to infect a 175cm² flask containing 2 x 10⁷ SF9 cells to amplify the recombinant virus. Western analysis performed as described herein identified a -28kD protein in infected cells using an anti-HIS antibody (Qiagen, Inc., Chatsworth, CA). SCAb-Tp1 produced in the Baculovirus system was compared to the native MAb-Δ-9TP1 in Western blots using E. coli expressed Δ-9 desaturase precursor (pDAB432D) as antigen. These blots indicated no difference in the binding pattern between the native MAb TP1 and the recombinant SCAb-TP1.

The SCAb-TP1 gene was rebuilt to facilitate cloning into the pDAB439 plant transformation vector. PCR reactions were performed using the DNA according to SEQ ID NO:43 with the primer pairs having DNA sequences according to SEQ ID NO:44/SEQ ID NO:46 and primer pairs having DNA sequences according to SEQ ID NO:45/SEQ ID NO:46. The resulting products (SEQ ID NO:47 and SEQ ID NO:48) were isolated from a 1% agarose gel, digested with Sfi I and ligated into pDAB439. An appropriate clone was identified for each construct, grown up and sequenced thus yielding the plasmids pDAB439/TPE containing SEQ ID NO:47 and pDAB439/TPnoE containing SEQ ID NO:48.

Plasmid pDAB439 was a 7040 base pairs double stranded plant transformation vector composed of the following sequences in clockwise order. The plasmid backbone was derived from pUC19 (Yanish-Perron et al., (1985) Gene 33:103-119). Nucleotides 1 to 2252 of pDAB439 corresponded to the reverse complement of nucleotides 435 to 2686 of pUC19. Nucleotides 2253 to 2271 of pDAB439 had the sequence TGCATGTGTT CTCCTTTTT (SEQ ID NO:52). Nucleotides 2272 to 4264 of pDAB439 were the maize ubiquitin promoter and first intron, and were PCR amplified from genomic DNA of maize genotype B73 (Christensen et al., (1992) Plant Mol. Biol. 18:675-689). Nucleotides 4265 to 4308 of pDAB439 had the sequence GGTACGGCCA TATTGGCCGA GCTCGGCCTC TCTGGCCGAT CCCC (SEA ID NO:53). Nucleotides 4309 to 4576 of pDAB439 corresponded to nucleotides 4420 to 4687 of plasmid pBI101 (Clontech, Palo Alto, CA) followed by the linker GG as nucleotides 4577 and 4578 of pDAB439. Nucleotides 4579 to 4743 of pDAB439 were the reverse complement of nucleotides 238-402 of pUC19. Nucleotides 4744 to 4807 of pDAB439 corresponded to: GCGGCCGCTT TAACGCCCGG GCATTTAAAT GGCGCGCCGC GATCGCTTGC AGATCTGCAT GGG (SEQ ID NO:54). Nucleotides 4808-5416 of pDAB439 comprised the double enhanced 35S promoter, with nucleotides 5070 to 5416 corresponding to nucleotides 7093 to 7439 of the Cauliflower Mosaic Virus genome (Franck et al., (1980) Cell 21:285-294). Nucleotides 4808 to 5061 of pDAB439 were a duplication of nucleotides 5068 to 5321. Nucleotides 5062 to 5067 of pDAB439 comprised the linker CATCGA. Nucleotides 5417-5436 of pDAB439 comprised the linker GGGGACTCTA GAGGATCCAG (SEQ ID NO:55). Nucleotides 5437 to 5547 of pDAB439 corresponded to nucleotides 167 to 277 of the Maize Streak Virus genome (Mullineaux et al., (1984) EMBO J. 3:3063-3068). Nucleotides 5548 to 5764 of pDAB439 corresponded to the modified first intron of the maize alcohol dehydrogenase gene (Adhl-S) (Dennis et al., (1984) Nucleic Acids Res. 12:3983-4000). The modification resulted in removal of 343 nucleotides (bases 1313 to 1656) with bases 1222 to 1312 (intron 5' end) and nucleotides 1657 to 1775 (intron 3' end) of the maize Adh1-S gene remaining. Nucleotides 5765 to 5802 of pDAB439 corresponded to Maize Streak Virus (MSV) nucleotides 278 to 312, followed by the linker sequence CAG. Both sections of the Maize Streak Virus, hereinafter MSV, sequence comprised the untranslated leader of the MSV coat protein V2 gene, and were interrupted in plasmid pDAB439 by the modified Adh1 intron. Nucleotides 5803 to 6359 of plasmid pDAB439 corresponded to nucleotides 29 to 585 of the phosphinotricin acetyl transferase (BAR) gene of Streptomyces hygroscopicus (White et al., (1989) Nucleic Acids Res. 18:1062). To facilitate cloning, nucleotides 34 and 575 of the published sequence were changed from A and G to G and A, respectively. This sequence served as the selectable marker in plant cells. Nucleotides 6360 to 6364 comprised the linker GATCT. Nucleotides 6365 to 6635 of pDAB439 corresponded to nucleotides 4420 to 4683 of plasmid pBI101 (Clontech, Palo Alto, CA) followed by the linker sequence AGATCGC. Nucleotides 6636 to 6639 of pDAB439 comprised the linker TCGG. The remaining sequence of pDAB439 (nucleotides 6640 to 7040) corresponded to nucleotides 284 to 684 of pUC19.

### EXAMPLE 9

### GENERATION OF TRANSGENIC MAIZE PLANTS CONTAINING AND EXPRESSING THE GENES OF INTEREST

Type II callus cultures were initiated from immature zygotic embryos of the genotype "Hi-II." (Armstrong et al, (1991) Maize Cooperation Newsletter, pp.92-93). Embryos were isolated from greenhouse-grown ears from crosses between Hi-II parent A and Hi-II parent B or F2 embryos derived from a self- or sib-pollination of a Hi-II plant. Immature embryos (1.5 to 3.5 mm) were cultured on initiation medium consisting of N6 salts and vitamins (Chu et al, (1978) The N6 medium and its application to anther culture of cereal crops. Proc. Symp. Plant Tissue Culture, Peking Press, 43-56) 1.0 mg/L 2,4-D, 25 mM L-proline, 100 mg/L casein hydrolysate, 10 mg/L AgNO₃, 2.5 g/L GELRITE, and 20 g/L sucrose, with a pH of 5.8. Selection for Type II callus took place for ca. 2-12 weeks. After four to six weeks callus was subcultured onto maintenance medium (initiation medium in which AgNO₃ was omitted and L-proline was reduced to 6 mM).

The plasmids pDAB439/TPE and pDAB439/TPnoE were transformed into embryogenic callus via helium bombardment. For blasting 140 µg of plasmid DNA was precipitated onto 60 mg of alcohol-rinsed, spherical gold particles (1.5 - 3.0 µm diameter) by adding 74 µL of 2.5 M CaCl₂ H₂O and 30 µL of 0.1 M spermidine (free base) to 300 µL of plasmid DNA and H₂O. The solution was immediately vortexed and the DNA-coated gold particles were allowed to settle. The resulting clear supernatant was removed and the gold particles were resuspended in 1 mL of absolute ethanol. This suspension was diluted with absolute ethanol to obtain 15 mg DNA-coated gold/mL. Approximately 600 mg of embryogenic callus tissue was spread over the surface of Type II callus maintenance medium as described herein lacking casein hydrolysate and L-proline, but supplemented with 0.2 M sorbitol and 0.2 M mannitol as an osmoticum. Following a 4 h pre-treatment, tissue was transferred to culture dishes containing blasting medium (osmotic media solidified with 20 g/L tissue culture agar (JRH Biosciences, Lenexa, KS) instead of 7 g/L GELRITE (Schweizerhall, South Plainfield, NJ). Helium blasting accelerated suspended DNA-coated gold particles towards and into the prepared tissue targets. The device used was an earlier prototype of that described in US Patent #5,141,131 which is incorporated herein by reference. Tissues were covered with a stainless steel screen (104 µm openings) and placed under a partial vacuum of 25 inches of Hg in the device chamber. The DNA-coated gold particles were further diluted 1:1 with absolute ethanol prior to blasting and were accelerated at the callus targets four times using a helium pressure of 1500 psi, with each blast delivering 20 µL of the DNA/gold suspension. Immediately post-blasting, tissue was transferred to osmotic media for a 16-24 h recovery period. Afterwards, the tissue was divided into small pieces and transferred to selection medium (maintenance medium lacking casein hydrolysate and L-proline but having 30 mg/L BASTA (Agrevo)). Every four weeks for 3 months, tissue pieces were non-selectively transferred to fresh selection medium. After 7 weeks and up to 22 weeks, callus sectors found proliferating against a background of growth-inhibited tissue were removed and isolated. The resulting BASTA-resistant tissue was subcultured biweekly onto fresh selection medium. Following gas chromatography/fatty acid methyl ester, hereinafter GC/FAME, analyses, as described herein, positive transgenic lines were identified and transferred to regeneration media.
Regeneration was initiated by transferring callus tissue to cytokinin-based induction medium, which consisted of Murashige and Skoog salts, hereinafter MS salts, and vitamins (Murashige and Skoog, (1962) Physiol. Plant. 15: 473-497) 30 g/L sucrose, 100 mg/L *myo*-inositol, 30 g/L mannitol, 5 mg/L 6-benzylaminopurine, hereinafter BAP, 0.025 mg/L 2,4-D, 30 mg/L BASTA, and 2.5 g/L GELRITE (Schweizerhall) at pH 5.7. The cultures were placed in low light (125 ft-candles) for one week followed by one week in high light (325 ft-candles). Following a two week induction period, tissue was non-selectively transferred to hormone-free regeneration medium, which was identical to the induction medium except that it lacked 2,4-D and BAP, and was kept in high light. Small (1.5-3 cm) plantlets were removed and placed in 150x25 mm culture tubes containing SH medium (SH salts and vitamins (Schenk and Hildebrandt, (1972) Can. J. Bot. 50:199-204), 10 g/L sucrose, 100 mg/L myo-inositol, 5 mL/L FeEDTA, and 2.5 g/L GELRITE (Schweizerhall), pH 5.8). Plantlets were then transferred to 10 cm pots containing approximately 0.1 kg of METRO-MIX 360 (The Scotts Co. Marysville, OH) in the greenhouse as soon as they exhibited growth and developed a sufficient root system. They were grown with a 16 h photoperiod supplemented by a combination of high pressure sodium and metal halide lamps, and were watered as needed with a combination of three independent Peters Excel fertilizer formulations (Grace-Sierra Horticultural Products Company, Milpitas, CA). At the 3-5 leaf stage, plants were transferred to five gallon pots containing approximately 4 kg METRO-MIX 360. Primary regenerants were self- or sib-pollinated, or outcrossed to either elite inbreds or transgenic plants after an additional 6 - 10 weeks in the 5 gallon pots. R₁ seed was collected at 40-45 days post-pollination.

Embryogenic callus material containing the genes of interest was maintained as described herein. Continuous production of somatic embryos, which make up a large portion of embryogenic callus, was performed by transferring the callus tissue every two weeks. While the somatic embryos continued to proliferate, they usually remained in an early stage of embryo development because of the continued presence of 2,4-D in the culture medium. Somatic embryos could be regenerated into plantlets when callus was subjected to the regeneration procedure described herein. During regeneration, somatic embryos formed roots and a shoot, subsequently ceasing development as an embryo.

Somatic embryos were made to develop as seed embryos by growing embryogenic callus on MS medium containing 6% (w/v) sucrose. The callus was grown for 7 days and then somatic embryos were individually transferred to MS medium with 6% sucrose and 10 µM abscisic acid, hereinafter ABA.

### EXAMPLE 10

### SOUTHERN ANALYSIS OF TRANSFORMED CALLUS AND PLANT TISSUES

BASTA resistant lines transformed with various plasmids were characterized by Southern analysis to confirm the presence of the transgene using a DNA probe specific for the coding region of the gene of interest. DNA from leaf material was analyzed.

Leaf material from plants was harvested at the 6-8 leaf stage. Genomic DNA was prepared from lyophilized tissue as described by Saghai-Maroof et. al. ((1984) Proceed. Nat. Acad. Sci. USA 81:8014-8018). Eight µg of each DNA was digested with the restriction enzyme(s) specific for each plasmid construct using conditions suggested by the manufacturer (Bethesda Research Laboratory) and separated by electrophoresis on a 0.8% agarose gel. The DNA was then blotted onto nylon membranes as described by Southern ((1975) J. Mol. Biol., 98:503-517). The radioactive probe was then hybridized to the genomic DNA on the blots in 45 mL of minimal hybridization buffer [10% polyethylene glycol, 7% SDS, 0.6x SSC, 10 mM sodium phosphate, 5 mM EDTA and 100 µg/mL denatured salmon sperm DNA] overnight at 60° C. After hybridization, blots were washed at 60° C in 0.25X SSC and 0.2% SDS for 45 min., blotted dry and exposed to XAR-5 film (Kodak) overnight on two intensifying screens (DuPont).

All lines analyzed showed at least one copy of the expected sized fragment indicating a complete copy of SCAb-TP1 gene.

### EXAMPLE 11

### SCAB-TP1 EXPRESSION IN MAIZE

For RNA isolation, the tissue was pulverized using a Bessman Tissue Pulverizer (Spectrum Medical Industries, Houston, TX). RNA was extracted from the frozen powder using RNEASY Plant mini kits (Qiagen) according to the manufacturer's instructions. For expression analysis (Northern analysis), 5 µg RNA per sample was fractionated by electrophoresis in non-denaturing 10 mM NaPO₄ pH 6.8, 1.0% agarose gels. The volume of sample containing said RNA was dried using a SAVANT SpeedVac (Savant Instruments, Inc., Farmingdale, NY), resuspended in 8 µL water, and denatured with an equal volume of 2x sample buffer (40 mM NaHPO₄, pH 6.8; 10 mM EDTA, 6% formaldehyde, 50% formamide) and heated to 68° C for 15 minutes. The denatured sample was chilled on ice and 4 µL loading buffer (50% glycerol, 10 mM EDTA, 5 mM NaPO₄, 0.25% bromophenol blue) was added. The samples were loaded on the gel and electrophoresed for 3 h at 60 V in 10 mM phosphate buffer. RNA was transferred from the gel to GENESCREEN PLUS membrane (NEN Research Products, Boston, MA) by capillary transfer with sterile water as the transfer medium. Following transfer, the RNA was crosslinked to said membrane by UV (STRATALINKER, Stratagene Cloning Systems, Inc., La Jolla, CA).

The RNA blot was prehybridized for 3 h at 42° C in hybridization buffer (50 mM sodium phosphate, pH 6.5, 0.8 M sodium chloride, 1 M EDTA, 0.2% sodium dodecyl sulfate, 0.05% bovine serum albumin, 0.05% Ficoll Type 40, 10% dextran sulfate). A hybridization probed specific for the antibody made against the transit peptide was labelled with 50 µCi of α-³²P-dCTP (NEN Research Products) using READY-TO-GO labelling beads (Pharmacia, Piscataway, NJ) according to the manufacturers instructions and purified over NUCTRAP columns (Stratagene). The labeled probe was denatured by boiling for 5 min, chilled on ice for 5 min, and added directly to the prehybridization blots. Hybridization was done in SEAL-A-MEAL bags (Dazey Corp., Industrial Airport, KA), at 42° C for 16 h. Blots were washed 6 times for 0.5 h each in large excess of prewarmed washing solution (20 mM NaPO₄, pH 6.5, 50 mM NaCl, 1 mM EDTA, and 0.1% sodium dodecyl sulfate) at 60° C. The blot was exposed to a phosphor storage screen, scanned on a Molecular Dynamics Personal PHOSPHORIMAGER (Molecular Dynamics, Inc., Sunnyvale, CA) for analysis.

For analysis of R₀ plants, leaf tissue (- 0.2 g) was harvested from plants transformed with pDAb439/TPE and pDAB439/TPnoE. RNA was extracted, fractionated, blotted to membranes and probed as described above. Lines that were positive for transgenic RNA at the callus stage were found to be positive at the R0 plants stage. These data indicated that the transgene was integrated and expressing mRNA related to the gene of interest.

To examine protein expression, SCAb-TP1 protein was purified from transformed maize callus tissue using the Qiagen Ni-NTA kit. Callus tissue (0.75-1.45g) was ground with 400-600 µL 1X binding buffer containing 1mM Phenylmethylsulfonyl fluoride (PMSF) using a plastic tissue grinder and a small amount of sand. The lysate was then spun in a microfuge for 10 min to pellet cellular debris. The supernatant was filtered through a 0.2 µm syringe filter and applied to Ni-NTA spin column (Qiagen). The column was washed with 600 µL wash buffer and eluted with 400 µL elution buffer. The eluted material was then precipitated with acetone or concentrated with 3000 mw micro spin column (Amicon, Beverly, MA) and buffer exchanged with 10 mM phosphate. The concentrated protein was then run in 4-20% SDS-PAGE gels (Integrated Separation Systems, Natick, MA) and Western analysis were performed as described herein. A MRGS 6X HIS MAb preparation (Qiagen) was used as primary antibody. Detectable levels of SCAb-TP1 protein were observed in lines transformed with pDAB439/TPE and pDAB439/TPnoE.

### EXAMPLE 12

### EFFECT OF SCAB-TP1 EXPRESSION ON ENDOGENOUS Δ-9 DEATURASE LEVELS IN TRANSFORMED MAIZE

The effects of SCAb-TP1 protein expression on endogenous levels of Δ-9 desaturase were examined using transformed maize callus tissues via Western analyses as described herein. Polyclonal antibodies immunologically reactive against mature Δ-9 desaturase were used as the primary antibody. Western data indicated that expression of SCAb-TP1 in callus tissue caused about 70% reduction in the levels of Δ-9 desaturase compared with control callus lines that were transformed with unrelated genes. Protein analysis on leaf tissues of R₀ plants also showed reduced levels (10 to 50%) of Δ-9 desaturase when compared to control lines. These data indicated a direct correlation between the presence and expression of the transgene and reduced Δ-9 desaturase levels (Table 1).

**Table 1. Comparison of Δ-9 desaturase levels in maize lines transformed the pDAB439/TPE and pDAB439/TPnoE relative to control.**

| Line | SCAb-TP1 Protein | Reduced Δ-9 desaturase |
|---|---|---|
| TPE-04 | + | yes |
| TPE-05 | + | yes |
| TPE-06 | + | yes |
| TPE-07 | + | yes |
| TPE-08 | nd | yes |
| TPE-09 | + | yes |
| TPE-10 | + | yes |
| TPnoE-01 | nd | yes |
| TPnoE-04 | nd | yes |
| TPnoE-05 | + | yes |
| TPnoE-07 | + | yes |
| TPnoE-11 | + | yes |
| TPnoE-12 | nd | yes |

| | | |
|---|---|---|
| nd: not determined | | |

Fertile plants containing pDAB463/TPnoE were crossed with inbred CQ715 HO-I (Mycogen Seeds, San Diego, CA) as pollen donors and seed were obtained. These seed were planted and molecular analyses were performed on leaf samples to determine the presence of SCAB-TP1 gene, SCAB-TP1 protein and their effect on Δ-9 desaturase levels. Reduction in Δ9-desaturase levels (40-70%) was found to correlated with the presence of the transgene and SCAB-TP1 expressed therefrom.

### EXAMPLE 13

### Antibody-Mediated Down-Regulation of Maize Palmitoyl-ACP Thioesterase (PTE)

Antibodies directed against the chloroplast transit peptide domain of the PTE protein are used in this example to block import of PTE into the chloroplast where early steps of fatty acid biosynthesis occur.

### A. Analysis of PTE Transit Peptides from Various Maize Genotypes.

Successful application of antibody mediated downregulation requires that the amino acid sequence of the transit peptide be highly conserved between the maize lines used for transformation and regeneration in the laboratory, and the elite maize lines into which the genes will be introgressed for commercial development. DNA sequences encoding the transit peptide domain of PTE were amplified from eight different corn varieties: CS608, CQ806, HO1, Hill, OQ414, HO601, 2XH753 and 2XH755. The deduced protein sequences were almost identical among all lines, differing only by the absence of one or two amino acids of the 90 amino acids which comprise the transit peptide. These theoretical amino acid sequences were used to design a region for antibody targeting.

### B. Design of Antibodies Targeted to the maize PTE Transit Peptide

The amino acid sequence of PTE precursor protein from maize line CS608 was analyzed to identify a region on the transit peptide portion that exhibits high antigenicity and surface probability. A sequence (PGSPAPAAPKNGLGERPESLDVRG)(SEQ ID NO:56) extending from amino acid residue number 12 to 35 was selected. The sequence information was sent to Zymed Laboratories Inc. for peptide synthesis and polyclonal antibody production in two rabbits.

### C. Baculovirus expression of PTE precursor protein

The gene coding for PTE precursor (including signal peptide) was cloned into the baculovirus transfer vector pAcSG2 (PharMingen, San Diego, CA). A cotransfection was performed in a 6-well plate with 6 x 10⁵Sf9 cells/well. BacPAK6 linear DNA (Clontech, Palo Alto, CA) was used as parental DNA in the cotransfection. Bacfectin reagent was used to facilitate cotransfection (included with BacPAK6 DNA). Transfection media was harvested at 3 and 6 days post-transfection. Sf9 cells (6 x 10⁵cells/well) were infected with 100µl transfection media. After 3 days, cells were harvested and western blots were performed. Blots were probed with rabbit polyclonal antibody generated against the mature PTE protein or to the transit peptide of the precursor protein (rabbit 1 or rabbit 2). The blot probed with antibody to the mature protein showed 2 distinct bands for PTE with or without the transit peptide. Blots probed with antibody to the transit peptide only detected the higher molecular weight form of the PTE containing the transit peptide.

Production of hybridoma cell lines that produce antibodies that bind to the PTE precursor protein, cloning of the antibody genes and single chain antibody gene, and transformation into corn can be carried out as described in foregoing Examples 5 through 9.

## Claims

1. A nucleic acid construct comprising in the 5' to 3' direction of transcription.
a promoter functional in a plant cell,
a nucleic acid sequence that encodes an antibody or fragment thereof having the ability to bind to a transit peptide that directs a passenger protein associated with the transit peptide to an organdie of a plant cell, and
a termination region functional in a plant cell.

2. A method of decreasing the steady state level of a passenger protein in a plant cell which comprises placing a nucleic acid construct of Claim 1 in the plant cell.

3. A plant cell comprising a nucleic acid construct according to Claim 1.

4. The plant cell of Claim 3, wherein the steady state level of a passenger protein found therein has been decreased by the method of Claim 2.

5. A plant or progeny thereof derived from the plant cell of Claim 2 or 3.

6. A construct, method, cell, plant, or progeny, according to any preceding claim, wherein said plant cell is a dicotyledon.

7. A construct, method, cell, plant, or progeny, according to any one of Claims 1 to 5, wherein said plant cell is a monocotyledon.

8. A construct, method, cell, plant, or progeny, according to Claim 7, wherein said plant is a maize plant.

9. A construct, method, cell, plant, or progeny, according to Claim 8. wherein said transit peptide is the transit peptide for maize stearoyl-ACP Δ-9 desaturase or maize palmitoyl-ACP thioesterase.

10. A construct, method, cell, plant, or progeny, according to any preceding claim, wherein said organelle is selected from the group consisting of chloroplast, amyloplast, chromoplast, leucoplast, mitochrondria, and the nucleus.

11. A construct, method, cell, plant, or progeny, according to any preceding claim, wherein said antibody or fragment thereof is a single chain antibody molecule.

12. A construct, method, cell, plant, or progeny, according to any preceding claim, wherein said epitope comprises at least 6 amino acids, said amino acids being continuously adjacent to each other in said transit peptide.

13. A construct, method, cell, plant, or progeny, according to any one of Claims 1 to 5, wherein said nucleic acid sequence is selected from the group consisting of SEQ ID NO:21, SEQ ID NO:22, SEQ ID NO:24. SEQ ID NO:25, SEQ ID NO:31, SEQ ID NO:43, and SEQ ID NO:48.

14. A hybridoma cell line designated 10E10, having ATTC Designation HB-12544.

15. An antibody produced by the hybridoma cell line of Claim 14.

## Patentansprüche

1. Nukleinsäurekonstrukt, umfassend in 5' nach 3'-Richtung der Transkription, einen in einer Pflanzenzelle funktionellen Promotor,
eine Nukleinsäuresequenz, welche für einen Antikörper oder ein Fragment hiervon kodiert, wobei der Antikörper oder das Fragment hiervon die Fähigkeit besitzt, an ein Transitpeptid zu binden, welches ein mit dem Transitpeptid assoziiertes Passagierprotein zu einer Organelle einer Pflanzenzelle dirigiert, und
eine in einer Pflanzenzelle funktionelle Terminationsregion.

2. Verfahren zur Verringerung der stationären Menge eines Passagierproteins in einer Pflanzenzelle, welches das Einbringen eines Nukleinsäurekonstrukts nach Anspruch 1 in die Pflanzenzelle umfasst.

3. Pflanzenzelle, umfassend ein Nukleinsäurekonstrukt nach Anspruch 1.

4. Pflanzenzelle nach Anspruch 3, wobei die stationäre Menge eines darin gefundenen Passagierproteins durch das Verfahren nach Anspruch 2 verringert worden ist.

5. Pflanze oder Nachkommenschaft hiervon, welche aus der Pflanzenzelle nach Anspruch 2 oder 3 erhalten wird.

6. Konstrukt, Verfahren, Zelle, Pflanze oder Nachkommenschaft nach einem der vorhergehenden Ansprüche, wobei die Pflanzenzelle zweikeimblättrig ist.

7. Konstrukt, Verfahren, Zelle, Pflanze oder Nachkommenschaft nach einem der Ansprüche 1 bis 5, wobei die Pflanzenzelle einkeimblättrig ist.

8. Konstrukt, Verfahren, Zelle, Pflanze oder Nachkommenschaft nach Anspruch 7, wobei die Pflanze eine Maispflanze ist.

9. Konstrukt, Verfahren, Zelle, Pflanze oder Nachkommenschaft nach Anspruch 8, wobei das Transitpeptid das Transitpeptid für Stearoyl-ACP-Δ9-Desaturase aus Mais oder Palmitoyl-ACP-Thioesterase aus Mais ist.

10. Konstrukt, Verfahren, Zelle, Pflanze oder Nachkommenschaft nach einem der vorhergehenden Ansprüche, wobei die Organelle aus der Gruppe bestehend aus Chloroplast, Amyloplast, Chromoplast, Leukoplast, Mitochondrien und dem Zellkern ausgewählt ist.

11. Konstrukt, Verfahren, Zelle, Pflanze oder Nachkommenschaft nach einem der vorhergehenden Ansprüche, wobei der Antikörper oder das Fragment hiervon ein einzelkettiges Antikörpermolekül ist.

12. Konstrukt, Verfahren, Zelle, Pflanze oder Nachkommenschaft nach einem der vorhergehenden Ansprüche, wobei das Epitop mindestens 6 Aminosäuren umfasst, wobei die Aminosäuren in dem Transitpeptid kontinuierlich aneinander gereiht sind.

13. Konstrukt, Verfahren, Zelle, Pflanze oder Nachkommenschaft nach einem der Ansprüche 1 bis 5, wobei die Nukleinsäuresequenz aus der Gruppe bestehend aus SEQ ID NO:21, SEQ lD NO:22, SEQ ID NO:24, SEQ lD NO:25, SEQ ID NO:31, SEQ ID NO:43 und SEQ ID NO:48 ausgewählt ist.

14. Hybridomzelllinie mit der Bezeichnung 10E10, welche die ATCC-Bezeichnung HB-12544 besitzt.

15. Antikörper, produziert von der Hybridomzelllinie nach Anspruch 14.

## Revendications

1. Construction d'acide nucléique comprenant, dans le sens 5'→3' de transcription :
- un promoteur fonctionnel chez une cellule végétale,
- une séquence d'acide nucléique codant un anticorps ou un fragment d'anticorps capable de se lier à un peptide transporteur qui amène une protéine passagère, associée au peptide transporteur, à une organelle d'une cellule végétale,
- et une région de terminaison fonctionnelle chez une cellule végétale.

2. Procédé de réduction du niveau stationnaire d'une protéine passagère dans une cellule végétale, qui comporte le fait de placer dans cette cellule végétale une construction d'acide nucléique conforme à la revendication 1.

3. Cellule végétale comprenant une construction d'acide nucléique conforme à la revendication 1.

4. Cellule végétale conforme à la revendication 3, dans laquelle on a réduit, en suivant un procédé conforme à la revendication 2, le niveau stationnaire d'une protéine passagère qui s'y trouve.

5. Végétal dérivé d'une cellule végétale conforme à la revendication 2 ou 3, ou descendance d'un tel végétal.

6. Construction, procédé, cellule, végétal, ou descendance, conforme à l'une des revendications précédentes, ladite cellule végétale étant d'une plante dicotylédone.

7. Construction, procédé cellule, végétal, ou descendance, conforme à l'une des revendications 1 à 5, ladite cellule végétale étant d'une plante monocotylédone.

8. Construction, procédé, cellule, végétal, ou descendance, conforme à la revendication 7, ledit végétal étant un maïs.

9. Construction, procédé, cellule, végétal, ou descendance, conforme à la revendication 8, où ledit peptide transporteur est le peptide assurant le transport de la stéaroyl-ACP Δ-9 désaturase de maïs ou de la palmitoyl-ACP thioesterase de maïs.

10. Construction, procédé, cellule, végétal, ou descendance, conforme à l'une des revendications précédentes, où ladite organelle est choisie dans l'ensemble constitué par les chloroplastes, les amyloplastes, les chromoplastes, les leucoplastes, les mitochondries et le noyau.

11. Construction, procédé, cellule, végétal, ou descendance, conforme à l'une des revendications précédentes, où ledit anticorps ou fragment d'anticorps est une molécule d'anticorps à chaîne unique.

12. Construction, procédé, cellule, végétal, ou descendance, conforme à l'une des revendications précédentes, où ledit épitope comporte au moins 6 résidus d'acides aminés, lesquels résidus d'acides aminés forment, au sein dudit peptide transporteur, une suite continue de résidus adjacents l'un à l'autre.

13. Construction, procédé, cellule, végétal, ou descendance, conforme à l'une des revendications 1 à 5, où ladite séquence d'acide nucléique est choisie dans l'ensemble formé par la Séquence N° 21, la Séquence N° 22, la Séquence N° 24, la Séquence N° 25, la Séquence N° 31, la Séquence N° 43, et la Séquence N° 48.

14. Lignée de cellules d'hybridome, appelée 10E10, et désignée HB-12544 à l'ATTC.

15. Anticorps produit par la lignée de cellules d'hybridome conforme à la revendication 14.
